# EUROPEAN PATENT APPLICATION

(11) **EP 4 825 944 A1**
(43) Date of publication of application: **21.10.2026**
(21) Application number: 26168678.6
(22) Date of filing: 27.03.2026
(51) Int. Cl.: C09K 11/06, C07D 209/86, H10K 50/15, H10K 85/60, C09K 11/02, C09K 11/56, C09K 11/88

(54) **LOW MOLECULAR WEIGHT COMPOUND, COMPOSITION COMPRISING LOW MOLECULAR WEIGHT COMPOUND, ORGANIC FILM COMPRISING CURED MATERIAL PREPARED BY CURING THE COMPOSITION, AND ELECTROLUMINESCENT DEVICE COMPRISING THE ORGANIC FILM**

(30) Priority: 15.04.2025 JP 2025067073; 15.01.2026 KR 20260008288
(71) Applicant: Samsung Electronics Co., Ltd., Suwon-si, Gyeonggi-do 16677 (KR); Samsung Display Co., Ltd., Yongin-si, Gyeonggi-do 17113 (KR)
(72) Inventor: KONISHI, Yusaku, Yokohama-shi, 220-0011 (JP); YAMADA, Yukika, Yokohama-shi, 220-0011 (JP); ISHII, Norihito, Yokohama-shi, 220-0011 (JP); FURUTA, Keigo, Yokohama-shi, 220-0011 (JP); ENDO, Hiroko, Yokohama-shi, 220-0011 (JP); KATO, Fumiaki, Yokohama-shi, 220-0011 (JP); MIN, Ji Hyun, 16678 Suwon-si (KR); SEO, Hong Kyu, 16678 Suwon-si (KR); KWON, Ha il, 16678 Suwon-si (KR)
(74) Representative: Elkington and Fife LLP

(57) **Abstract**

A low molecular weight compound represented by Chemical Formula (1) or Chemical Formula (2), each of which is described herein; a composition including the low molecular weight compound and a salt compound, the salt compound including a borate anion represented by Chemical Formula (3), which is described herein, and an iodonium cation; an organic film prepared by curing the composition and a carboxyl-containing compound; and an electroluminescent device including the organic film are disclosed.

## Description

### FIELD OF THE INVENTION

A low molecular weight compound, a composition comprising the low molecular weight compound, an organic film comprising a cured material prepared by curing the above composition, and an electroluminescent device including the organic film are disclosed.

### BACKGROUND OF THE INVENTION

There is a strong interest in quantum dots that cover a wide color for a RGB light source, and research and development of electroluminescence devices having the quantum dots in a light emitting layer (hereinafter, also referred to as a quantum dot electroluminescent device, or QD-LED). Such quantum dots and devices are actively progressing as a display device, e.g., a lighting device having a thin film, and/or that is lightweight or has a relatively low consumption power.

Through the use of specific aging technology, the luminous efficiency and device life of the QD-LED may be drastically improved (see, Patent Documents 1 and 2).

[Patent Document 1] Japanese Patent Application No. 2016-540357

[Patent Document 2] US Laid-open Publication No. 2024/0014358

### SUMMARY OF THE INVENTION

Through our recent efforts to enhance resistance to positive aging, reduce the driving voltage of electroluminescent devices, improve luminous efficiency, and extend the lifetime of the devices, it was found that one or more of the above technical objectives could be achieved with the presence of a low molecular weight compound represented by Chemical Formula (1) or Chemical Formula (2). 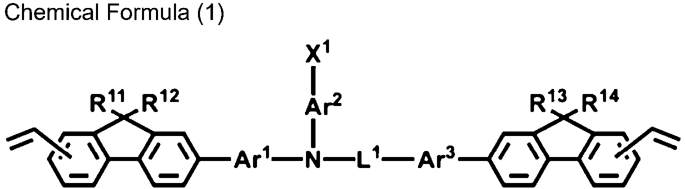 in Chemical Formula (1),
R¹¹ to R¹⁴ are, each independently, a hydrogen atom, or a hydrocarbon group having 1 to 16 carbon atoms,
Ar¹ is single bond, a substituted or unsubstituted aromatic hydrocarbon ring group, or a substituted or unsubstituted aromatic heterocyclic group,
Ar² is a substituted or unsubstituted aromatic hydrocarbon ring group, or a substituted or unsubstituted aromatic heterocyclic group,
Ar³ is a substituted or unsubstituted carbazole, a substituted or unsubstituted fluorene, a substituted or unsubstituted benzene, or a substituted or unsubstituted biphenylene group,
L¹ is single bond, or a group represented by chemical formula (1-a), 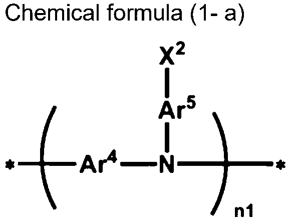
in chemical formula (1-a),
Ar⁴ and Ar⁵ are, each independently, a substituted or unsubstituted aromatic hydrocarbon ring group, or a substituted or unsubstituted aromatic heterocyclic group,
X² is a hydrogen atom, or a group selected from Group 1,
n1 is an integer of 1 or 2, provided that
when n1 is an integer, 2, each Ar⁴ are the same or different,
when n1 is an integer, 2, each Ar⁵ are the same or different,
when n1 is an integer, 2, each X² are the same or different,
* is a bonding position, and
X¹ is a hydrogen atom, or a group selected from Group 1: 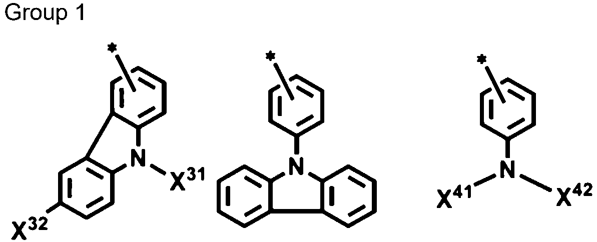
in Group 1,
X³¹ and X³² are, each independently, a hydrogen atom, a group selected from Group 2, or a combination thereof,
X⁴¹ and X⁴² are, each independently, a hydrogen atom, a group selected from Group 2, or a combination thereof, and
* is a bonding position: 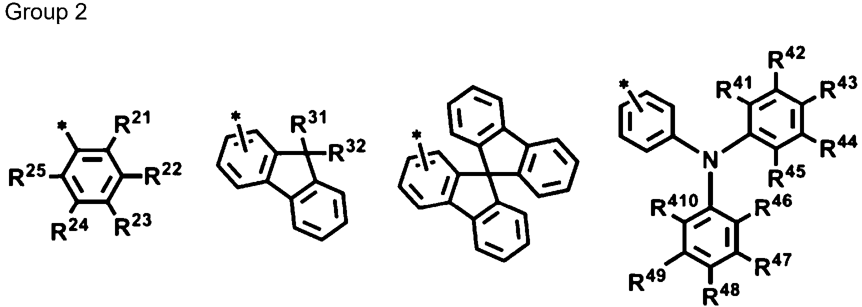
in Group 2,
R²¹ to R²⁵ are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
R³¹ and R³² are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
R⁴¹ to R⁴⁹ and R⁴¹⁰ are, each independently, a hydrogen atom, a fluorine atom, an unsubstituted linear alkyl group having 1 to 12 carbon atoms, an unsubstituted branched alkyl group having a carbon number of 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3, and
* is a bonding position: 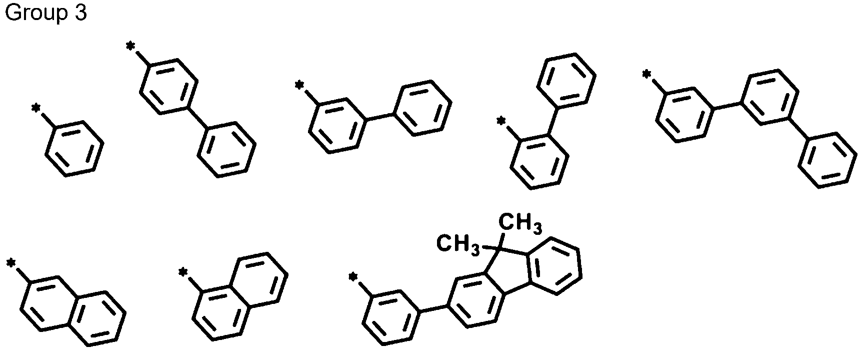
in Group 3,
* is a bonding position: 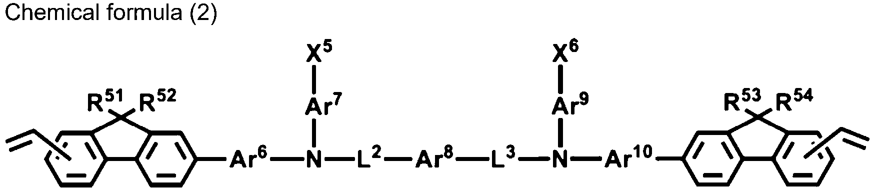
in chemical formula (2),
R⁵¹ to R⁵⁴ are, each independently, a hydrogen atom, or a hydrocarbon group having 1 to 16 carbon atoms,
Ar⁶ and Ar¹⁰ are, each independently, single bond, a substituted or unsubstituted aromatic hydrocarbon ring group, or a substituted or unsubstituted aromatic heterocyclic group,
Ar⁷ and Ar⁹ are, each independently, a substituted or unsubstituted aromatic hydrocarbon ring group, or a substituted or unsubstituted aromatic heterocyclic group,
Ar⁸ is a substituted or unsubstituted carbazole, a substituted or unsubstituted fluorene, a substituted or unsubstituted benzene, or a substituted or unsubstituted biphenyl ring,
L² is a single bond, or a group represented by chemical formula (2-a): 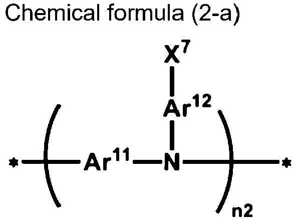
in chemical formula (2-a),
Ar¹¹ and Ar¹² are, each independently, a substituted or unsubstituted aromatic hydrocarbon ring group, or a substituted or unsubstituted aromatic heterocyclic group,
X⁷is a hydrogen atom, or a group selected from Group 1,
n2 is an integer of 1 or 2, wherein
when n2 is an integer, 2, each Ar¹¹ are the same or different,
when n2 is an integer, 2, each Ar¹² are the same or different,
when n2 is an integer, 2, each X⁷ are the same or different,
* is a bonding position, and
L³ is a single bond, or a group represented by chemical formula (2-b), 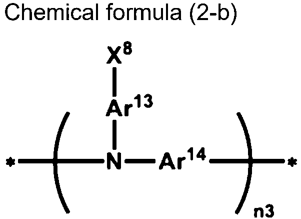
in chemical formula (2-b),
Ar¹³ and Ar¹⁴ are, each independently, a substituted or unsubstituted aromatic hydrocarbon ring group, or a substituted or unsubstituted aromatic heterocyclic group,
X⁸ is a hydrogen atom, or a group selected from Group 1,
n3 is an integer of 1 or 2, wherein
when n3 is an integer of 2, each Ar¹³ are the same or different,
when n3 is an integer of 2, each Ar¹⁴ are the same or different,
when n3 is an integer of 2, each X⁸ are the same or different,
* is a bonding position, and
X⁵ and X⁶ are, each independently, a hydrogen atom, or a group selected from Group 1.

According to the embodiment, a means is provided that can improve resistance to positive aging, reduce the driving voltage, enhance luminous efficiency, and improve the device lifespan of an electroluminescent device.

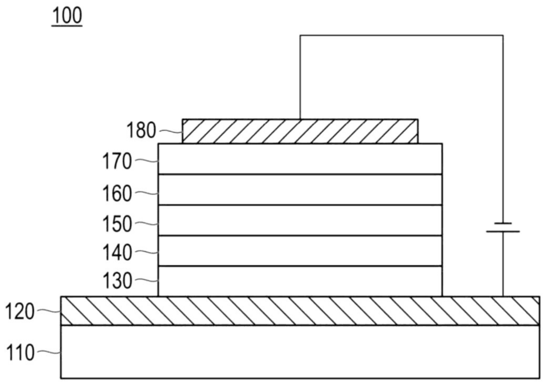

### BRIEF DESCRIPTION OF THE DRAWING

The Figure (Drawing) is a schematic representation of an electroluminescence device according to an embodiment.

### DETAILED DESCRIPTION OF THE EMBODIMENTS

In accordance with our research and our understanding through such research, we find that the hole injection layer deteriorates by formation of anions during aging (e.g., acrylic acid anions), and as a result, current density degrades (i.e., dedoping), which in turn leads to a rise in driving voltage, luminous efficiency deterioration, life of the device deterioration, etc. Accordingly, device performance improvement by positive aging and device performance deterioration due to the deterioration of hole injection layer by negative aging is a technical trade-off.

We sought out to achieve a positive aging effect, and thus, reduce the driving voltage of electroluminescent devices, improve luminous efficiency, or extend the lifetime of the devices. In order to solve the above technical objectives, we conducted a thorough examination of the degradation pathways of devices. As a result, we found that one or more of the objectives could be achieved by including a low molecular weight compound represented by Chemical Formula (1) or Chemical Formula (2).

An embodiment provides a low molecular weight compound represented by Chemical Formula (1) or Chemical Formula (2): 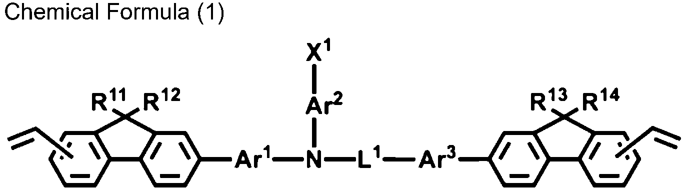 in chemical Formula (1),
R¹¹ to R¹⁴ are, each independently, a hydrogen atom, or a hydrocarbon group having 1 to 16 carbon atoms,
Ar¹ is single bond, a substituted or unsubstituted aromatic hydrocarbon ring group, or a substituted or unsubstituted aromatic heterocyclic group,
Ar² is a substituted or unsubstituted aromatic hydrocarbon ring group, or a substituted or unsubstituted aromatic heterocyclic group,
Ar³ is a substituted or unsubstituted carbazole, a substituted or unsubstituted fluorene, a substituted or unsubstituted benzene, or a substituted or unsubstituted biphenylene group,
L¹ is single bond, or a group represented by chemical formula (1-a), 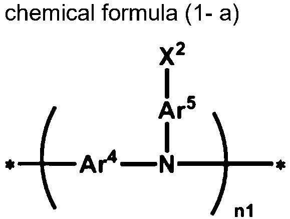
in chemical formula (1-a),
Ar⁴ and Ar⁵ are, each independently, a substituted or unsubstituted aromatic hydrocarbon ring group, or a substituted or unsubstituted aromatic heterocyclic group,
X² is a hydrogen atom, or a group selected from Group 1,
n1 is an integer of 1 or 2, provided that
when n1 is an integer, 2, each Ar⁴ are the same or different,
when n1 is an integer, 2, each Ar⁵ are the same or different,
when n1 is an integer, 2, each X² are the same or different,
* is a bonding position, and
X¹ is a hydrogen atom, or a group selected from Group 1: 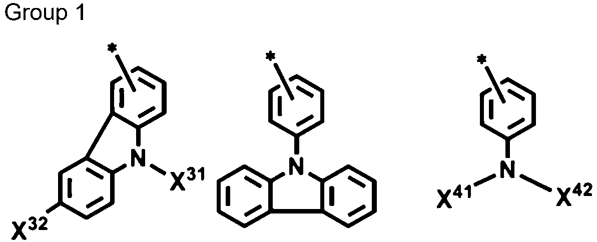
in Group 1,
X³¹ and X³² are, each independently, a hydrogen atom, a group selected from Group 2, or a combination thereof,
X⁴¹and X⁴² are, each independently, a hydrogen atom, a group selected from Group 2, or a combination thereof, and
* is a bonding position: 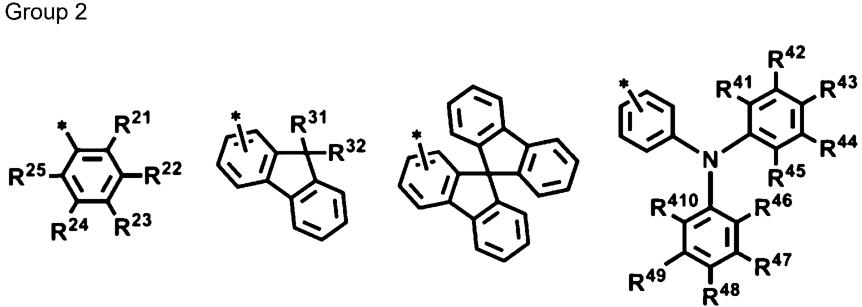
in Group 2,
R²¹ to R²⁵ are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
R³¹ and R³² are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
R⁴¹ to R⁴⁹ and R⁴¹⁰ are, each independently, a hydrogen atom, a fluorine atom, an unsubstituted linear alkyl group having 1 to 12 carbon atoms, an unsubstituted branched alkyl group having a carbon number of 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3, and
* is a bonding position: 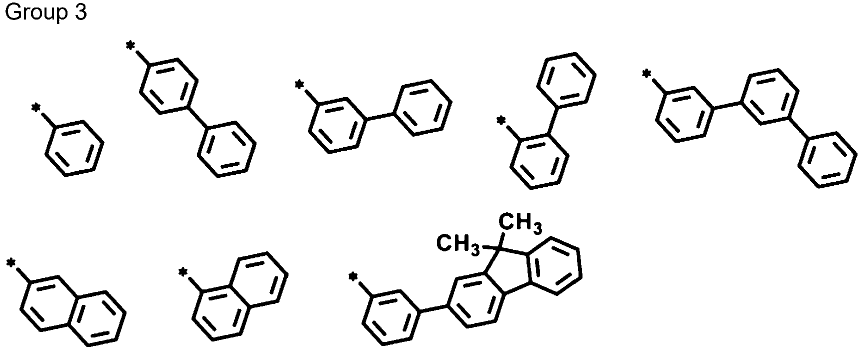
in Group 3,
* is a bonding position: 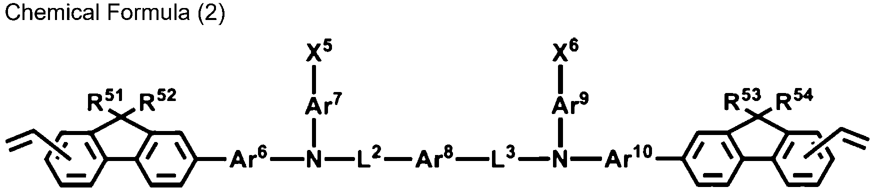
in chemical Formula (2),
R⁵¹ to R⁵⁴ are, each independently, a hydrogen atom, or a hydrocarbon group having 1 to 16 carbon atoms,
Ar⁶ and Ar¹⁰ are, each independently, single bond, a substituted or unsubstituted aromatic hydrocarbon ring group, or a substituted or unsubstituted aromatic heterocyclic group,
Ar⁷ and Ar⁹ are, each independently, a substituted or unsubstituted aromatic hydrocarbon ring group, or a substituted or unsubstituted aromatic heterocyclic group,
Ar⁸ is a substituted or unsubstituted carbazole, a substituted or unsubstituted fluorene, a substituted or unsubstituted benzene, or a substituted or unsubstituted biphenyl ring,
L² is a single bond, or a group represented by chemical formula (2-a): 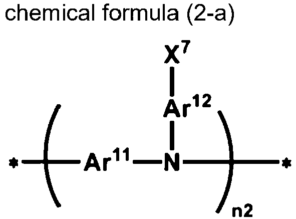
in chemical formula (2-a),
Ar¹¹ and Ar¹² are, each independently, a substituted or unsubstituted aromatic hydrocarbon ring group, or a substituted or unsubstituted aromatic heterocyclic group,
X⁷is a hydrogen atom, or a group selected from Group 1,
n2 is an integer of 1 or 2, wherein
when n2 is an integer, 2, each Ar¹¹ are the same or different,
when n2 is an integer, 2, each Ar¹² are the same or different,
when n2 is an integer, 2, each X⁷ are the same or different,
* is a bonding position, and
L³ is a single bond, or a group represented by chemical formula (2-b), 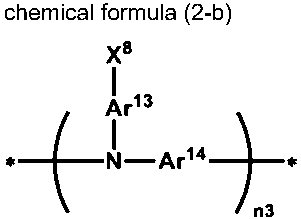
in chemical formula (2-b),
Ar¹³ and Ar¹⁴ are, each independently, a substituted or unsubstituted aromatic hydrocarbon ring group, or a substituted or unsubstituted aromatic heterocyclic group,
X⁸ is a hydrogen atom, or a group selected from Group 1,
n3 is an integer of 1 or 2, wherein
when n3 is an integer of 2, each Ar¹³ are the same or different,
when n3 is an integer of 2, each Ar¹⁴ are the same or different,
when n3 is an integer of 2, each X⁸ are the same or different,
* is a bonding position, and
X⁵ and X⁶ are, each independently, a hydrogen atom, or a group selected from Group 1.

In the context of the specification, "low molecular weight compound represented by Chemical Formula (1)" is also referred to as "low molecular weight compound (1)". Also, in the specification, "low molecular weight compound represented by Chemical Formula (2)" is also referred to as "low molecular weight compound (2)".

Another embodiment provides a composition comprising a low molecular weight compound according to an embodiment, and a salt compound including a borate anion represented by Chemical Formula (3), and an iodonium cation: 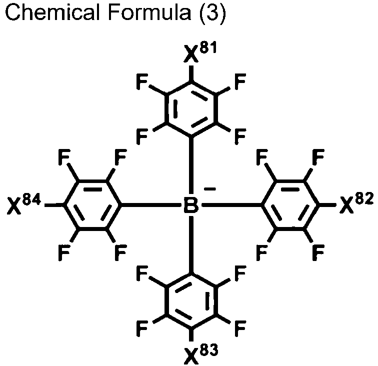 in Chemical Formula (3),
X ⁸¹ to X ⁸⁴ are each independently a hydrogen atom, a fluorine atom, or a group represented by chemical formula (3-a),

   chemical formula (3-a) * -L⁴-(Ar¹⁶)ₙ₆-(Z)ₙ₄
In chemical formula (3-a),
L⁴ is, each independently, single bond, or a group represented by chemical formula (3-b),
Ar¹⁶ is a substituted or unsubstituted aromatic hydrocarbon ring group, or a substituted or unsubstituted aromatic heterocyclic group,
Z is a fluorine atom, or a group selected from Group 4,
n4 is an integer from 1 to 5,
n6 is an integer of 0 or 1,
when there are two or more groups represented by chemical formula (3-a), each L⁴ are the same or different,
when there are two or more groups represented by chemical formula (3-a), each Ar¹⁶ are the same or different,
when there are two or more groups represented by chemical formula (3-a), each Z are the same or different,
when there are two or more groups represented by chemical formula (3-a), each n4 are the same or different,
when there are two or more groups represented by chemical formula (3-a), each n6 are the same or different,
when n4 is greater than or equal to 2, each Z are the same or different, and
* is a bonding position, 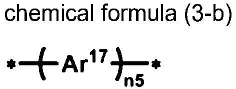
In chemical formula (3-b),
Ar¹⁷ is a substituted or unsubstituted aromatic hydrocarbon ring group, or a substituted or unsubstituted aromatic heterocyclic group,
n5 is an integer from 1 to 5, and when n5 is greater than or equal to 2, each Ar¹⁷ are the same or different, and
* is a bonding position, 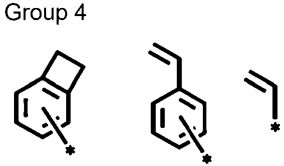
in Group 4,
* is a bonding position.

Another embodiment provides an organic film comprising a cured material obtained by curing a composition according to an embodiment, and a carboxyl group-containing compound.

Still another embodiment provides an electroluminescent device including a first electrode layer providing holes, a second electrode layer providing electrons, and an organic layer disposed between the first electrode layer and the second electrode layer, wherein at least one layer of the organic layer includes an organic thin film. In the context of the specification, the electroluminescent device is also simply referred to as "LED". Quantum dot electroluminescent device is also simply referred to as "QD-LED". Organic electroluminescent devices are also called simply "OLEDs".

The electroluminescent device may be fabricated by sequentially laminating a hole transport layer, a light-emitting layer, a charge transport layer, etc. As a method of laminating, a deposition method using a low molecular weight material, and a wet process using a polymer material are used. Among them, for example, the wet process may be used from the perspective of expansion, efficiency, and cost reduction.

The hole transport material prepared by curing the low molecular weight compound according to an embodiment may have excellent resistance to the aging treatment. The mechanism for exhibiting the effect may be best described or understood below. It is to be understood, however, that such an explanation in no way limits the reach or the scope of the claims presented.

In the hole injection layer, it is known that the hole injection characteristics may be improved by forming a charge transfer complex. If the cations and anions do not form a charge transfer complex, there remains a strong possibility that hole injection characteristics may be degraded.

Conventional hole injection materials have a relatively small difference between the ionization potential of the hole injection material included in hole injection layer and the electron affinity of borate anion (dopant) than the difference between the ionization potential of the hole injection material and electron affinity of acid anions resulting from positive aging. Accordingly, acid anions resulting from positive aging and borate anion exchange occurs, the formed charge transfer complex collapses, and the cations and acid anions that do not form charge transfer complexes combine. Therefore, the electric conductivity of hole injection layer deteriorates, and the characteristics, for example, driving voltage, luminous efficiency, device lifespan etc. may deteriorate.

Meanwhile, in a composition according to an embodiment, the composition may have a larger difference between the ionization potential of the low molecular weight compound, which is a hole injection material, and electron affinity of the borate anion (dopant) included in the salt compound than the difference between the ionization potential of the low molecular weight compound and electron affinity of the anions resulting from the positive aging, and the ionization potential of the low molecular weight compound is deeper than the ionization potential of the iodonium cation included in the salt compound. Accordingly, even though positive aging is performed, it is believed that the collapse of charge-transfer complexes of the borate anions of the salt compound and the low molecular weight compounds may be suppressed, the conductivity of the hole injection layer is maintained, and the degradation of each characteristic (e.g., driving voltage, luminous efficiency, device lifetime, etc.) may be suppressed.

In addition, the dopant may be opened (cleaved) by heat treatment and doped to the hole transport material, the opened dopant may also work as a cation polymerization initiator. Accordingly, at the dopant cleavage temperature, if a crosslinking group were to be present among the hole transport materials (that is, the curing reaction has not be completed) the crosslinking group and the opened dopant may react, and the amount of dopant that may be used in doping may reduce.

In contrast, with a low molecular weight compound according to an embodiment, the compound may have a lower curing temperature than that used in doping process, and the curing process may be completed prior to the doping process. Accordingly, it is possible to suppress the formation of additional crosslinks in the hole transport material, and thus, the obtained electroluminescent device may have reduced driving voltage, improved high luminous efficiency, and enhanced durability (luminous life), compared to those including conventional materials.

The above mechanism is based on speculation, and its correctness or incorrectness does not affect the technical scope of the present invention. Similarly, with regard to other speculative matters in this specification, their correctness or incorrectness does not affect the technical scope of the present invention.

Hereinafter, the embodiments of the present invention will be described. The embodiments are not limited to the following forms and can be variously modified within the scope of the claims. In addition, the embodiments described in this specification can be combined arbitrarily to form other embodiments. Also, each drawing is exaggerated for the convenience of explanation, and the dimensional ratios of each component in each drawing may differ from reality.

The terminology used herein is for the purpose of describing particular embodiments only and is not intended to be limiting. As used herein, the singular forms "a," "an," and "the" are intended to include the plural forms, including "at least one," unless the content clearly indicates otherwise. Therefore, reference to "an" element in a claim followed by reference to "the" element is inclusive of one element as well as a plurality of the elements.

"At least one" is not to be construed as limiting "a" or "an." "Or" means "and/or." As used herein, the term "and/or" includes any and all combinations of one or more of the associated listed items.

It will be further understood that the terms "comprise" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ±10% or ±5% of the stated value.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein. In this specification, unless otherwise specifically stated, measurements of handling and physical properties are conducted at room temperature (greater than or equal to 20°C and less than or equal to 25°C) and relative humidity between 40% RH and 50% RH.

In this specification, 'x and y are each independent' means that x and y may be the same or may be different.

In this specification, a group derived from "compound z" or a group originating from "compound z" means that when "compound z" is made into a cyclic compound, from that ring structure, the hydrogen atoms directly bonded to the ring-forming atoms are removed according to the valence to represent an unsaturated group.

In this specification, the number of ring-forming atoms refers to the number of atoms constituting the corresponding ring itself of the compound (e.g., monocyclic compound, condensed ring compound, crosslinked compound, carbocyclic compound, and heterocyclic compound) having a structure in which atoms are bonded in a ring (e.g., monocycle, condensed ring, ring assembly, etc.). Atoms that do not form a ring (e.g., a hydrogen atom that terminates the bond of the atoms forming a ring) or atoms included in a substituent when the ring is replaced by a substituent are not included in the number of ring-forming atoms. The number of ring-forming atoms described below is assumed to be the same unless otherwise specified.

For example, a benzene ring has 6 ring-forming atoms, a naphthalene ring has 10 ring-forming atoms, a pyridine ring has 6 ring-forming atoms, and a furan ring has 5 ring-forming atoms.

For example, a benzene ring has 6 ring-forming atoms, a naphthalene ring has 10 ring-forming atoms, a pyridine ring has 6 ring-forming atoms, and a furan ring has 5 ring-forming atoms.

When the benzene ring is substituted with a substituent, for example, an alkyl group, the number of carbon atoms of the alkyl group is not included in the number of ring-forming atoms of the benzene ring. Accordingly, the number of ring-forming atoms of the benzene ring substituted by the alkyl group is 6. In addition, when the naphthalene ring is substituted with an alkyl group as a substituent, for example, the number of atoms of the alkyl group is not included in the number of ring-forming atoms of the naphthalene ring. Accordingly, the number of ring-forming atoms of the naphthalene ring substituted by the alkyl group is 10.

For example, the number of hydrogen atoms bonded to the pyridine ring or the atoms constituting the substituent is not included in the number of ring-forming atoms of the pyridine ring. Accordingly, the number of ring-forming atoms of the pyridine ring to which the hydrogen atom or substituent is bonded is 6.

### <Substituent>

In the present specification, unless specifically defined, the term "substituted" refers to being substituted with an alkyl group, a cycloalkyl group, a hydroxyalkyl group, an alkoxy group, an alkoxyalkyl group, a cycloalkoxy group, an alkenyl group, an alkynyl group, a primary amino group (-NH₂), a secondary amino group -NH(R¹): R¹ is an alkyl group or an aryl group), a tertiary amino group (-N(R¹)(R²): R¹ and R² are each independently an alkyl group or an aryl group, and in this case, R¹ and R² may form a ring), an aryl group, an aryloxy group, an alkylthio group, a cycloalkylthio group, an arylthio group, an alkoxycarbonyl group, an aryloxycarbonyl group, a hydroxy group (-OH), a carboxyl group (-COOH), a thiol group (-SH), a cyano group (-CN), a halogen atom (a fluorine atom, a chlorine atom, a bromine atom, or an iodine atom), or a combination thereof. On the other hand, when a group is substituted, the form of the group which is included in the definition of a substituent does not include a form which has been further substituted with the group as a substituent. For example, when the substituent is an alkyl group, this alkyl group as a substituent is not further substituted with an alkyl group. However, in some cases, this does not apply when a hetero substituent is introduced. For example, when an alkyl group is substituted with an aromatic hydrocarbon group, the aromatic hydrocarbon group may also be substituted with an alkyl group.

Herein, the alkyl group as the substituent may be either a linear or branched alkyl group, for example a linear alkyl group having 1 to 30 carbon atoms or a branched alkyl group having 3 to 30 carbon atoms. Specifically, the alkyl group may be a methyl group, an ethyl group, an n-propyl group, an isopropyl group, an n-butyl group, an isobutyl group, a sec-butyl group, a tert-butyl group, an n-pentyl group, an isopentyl group, a tert-pentyl group, a neopentyl group, a 1,2-dimethylpropyl group, an n-hexyl group, an isohexyl group, a 1,3-dimethylbutyl group, a 1-isopropylpropyl group, a 1,2-dimethylbutyl group, an n-heptyl group, a 1,4-dimethylpentyl group, a 3-ethylpentyl group, a 2-methyl-1-isopropylpropyl group, a 1-ethyl-3-methylbutyl group, an n-octyl group, a 2-ethylhexyl group, a 3-methyl-1-isopropylbutyl group, a 2-methyl-1-isopropylbutyl group, a 1-tert-butyl-2-methylpropyl group, an n-nonyl group, a 3,5,5-trimethylhexyl group, an n-decyl group, an isodecyl group, an n-undecyl group, a 1-methyldecyl group, an n-dodecyl group, an n-tridecyl group, an n-tetradecyl group, an n-pentadecyl group, an n-hexadecyl group, an n-heptadecyl group, an n-octadecyl group, an nonadecyl group, an icosyl group, and the like..

As the substituent, the cycloalkyl group may include for example, a cyclopropyl group, a cyclobutyl group, a cyclopentyl group, and a cyclohexyl group.

As the substituent, the haloalkyl group may include for example, a fluoro-methyl group, difluoromethyl group, trifluoromethyl group, bromo-methyl group, dibromomethyl group, tribromomethyl group, iodo-methyl group, diiodomethyl group, and triiodomethyl group.

The hydroxyalkyl group may be, for example, an alkyl group that is substituted with 1 to 3 (e.g., 1 or 2, and for example 1) hydroxy groups (for example, hydroxymethyl group, hydroxyethyl group).

The alkoxy group as the substituent may be either a linear or branched alkoxy group, but desirably a linear alkoxy group having 1 to 20 carbon atoms or a branched alkoxy group having 3 to 20 carbon atoms. For example, the alkoxy group may be, for example, a methoxy group, an ethoxy group, a propoxy group, an isopropoxy group, a butoxy group, a pentyloxy group, a hexyloxy group, a heptyloxy group, an octyloxy group, a nonyloxy group, a decyloxy group, an undecyloxy group, a dodecyloxy group, a tridecyloxy group, a tetradecyloxy group, a pentadecyloxy group, a hexadecyloxy group, a heptadecyloxy group, an octadecyloxy group, a 2-ethylhexyloxy group, a 3-ethylpentyloxy group, and the like.

The hydroxyalkyl group may be, for example, an alkyl group that is substituted with 1 to 3 (e.g., 1 or 2, and for example 1) hydroxy groups (for example, hydroxymethyl group, hydroxyethyl group).

The cycloalkoxy group as a substituent may be, for example, a cyclopropyl oxy group, a cyclobutyl oxy group, a cyclopentyl oxy group, a cyclohexyl oxy group, and the like.

The alkenyl group as a substituent may include, for example, a vinyl group, an allyl group, a 1-propenyl group, an isopropenyl group, a 1-butenyl group, a 2-butenyl group, a 3-butenyl group, a 1-pentenyl group, a 2-pentenyl group, a 3-pentenyl group , a 1-hexenyl group, a 2-hexenyl group, a 3-hexenyl group, a 1-heptenyl group, a 2-heptenyl group, a 5-heptenyl group, a 1-octenyl group, a 3-octenyl group, a 5-octenyl group, and the like.

The alkynyl group as a substituent may include, for example, an acetylenyl group, a 1-propynyl group, a 2-propynyl group, a 1-butynyl group, a 2-butynyl group, a 3-butynyl group, a 1-pentetyl group, a 2-pentetyl group, a 3-pentetyl group , 1-hexynyl group, a 2-hexynyl group, a 3-hexynyl group, a 1-heptinyl group, a 2-heptinyl group, a 5-heptinyl group, a 1-octynyl group, a 3-octynyl group, a 5-octynyl group, and the like.

The secondary amino groups as a substituent may include, for example, an alkylamino group having 1 to 10 carbon atoms such as a methylamino group, an ethylamino group, an n-propylamino group, an n-butylamino group, an isobutylamino group, and the like, and a monoarylamino group such as a monophenylamino group, a mononaphthylamino group, and the like.

The tertiary amino group as a substituent may include, for example, a dialkylamino group having 2 to 20 carbon atoms, such as a dimethylamino group, a diethylamino group, a di-n-propylamino group, a di-n-butylamino group, and a methylethylamino group, and a diarylamino group, such as a diphenylamino group and a dinaphthylamino group.

An aromatic hydrocarbon ring group may represent a group derived from a hydrocarbon ring that is aromatic in whole or in part. If there are two or more aromatic hydrocarbon rings, which are partially or entirely aromatic, these rings may be bonded to each other by a single bond, or condensed. As an aromatic hydrocarbon group as a substituent, an aromatic hydrocarbon group having 6 to 30 carbon atoms may be given as an example. Examples may include a benzene ring group, a naphthalene ring group, a benzocyclobutene ring group, a biphenyl ring group, a fluorene ring group, a spiro-bifluorene ring group, an anthracene ring group, a pyrene ring group, an azulene ring group, an acenaphthylene ring group, a terphenyl ring group, and a phenanthrene ring group.

Examples of an aryl group as a substituent represents a monovalent group of the aromatic hydrocarbon ring group. For example, a phenyl group, a naphthyl group, a benzocyclobutenyl group, a biphenyl group, a fluorenyl group, a spiro-bifluoroenyl group, an anthryl group, a pyrenyl group, an azulenyl group, an acenaphthyl group, a terphenyl group, and a phenanthryl group may be included.

Examples of the aryloxy group as a substituent may include a phenoxy groups and a naphthyloxy group.

Examples of the alkylthio group as a substituent may include a methylthio group, an ethylthio group, a propylthio group, a pentylthio group, a hexylthio group, an octylthio group, and a dodecylthio group.

Examples of the cycloalkylthio group as a substituent may include a cyclopentylthio group, a cyclohexylthio group, etc.

Examples of the arylthio group as a substituent may include a phenylthio group and a naphthylthio group.

Examples of the alkoxycarbonyl group as a substituent may include a methyloxycarbonyl group, an ethyloxycarbonyl group, a butyloxycarbonyl group, an octyloxycarbonyl group, and a dodecyloxycarbonyl group.

Examples of aryloxycarbonyl groups as a substituent may include a phenyloxycarbonyl group and a naphthyloxycarbonyl group.

In addition, an aromatic heterocyclic group represents a group derived from a heterocycle having aromaticity. In this specification, a heterocycle having aromaticity represents, in part or in whole, a heterocycle having aromaticity. When a part of a heterocycle having aromaticity possesses aromaticity, the aromaticity may originate from the heterocyclic portion of the ring or from the hydrocarbon portion of the ring. An heterocycle having aromaticity may include a substituent derived from an aromatic compound that is a cyclic compound with aromaticity, not particularly limited, but for example, having one or more heteroatoms (for example, nitrogen atom (N), oxygen atom (0), phosphorus atom (P), sulfur atom (S), silicon atom (Si), selenium atom (Se), etc.) as ring-forming atoms, while the remaining ring-forming atoms are carbon atoms (C). Examples of aromatic heterocyles having 5 to 60 ring-forming atoms may include, for example, pyridine ring, pyrazine ring, pyridazine ring, pyrimidine ring, triazine ring, quinoline ring, isoquinoline ring, quinoxaline ring, quinazoline ring, naphthyridine ring, acridine ring, phenazine ring, benzoquinoline ring, benzisoquinoline ring, phenantridine ring, phenanthroline ring, benzoquinone ring, coumarin ring, fluorenone ring, furan ring, thiophene ring, benzofuran ring, benzothiophene ring, dibenzofuran ring, dibenzothiophene ring, pyrrole ring, indole ring, carbazole ring, imidazole ring, benzimidazole ring, pyrazole ring, indazole ring, oxazole ring, isoxazole ring, benzoxazole ring, benzoisoxazole ring, thiazole ring, isothiazole ring, benzothiazole ring, benzoisothiazole ring, imidazolinone ring, benzimidazolinone ring, imidazopyridine ring, imidazopyrimidine ring, azadibenzofuran ring, azacarbazole ring, azadibenzothiophene ring, diazadibenzofuran ring, diazacarbazole ring, diazadibenzothiophene ring, xanthone ring, thioxanthone ring, or a combination thereof.

In this specification, unless otherwise stated, a substituent may include positional isomers. For example, a naphthyl group may be a 1-naphthyl group and/or a 2-naphthyl group, and a biphenyl group may be at least one of (1,1'-biphenyl)-2-yl, (1,1'-biphenyl)-3-yl, and (1,1'-biphenyl)-4-yl.

In this specification, the term "carbon number" for a group that may have substituents means the number of carbons not including the carbons of the substituents, unless otherwise specifically stated.

In chemical formulas within this specification, unless otherwise defined, if a chemical bond is not shown at a position where a chemical bond should be indicated, it means that a hydrogen atom is bonded at that position.

In this specification, 'the substituent is a hydrogen atom' means that it is unsubstituted.

In this specification, the symbols accompanied by '*' (asterisk) in the text or in chemical formulas indicate a bonding site with another atom.

### Low molecular weight compound

An embodiment provides a low molecular weight compound that is represented by Chemical Formula (1) or Chemical Formula (2): 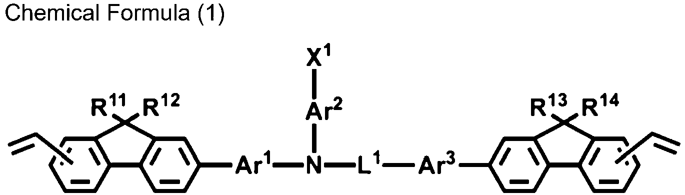 in Chemical Formula (1),
R¹¹ to R¹⁴ are each independently a hydrogen atom or a hydrocarbon group having 1 to 16 carbon atoms,
Ar¹ is single bond, a substituted or unsubstituted aromatic hydrocarbon ring group, or a substituted or unsubstituted aromatic heterocyclic group,
Ar² is a substituted or unsubstituted aromatic hydrocarbon ring group, or a substituted or unsubstituted aromatic heterocyclic group,
Ar³ is a substituted or unsubstituted carbazole, a substituted or unsubstituted fluorene, a substituted or unsubstituted benzene, or a substituted or unsubstituted biphenylene group,
L¹ is single bond, or a group represented by chemical formula (1-a), 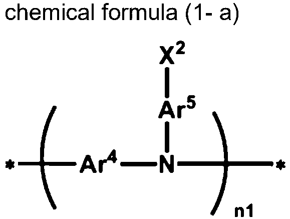
in chemical formula (1-a),
Ar⁴ and Ar⁵ are, each independently, a substituted or unsubstituted aromatic hydrocarbon ring group, or a substituted or unsubstituted aromatic heterocyclic group,
X² is a hydrogen atom, or a group selected from Group 1,
n1 is an integer of 1 or 2, provided that
when n1 is an integer, 2, each Ar⁴ are the same or different,
when n1 is an integer, 2, each Ar⁵ are the same or different,
when n1 is an integer, 2, each X² are the same or different,
* is a bonding position, and
X¹ is a hydrogen atom, or a group selected from Group 1:

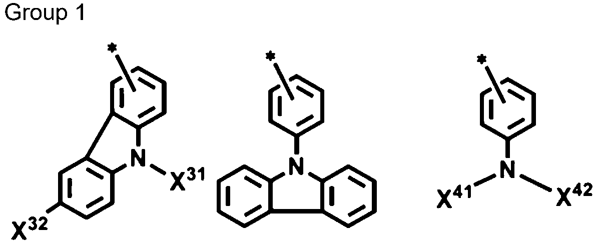

in Group 1,
X³¹ and X³² are, each independently, a hydrogen atom, a group selected from Group 2, or a combination thereof,
X⁴¹ and X⁴² are, each independently, a hydrogen atom, a group selected from Group 2, or a combination thereof, and
* is a bonding position: 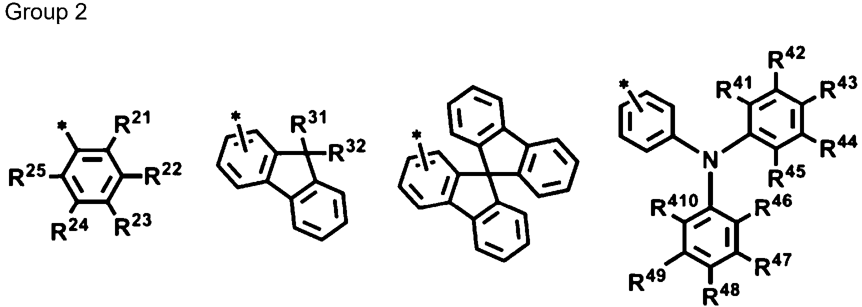
in Group 2,
R²¹ to R²⁵ are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
R³¹ and R³² are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
R⁴¹ to R⁴⁹ and R⁴¹⁰ are, each independently, a hydrogen atom, a fluorine atom, an unsubstituted linear alkyl group having 1 to 12 carbon atoms, an unsubstituted branched alkyl group having a carbon number of 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3, and * is a bonding position:

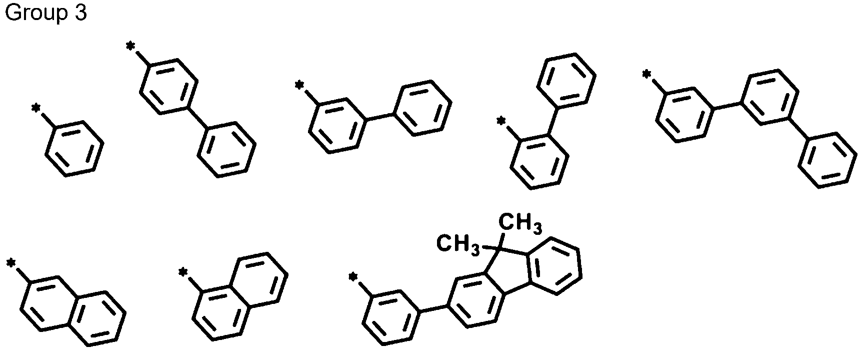

in Group 3,
* is a bonding position: 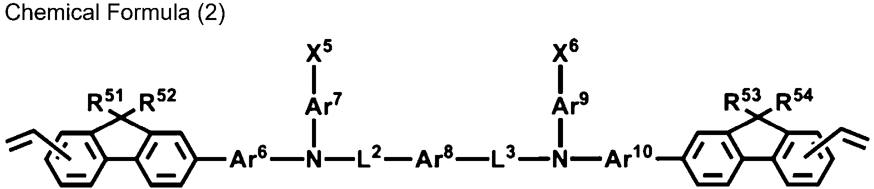
in Chemical Formula (2),
R⁵¹ to R⁵⁴ are, each independently, a hydrogen atom, or a hydrocarbon group having 1 to 16 carbon atoms,
Ar⁶ and Ar¹⁰ are, each independently, single bond, a substituted or unsubstituted aromatic hydrocarbon ring group, or a substituted or unsubstituted aromatic heterocyclic group,
Ar⁷ and Ar⁹ are, each independently, a substituted or unsubstituted aromatic hydrocarbon ring group, or a substituted or unsubstituted aromatic heterocyclic group,
Ar⁸ is a substituted or unsubstituted carbazole, a substituted or unsubstituted fluorene, a substituted or unsubstituted benzene, or a substituted or unsubstituted biphenyl ring,
L² is a single bond, or a group represented by chemical formula (2-a): 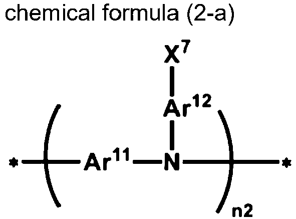
in chemical formula (2-a),
Ar¹¹ and Ar¹² are, each independently, a substituted or unsubstituted aromatic hydrocarbon ring group, or a substituted or unsubstituted aromatic heterocyclic group,
X⁷is a hydrogen atom, or a group selected from Group 1,
n2 is an integer of 1 or 2, wherein
when n2 is an integer, 2, each Ar¹¹ are the same or different,
when n2 is an integer, 2, each Ar¹² are the same or different,
when n2 is an integer, 2, each X⁷ are the same or different,
* is a bonding position, and
L³ is a single bond, or a group represented by chemical formula (2-b), 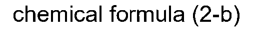 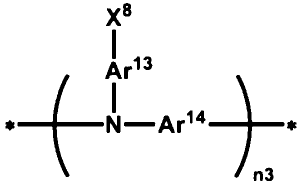
in chemical formula (2-b),
Ar¹³ and Ar¹⁴ are, each independently, a substituted or unsubstituted aromatic hydrocarbon ring group, or a substituted or unsubstituted aromatic heterocyclic group,
X⁸ is a hydrogen atom, or a group selected from Group 1,
n3 is an integer of 1 or 2, wherein
when n3 is an integer of 2, each Ar¹³ are the same or different,
when n3 is an integer of 2, each Ar¹⁴ are the same or different,
when n3 is an integer of 2, each X⁸ are the same or different,
* is a bonding position, and
X⁵ and X⁶ are, each independently, a hydrogen atom, or a group selected from Group 1.

In Chemical Formula (1), R¹¹ to R¹⁴ may, each independently, be a hydrogen atom, or a hydrocarbon group having 1 to 16 carbon atoms. Among them, R¹¹ to R¹⁴ may, each independently, be a hydrocarbon group having 1 to 16 carbon atoms, for example, 1 to 10 carbon atoms, 1 to 6 carbon atoms, 1 to 3 carbon atoms, for example, a methyl group or an ethyl group.

In Chemical Formula (1), Ar¹ may be a single bond, a substituted or unsubstituted aromatic hydrocarbon ring group, or a substituted or unsubstituted aromatic heterocyclic group. In Chemical Formula (1), Ar² may be a substituted or unsubstituted aromatic hydrocarbon ring group, or a substituted or unsubstituted aromatic heterocyclic group. Examples of aromatic hydrocarbon ring may be the same as described in the paragraph of substituents. Also, examples of aromatic heterocyclic ring group may be the same as described in the paragraph of substituents. Among them, Ar¹ and Ar² of Chemical Formula (1) may be each independently, for example, a substituted or unsubstituted aromatic hydrocarbon ring group, for example, a substituted or unsubstituted benzene ring group, a biphenyl ring group, or a fluorene ring group.

In Chemical Formula (1), Ar³ may be a substituted or unsubstituted carbazole ring group, a substituted or unsubstituted fluorene ring group, a substituted or unsubstituted benzene ring group, or a substituted or unsubstituted biphenyl ring group. Among them, Ar³ may be a substituted or unsubstituted fluorene ring group, a substituted or unsubstituted benzene ring group, or a substituted or unsubstituted biphenyl ring group.

In chemical formula (1-a), Ar⁴ and Ar⁵ may, each independently, be a substituted or unsubstituted aromatic hydrocarbon ring or a substituted or unsubstituted aromatic heterocyclic group. Examples of aromatic hydrocarbon ring group may be the same as described in the paragraph of substituents. Also, examples of aromatic heterocyclic group may be the same as those described in the paragraph of substituents . Among them, Ar⁴ and Ar⁵ of chemical formula (1-a) may, each independently, a substituted or unsubstituted aromatic hydrocarbon ring, for example, a substituted or unsubstituted benzene ring, a substituted or unsubstituted biphenyl ring, or a substituted or unsubstituted fluorenyl ring.

In Chemical Formula (1), X¹ may be a hydrogen atom, or a group listed in Group 1. In chemical formula (1-a), X² may be a hydrogen atom, or a group listed in Group 1. Among them, X¹ of Chemical Formula (1) and X² of chemical formula (1-a) may, each independently, be a hydrogen atom, or a group represented by any one in Group 1. For example, X¹ of Chemical Formula (1) and X² of chemical formula (1-a) may, each independently, a group represented in Group 1, and for example, a group represented below.

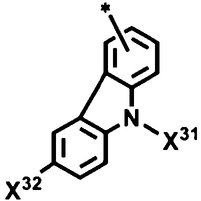

In Group 1, X³¹, X³², X⁴¹ and X⁴² may, each independently, be a hydrogen atom, a group represented in Group 2, or a combination thereof. In Group 2, R²¹ to R²⁵, R³¹ and R³², R⁴¹ to R⁴⁹, and R⁴¹⁰ may, each independently, be a hydrogen atom, a fluorine atom, an unsubstituted linear alkyl group having 1 to 12 carbon atoms, an unsubstituted branched alkyl group having 1 to 12 carbon atoms, a linear alkyl group having 1 to 12 carbon atoms and substituted by a fluorine atom, a branched alkyl group having 1 to 12 carbon atoms and substituted by a fluorine atom, or a group listed in Group 3.

In an embodiment, X³¹ may be a hydrogen atom, or a group represented by chemical formula (a):

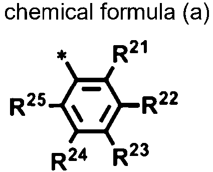

In an embodiment, X³² may be selected from Group 2, for example, a group represented by chemical formula (a):

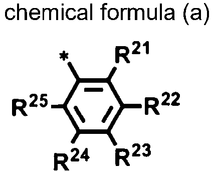

In an embodiment, X⁴¹ and X⁴² may, each independently, be selected from Group 2.

In an embodiment, both R²¹ and R²⁵ of chemical formula (a) may be hydrogen atoms. In addition, R²² to R²⁴ of the chemical formula (a) may, each independently, a hydrogen atom, a fluorine atom, or an unsubstituted linear alkyl group having 1 to 12 carbon atoms, or an unsubstituted branched alkyl group having 1 to 12 carbon atoms, for example, a hydrogen atom, a fluorine atom, an unsubstituted linear alkyl group having 3 to 8 carbon atoms, or an unsubstituted branched alkyl group having 3 to 8 carbon atoms.

In an embodiment, R³¹ and R³² of Group 2 may, each independently, be an unsubstituted linear alkyl group having 1 to 12 carbon atoms, an unsubstituted branched alkyl group having 1 to 12 carbon atoms.

In an embodiment, R⁴¹, R⁴⁵, R⁴⁶, and R⁴¹⁰ of Group 2 may all be hydrogen atoms. In addition, R⁴² to R⁴⁴ and R⁴⁷ to R⁴⁹ may, each independently, be a hydrogen atom, a fluorine atom, an unsubstituted linear alkyl group having 1 to 12 carbon atoms, or an unsubstituted branched alkyl group having 1 to 12 carbon atoms, for example, a hydrogen atom, a fluorine atom, an unsubstituted linear alkyl group having 3 to 8 carbon atoms, or an unsubstituted branched alkyl group having 3 to 8 carbon atoms.

In an embodiment, X¹ of chemical formula (1) and X² of chemical formula (1-a) may, each independently, be a hydrogen atom, or a group represented by Group (1-1):

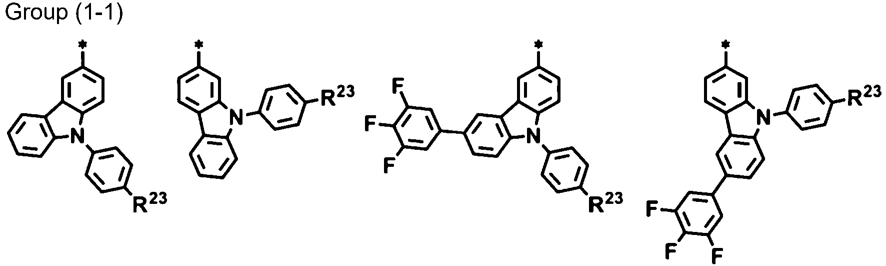

In the structures shown in Group (1-1), R²³ may be the same as in Group 2. That is, R²³ may be as hydrogen atom, a fluorine atom, an unsubstituted linear alkyl group having 1 to 12 carbon atoms, an unsubstituted branched alkyl group having 1 to 12 carbon atoms, a linear alkyl group having 1 to 12 carbon atoms and substituted by a fluorine atom, a branched alkyl group having 1 to 12 carbon atoms and substituted by a fluorine atom, or a group listed in Group 3. Alternatively, for example, R²³ may be a hydrogen atom, an unsubstituted linear alkyl group having 1 to 12 carbon atoms, or an unsubstituted branched alkyl group having 1 to 12 carbon atoms, for example, an unsubstituted linear alkyl group having 3 to 8 carbon atoms, or unsubstituted branched alkyl group having 3 to 8 carbon atoms.

In chemical formula (1-a), n1 may be 1 or 2. When n1 is 2, each Ar⁴ may be the same or different, each Ar⁵ may be the same or different, each X² may be the same or different.

In Chemical Formula (2), R⁵¹ to R⁵⁴ may, each independently, be a hydrogen atom, or a hydrocarbon group having 1 to 16 carbon atoms. Among them, R⁵¹ to R⁵⁴ may, each independently, be a hydrocarbon group having 1 to 16 carbon atoms, for example, a hydrocarbon group having 1 to 10 carbon atoms, a hydrocarbon group having 1 to 6 carbon atoms, a hydrocarbon group having 1 to 3 carbon atoms, or for example, a methyl group or an ethyl group.

In Chemical Formula (2), Ar⁶ and Ar¹⁰ may, each independently, be a single bond, a substituted or unsubstituted aromatic hydrocarbon ring group, or a substitution or unsubstituted aromatic heterocyclic group. In chemical formula (2), Ar⁷ and Ar⁹ may, each independently, be a substituted or unsubstituted aromatic hydrocarbon ring group, or a substituted or unsubstituted aromatic heterocyclic group. Examples of the aromatic hydrocarbon ring group may be the same as those specified in the <substituent>. Also, examples of the aromatic heterocyclic group may be the same as those described in the <substituent>. Among them, Ar⁷ and Ar⁹ of chemical formula (2) may, each independently, be a substituted or unsubstituted aromatic hydrocarbon ring group, for example, a substituted or unsubstituted benzene ring, a substituted or unsubstituted biphenyl ring, or a substituted or unsubstituted fluorene ring. In one embodiment, Ar⁶ and Ar¹⁰ of Chemical Formula (2) may, each independently, be a single bond.

In Chemical Formula (2), Ar⁸ may be a substituted or unsubstituted carbazole ring, a substituted or unsubstituted fluorene ring, substituted or unsubstituted benzene ring, or substituted or unsubstituted biphenyl ring. Among them, Ar⁸ may be, for example, substituted or unsubstituted fluorene ring, substituted or unsubstituted benzene ring, or substituted or unsubstituted a biphenyl ring.

In Chemical Formula (2), L² may be a single bond, or a group represented by chemical formula (2-a). For example, L² may be a single bond. If L² is a group represented by chemical formula (2-a), in chemical formula (2-a), Ar¹¹ and Ar¹² may, each independently, be a substituted or unsubstituted aromatic hydrocarbon ring, or a substituted or unsubstituted aromatic heterocyclic group. In Ar¹¹ and Ar¹², examples of the hydrocarbon ring group may be the same as those described in the <substituent>. Also, examples of the aromatic heterocyclic group may be the same as those described in the <substituent>. Among them, Ar¹¹ and Ar¹² of chemical formula (2-a) may, each independently, be, for example, a substituted or unsubstituted aromatic hydrocarbon ring, for example, a substituted or unsubstituted benzene ring, a substituted or unsubstituted biphenyl ring, or a substituted or unsubstituted fluorene ring.

Also, in chemical formula (2-a), X⁷ may be a hydrogen atom, or any of those listed in Group 1. Regarding X⁷, the same descriptions as of X¹ and X² may apply.

In chemical formula (2-a), n2 may be an integer 1 or 2. When n2 is 2, each Ar¹¹ may be the same or different, and when n2 is 2, each Ar¹² may be the same or different, and when n2 is 2, each X⁷ may be the same or different.

In Chemical Formula (2), L³ may be a single bond, or a group represented by chemical formula (2-b). For example, L³ may be a single bond. When L³ is a group represented by chemical formula (2-b), in chemical formula (2-b), Ar¹³ and Ar¹⁴ may, each independently, be a substituted or unsubstituted aromatic hydrocarbon ring, or a substituted or unsubstituted aromatic heterocyclic group. In Ar¹³ and Ar¹⁴, examples of the aromatic hydrocarbon ring may be the same as those described in the paragraph of substituents. Also, examples of the aromatic heterocyclic group may be the same as those described in the paragraph of substituents. Among them, in chemical formula (2-b), Ar¹³ and Ar¹⁴ may, each independently, be a substituted or unsubstituted aromatic hydrocarbon ring, for example, a substituted or unsubstituted benzene ring, a substituted or unsubstituted biphenyl ring, or a substituted or unsubstituted fluorene ring.

In chemical formula (2-b), X⁸ may be a hydrogen atom, or a group listed in Group 1. Regarding X⁸, the same descriptions as of X¹ and X² may apply.

In chemical formula (2-b), n3 may be an integer, 1 or 2. When n3 is 2, each Ar¹³ may be the same or different, when n3 is 2, each Ar ¹⁴ may be the same or different, and when n3 is 2, each X ⁸ may be the same or different.

In an embodiment, the low molecular weight compound according to the Chemical Formula (1) or Chemical Formula (2) may be any one listed below.

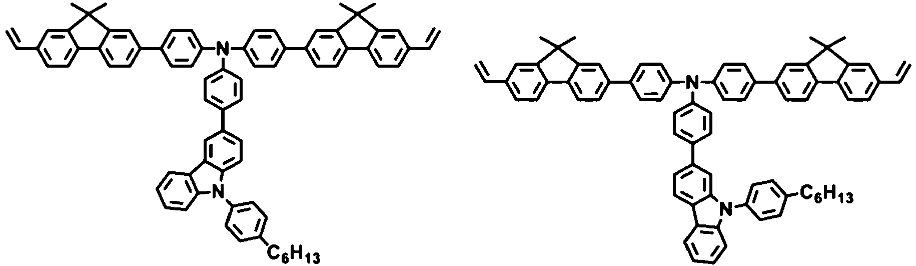

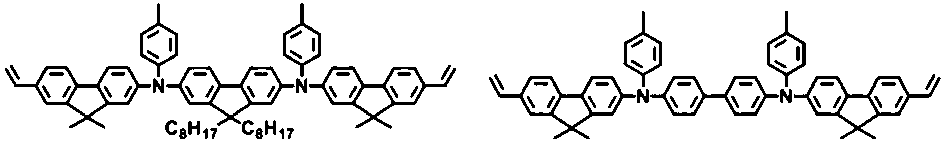

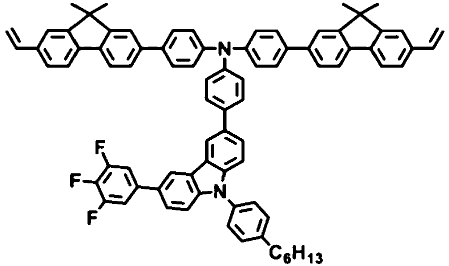

The method for confirming the structure of the low molecular weight compound according to an embodiment is not particularly limited. The structure of a low molecular weight compound according to an embodiment may be confirmed, for example, by known methods (for example, NMR, LC-MS, etc.).

Ionization potential of the low molecular weight compound, in terms of the difference from the electron affinity of the borate anion being within a specific range and from the perspective of further improving resistance to positive aging, for example, may be greater than or equal to about 4.80 eV and less than or equal to about 5.60 eV. For example, ionization potential of the low molecular weight compound may be greater than or equal to about 4.90 eV and less than or equal to about 5.50 eV, for example, greater than or equal to about 5.00 eV and less than or equal to about 5.40 eV. The ionization potential may be controlled by the adjustment of the HOMO level of the low molecular weight compound through the introduction of electron-donating groups. In addition, the ionization potential may be calculated from molecular orbital calculations, and can, for example, be calculated by the method described in the examples.

### Composition

According to another embodiment, a composition including the low molecular weight compound according to an embodiment, and a salt compound including a borate anion represented by Chemical Formula (3) and an iodonium cation is provided: 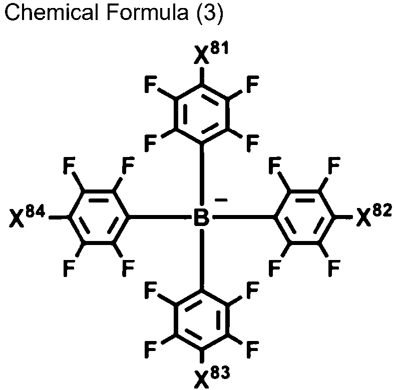 in Chemical Formula (3),
X⁸¹ to X⁸⁴ are, each independently, a hydrogen atom, a fluorine atom, or a group represented by chemical formula (3-a):

   chemical Formula (3-a) *-L⁴-(Ar¹⁶)ₙ₆-(Z)ₙ₄
in chemical formula (3-a),
L⁴ is, each independently, a single bond, or a group represented by the chemical formula (3-b),
Ar¹⁶ is a substituted or unsubstituted aromatic hydrocarbon ring group, or substituted or unsubstituted aromatic heterocyclic group,
Z is a fluorine atom, or a group represented in Group 4,
n4 is an integer from 1 to 5, and
n6 is an integer of 0 or 1, wherein,
if the group represented by chemical formula (3-a) exists in each, then, each L⁴ are the same or different,
if the group represented by chemical formula (3-a) exists in each, then, each Ar¹⁶ are the same or different,
if the group represented by chemical formula (3-a) exists in each, then, each Z are the same or different,
if the group represented by chemical formula (3-a) exists in each, then, each n4 are the same or different,
if the group represented by chemical formula (3-a) exists in each, then, each n6 are the same or different,
if n4 is greater than or equal to 2, then, each Z are the same or different, and
* is a bonding position, 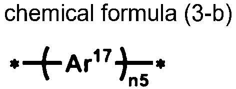
in chemical formula (3-b),
Ar¹⁷ is a substituted or unsubstituted aromatic hydrocarbon ring group, or a substituted or unsubstituted aromatic heterocyclic group,
n5 is an integer from 1 to 5, and if n5 is 2 greater than or equal to 2, then, each Ar¹⁷ are the same or different, and
* is a bonding position, 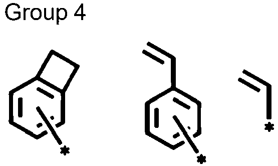
in Group 4,
* is a bonding position.

In Chemical Formula (3), X⁸¹ to X⁸⁴ are, each independently, a hydrogen atom, a fluorine atom, or a compound represented by chemical formula (3-a). Among them, X⁸¹ to X⁸⁴ may, each independently, be a fluorine atom, or a compound represented by chemical formula (3-a).

In chemical formula (3-a), L⁴ may be a single bond, or a group represented by chemical formula (3-b). If the group represented by chemical formula (3-a) exists in each, then, each L⁴ may be the same or different. In chemical formula (3-b), Ar¹⁷ may be a substituted or unsubstituted aromatic hydrocarbon ring group, or a substituted or unsubstituted aromatic heterocyclic group. Among them, Ar¹⁷ of chemical formula (3-b) may be, for example, a substituted or unsubstituted aromatic hydrocarbon ring group, for example, an aromatic hydrocarbon ring group substituted by a fluorine atom. The aromatic hydrocarbon ring group may be, for example, any one of benzene ring group or a fluorene ring group, or for example, may be a benzene ring group.

In chemical formula (3-a), Ar¹⁶ may be a substituted or unsubstituted aromatic hydrocarbon ring group, or substituted or unsubstituted aromatic heterocyclic group. If the group represented by chemical formula (3-a) exists in each, then, each Ar¹⁶ may be the same or different. Among them, Ar¹⁶ may be, for example, a substituted or unsubstituted aromatic hydrocarbon ring group, for example, an aromatic hydrocarbon ring group substituted by a fluorine atom. The aromatic hydrocarbon ring group may be, for example, any one of benzene ring group or a fluorene ring group, or for example, may be a benzene ring group.

In chemical formula (3-a), Z may be a fluorine atom, or a group represented in Group 4. If the group represented by chemical formula (3-a) exists in each, then, each Z may be the same or different. Among them, in view of the resistance to positive aging, Z may be the group represented in Group 4.

In chemical formula (3-a), n4 is an integer from 1 to 5. If the group represented by chemical formula (3-a) exists in each, then, each n4 may be the same or different. In an embodiment, n4 may be a integer of 1 or 2, for example, may be 1.

In chemical formula (3-a), n6 may be an integer of 0 or 1. If the group represented by chemical formula (3-a) exists in each, then, each n6 may be the same or different. In an embodiment, n6 may be an integer of 0 or 1, for example, n6 may be 0, or may be 1.

In chemical formula (3-a), if L⁴ is a single bond and n6 is 0, then, n4 is 1, and Z may be directly connected to the carbon atom of the benzene ring of chemical formula (3).

In chemical formula (3-b), n5 is an integer from 1 to 5. In an embodiment, n5 may be an integer of 1 or 2, or for example, may be 1.

The borate anion may include at least one selected from the anions represented below, and is not limited thereto.

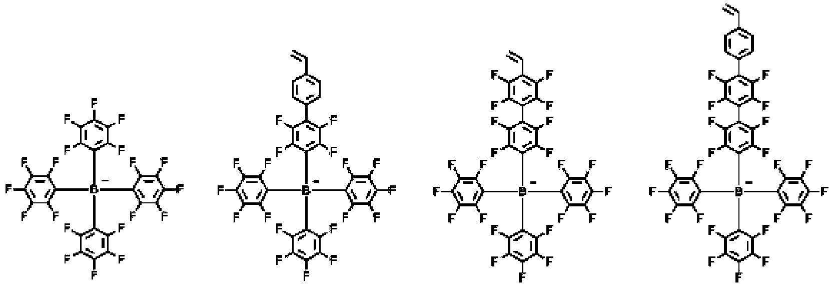

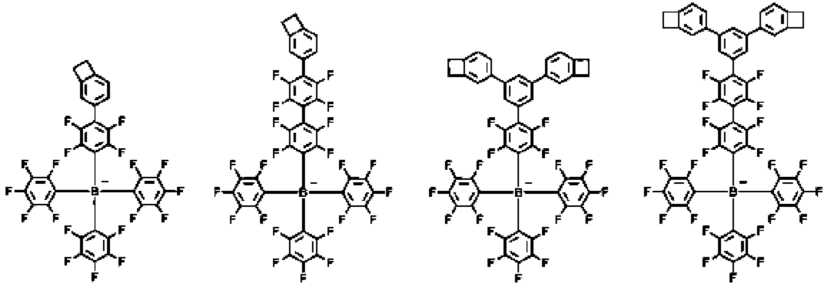

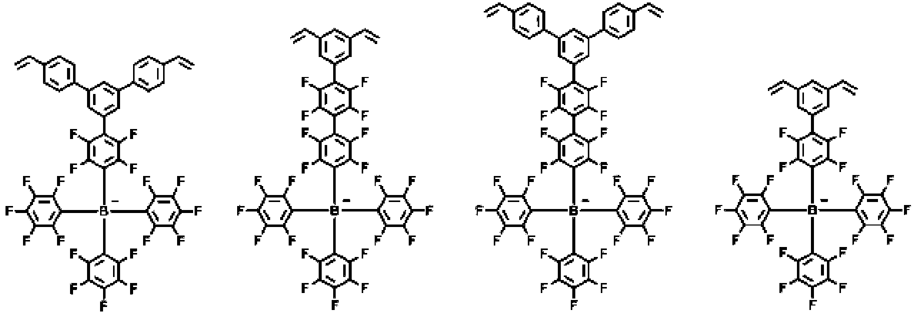

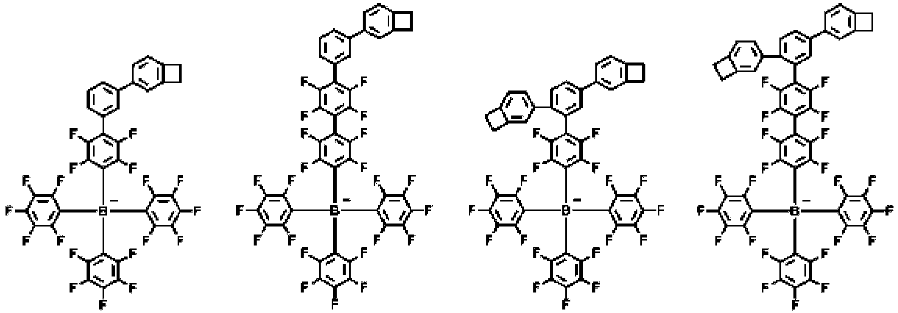

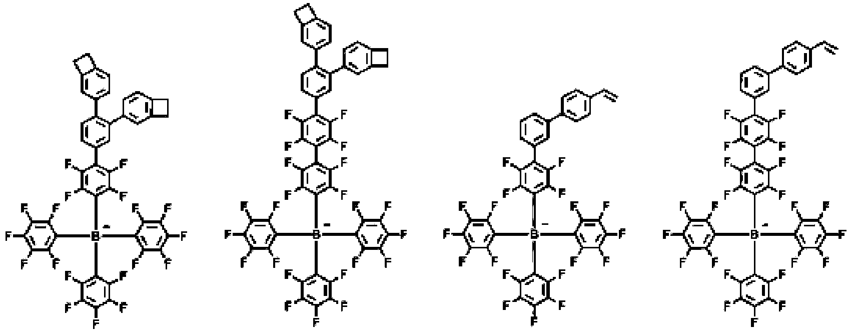

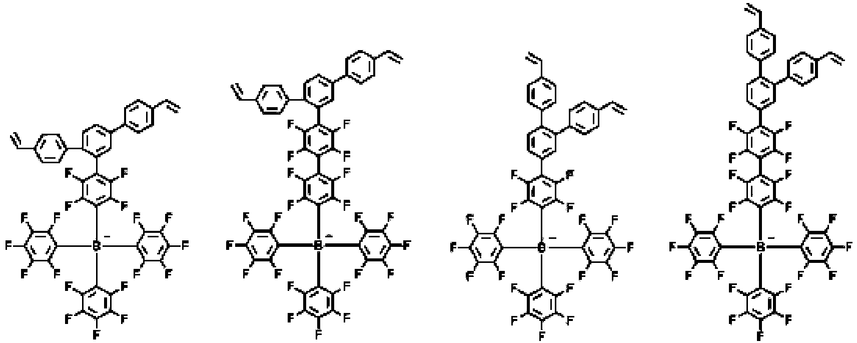

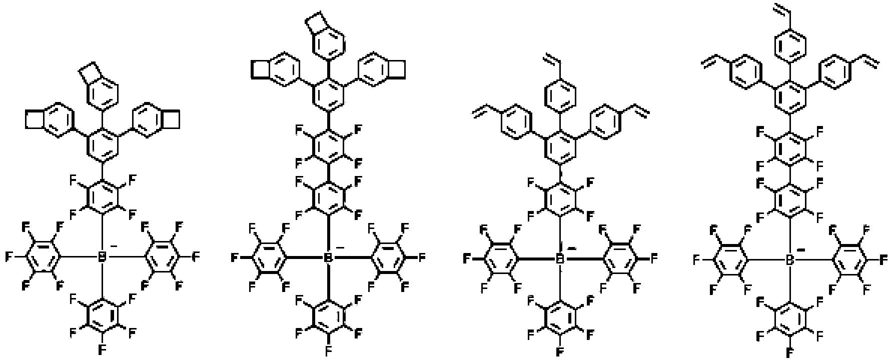

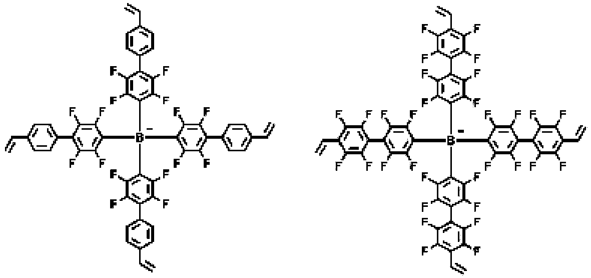

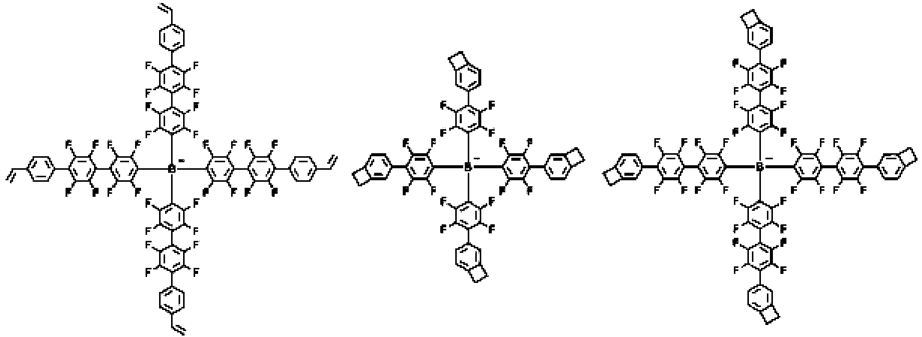

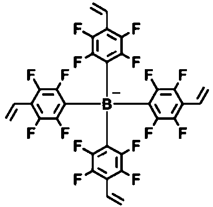

The borate anion may be included alone, or in combination of two or more types.

Among the borate anions, for example, the borate anion may be represented by the anions represented below.

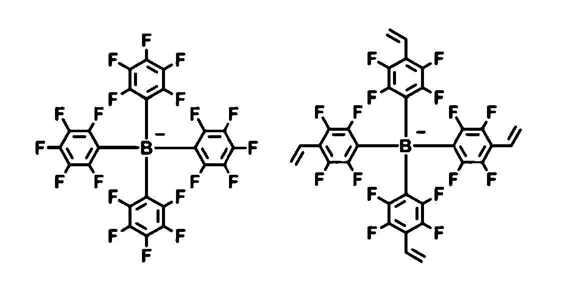

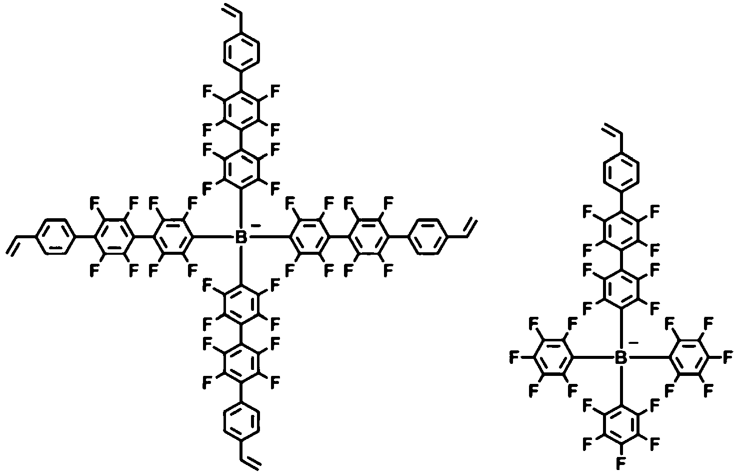

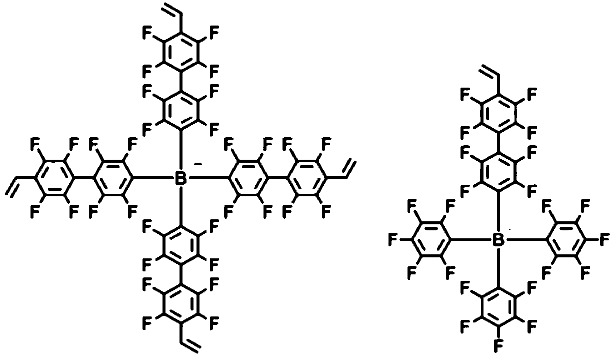

Electron affinity of a borate anion according to an embodiment may be, in terms of difference from the electron affinity of the low molecular weight compound being in a specific range and from the perspective of further improving resistance to positive aging, for example, of greater than or equal to about 5.50 eV and less than or equal to about 6.50 eV, for example, greater than or equal to about 5.60 eV and less than or equal to about 6.40 eV, or for example, greater than or equal to about 5.70 eV and less than or equal to about 6.30 eV. The electron affinity may be controlled by adjusting the LUMO level of the borate anion through the introduction of electron-withdrawing groups. In addition, the electron affinity may be calculated from molecular orbital calculations, and, for example, may be calculated by the method described in the examples.

In an embodiment, the energy difference (ΔE) between the ionization potential of the low molecular weight compound and the electron affinity of the borate anion may be greater than or equal to 0.75 eV, and the ionization potential of the low molecular weight compound may be at a deeper level than the ionization potential of the iodonium cation of the salt compound. If the energy difference (ΔE) is less than 0.75 eV, anion exchange of the acid occurring due to positive aging and the borate anion occurs, and the charge transfer complex does not form to lose conductivity of the electroluminescent device. Also, if the ionization potential of the low molecular weight compound is shallower than or equal to the ionization potential of the iodonium cation, then, doping does not occur and the charge transfer complex may not be formed. The energy difference (ΔE) may be greater than or equal to 0.75 eV, for example, greater than or equal to 0.80 eV. The bigger the energy difference (ΔE) is the better, but usually may be less than or equal to 1.50 eV.

The salt compound according to an embodiment includes the borate anion, and iodonium cation that is a counter ion of the borate anion.

Examples of the iodonium cation may include, for example, difuryliodonium cation, dithienyliodonium cation, bis(4,5-dimethyl-2-furyl) iodonium cation, bis(5-chloro-2-thienyl) iodonium cation, bis (5-cyano-2-furyl) iodonium cation, bis(5-nitro-2-thienyl) iodonium cation, bis(5-acetyl-2-furyl) iodonium cation, bis(5-carboxy-2-thienyl) iodonium cation, bis(5-methoxycarbonyl-2-furyl) iodonium cation, bis(5-phenyl-2-furyl) iodonium cation, bis(5-(p-methoxyphenyl)-2-thienyl) iodonium cation, bis(5-vinyl-2-furyl) iodonium cation, bis(5-ethynyl-2-thienyl) iodonium cation, bis(5-cyclohexyl-2-furyl) iodonium cation, bis (5-hydroxy-2-thienyl) iodonium cation, bis(5-phenoxy-2-furyl) iodonium cation, bis(5-mercapto-2-thienyl) iodonium cation, bis(5-butylthio-2-thienyl) iodonium cation, bis(5-phenylthio-2-thienyl) iodonium cation, diphenyliodonium cation, bis(p-tolyl)iodonium cation, bis(p-octylphenyl) iodonium cation, bis(p-octadecylphenyl) iodonium cation, bis(p-octyloxyphenyl) iodonium cation, bis(p-octadecyloxyphenyl) iodonium cation, phenyl(p-octadecyloxyphenyl) iodonium cation, 4-isopropyl-4'-methyldiphenyliodonium cation, (4-isobutylphenyl)-p-tolyliodonium cation, bis(1-naphthyl) iodonium cation, bis(4-phenylsulfanylphenyl) iodonium cation, phenyl(6-benzoyl- 9-ethyl- 9H-carbazol-3-yl) iodonium cation, (7-methoxy-2-oxo-2H-chromen-3-yl)-4'-isopropylphenyliodonium cation, and the like, and are not limited thereto.

The iodonium cation may be included alone, or may be used in combination of two or more types.

Among the iodonium cations, diphenyliodonium cation, 4-isopropyl-4'-methyldiphenyliodonium cation, bis(p-tolyl) iodonium cation, and (4-isobutylphenyl)(4'-tolyl) iodonium cation may be used.

As long as the ionization potential of the low molecular weight compound is deeper than that of the iodonium cation, the value of the ionization potential of the iodonium cation is not specifically limited. The ionization potential of the iodonium cation may be, for example, greater than or equal to about 4.00 eV and less than or equal to about 4.95 eV, for example, greater than or equal to about 4.10 eV and less than or equal to about 4.90 eV, or for example, greater than or equal to about 4.20 eV and less than or equal to about 4.85 eV. The ionization potential of the iodonium cation may be calculated from the molecular orbital calculations, or, for example, may be calculated by the method described in the examples.

The salt compound according to an embodiment may further include another cation, in addition to the iodonium cation. The other cations may include, for example, an onium cation, such as, for example, sulfonium cation, sulfoxonium cation, phosphonium cation, and the like.

Examples of the sulfonium cation may include, for example, dimethylphenylsulfonium cation, dimethyl(o-fluorophenyl) sulfonium cation, dimethyl (m-chlorophenyl) sulfonium cation, dimethyl (p-bromophenyl) sulfonium cation, dimethyl(p-cyanophenyl) sulfonium cation, dimethyl (m-nitrophenyl) sulfonium cation, dimethyl(2,4,6-tribromophenyl) sulfonium cation, dimethyl (pentafluorophenyl) sulfonium cation, dimethyl(p-(trifluoromethyl) phenyl) sulfonium cation, dimethyl (p-hydroxyphenyl) sulfonium cation, dimethyl(p-mercaptophenyl) sulfonium cation, dimethyl(p- methylsulfinylphenyl) sulfonium cation, dimethyl(p-methylsulfonylphenyl) sulfonium cation, dimethyl(o-acetylphenyl) sulfonium cation, dimethyl(o-benzoylphenyl) sulfonium cation, dimethyl (p-methylphenyl) sulfonium cation, dimethyl(p-isopropylphenyl) sulfonium cation, dimethyl(p-octadecylphenyl) sulfonium cation, dimethyl(p-cyclohexylphenyl) sulfonium cation, dimethyl(p-methoxyphenyl) sulfonium cation, dimethyl (o-methoxycarbonylphenyl) sulfonium cation, dimethyl(p-phenylsulfanylphenyl) sulfonium cation, (7-methoxy-2-oxo- 2H-chromen-4-yl) dimethylsulfonium cation, (4-methoxynaphthalen-1-yl) dimethylsulfonium cation, dimethyl(p-isopropoxycarbonylphenyl) sulfonium cation, dimethyl(2-naphthyl) sulfonium cation, dimethyl(9-anthryl) sulfonium cation, diethylphenylsulfonium cation, methylethylphenylsulfonium cation, methyldiphenylsulfonium cation, triphenylsulfonium cation, diisopropylphenylsulfonium cation, diphenyl(4-phenylsulfanyl-phenyl) sulfonium cation, 4,4'-bis(diphenylsulfonium) diphenylsulfide cation, 4,4'-bis[di[(4-(2-hydroxy-ethoxy)-phenyl)] sulfonium diphenylsulfide cation, 4,4'-bis(diphenylsulfonium) biphenylene cation, diphenyl(o-fluorophenyl) sulfonium cation, diphenyl(m-chlorophenyl) sulfonium cation, diphenyl(p-bromophenyl) sulfonium cation, diphenyl(p-cyanophenyl) sulfonium cation, diphenyl(m-nitrophenyl) sulfonium cation, diphenyl(2,4,6-tribromophenyl) sulfonium cation, diphenyl(pentafluorophenyl) sulfonium cation, diphenyl(p-(trifluoromethyl) phenyl) sulfonium cation, diphenyl(p-hydroxyphenyl) sulfonium cation, diphenyl(p-mercaptophenyl) sulfonium cation, diphenyl(p-methylsulfinylphenyl) sulfonium cation, diphenyl(p-methylsulfonylphenyl) sulfonium cation, diphenyl(o-acetylphenyl) sulfonium cation, diphenyl(o-benzoylphenyl) sulfonium cation, diphenyl(p-methylphenyl) sulfonium cation, diphenyl(p-isopropylphenyl) sulfonium cation, diphenyl(p-octadecylphenyl) sulfonium cation, diphenyl(p-cyclohexylphenyl) sulfonium cation, diphenyl(p-methoxyphenyl) sulfonium cation, diphenyl(o-methoxycarbonylphenyl) sulfonium cation, diphenyl(p-phenylsulfanylphenyl) sulfonium cation, (7-methoxy-2-oxo-2H-chromen-4-yl) diphenylsulfonium cation, (4-methoxynaphthalen-1-yl) diphenylsulfonium cation, diphenyl(p-isopropoxycarbonylphenyl) sulfonium cation, diphenyl(2-naphthyl) sulfonium cation, diphenyl(9-anthryl) sulfonium cation, ethyldiphenylsulfonium cation, methylethyl(o-tolyl) sulfonium cation, methyldi(p-tolyl) sulfonium cation, tri (p-tolyl) sulfonium cation, diisopropyl(4-phenylsulfanylphenyl) sulfonium cation, diphenyl(2-thienyl) sulfonium cation, diphenyl(2-furyl) sulfonium cation, diphenyl(9-ethyl-9Hcarbazol-3-yl) sulfonium cation, and the like, but are not limited thereto.

Examples of sulfoxonium cation include, for example, dimethylphenylsulfoxonium cation, dimethyl(o-fluorophenyl) sulfoxonium cation, dimethyl(m-chlorophenyl) sulfoxonium cation, dimethyl(p-bromophenyl) sulfoxonium cation, dimethyl(p-cyanophenyl) sulfoxonium cation, dimethyl(m-nitrophenyl) sulfoxonium cation , dimethyl(2,4,6-tribromophenyl) sulfoxonium cation, dimethyl(pentafluorophenyl) sulfoxonium cation, dimethyl(p-(trifluoromethyl)phenyl) sulfoxonium cation, dimethyl(p-hydroxyphenyl) sulfoxonium cation, dimethyl(p-mercaptophenyl) sulfoxonium cation, dimethyl(p-methylsulfinylphenyl) sulfoxonium cation, dimethyl(p-methylsulfonylphenyl) sulfoxonium cation, dimethyl(o-acetylphenyl) sulfoxonium cation, dimethyl(o-benzoylphenyl) sulfoxonium cation, dimethyl(p-methylphenyl) sulfoxonium cation, dimethyl (p-isopropylphenyl) sulfoxonium cation, dimethyl(p-octadecylphenyl) sulfoxonium cation, dimethyl(p-cyclohexylphenyl) sulfoxonium cation, dimethyl(p-methoxyphenyl) sulfoxonium cation, dimethyl(o-methoxycarbonylphenyl) sulfoxonium cation, dimethyl(p-phenylsulfanylphenyl) sulfoxonium cation, (7-methoxy-2-oxo-2H-chromen-4-yl) dimethylsulfoxonium cation, (4-methoxynaphthalen-1-yl) dimethylsulfoxonium cation, dimethyl(p-isopropoxycarbonylphenyl) sulfoxonium cation, dimethyl(2-naphthyl) sulfoxonium cation, dimethyl(9-anthryl) sulfoxonium cation, diethylphenyl sulfoxonium cation, methylethylphenyl sulfoxonium cation, methyldiphenyl sulfoxonium cation, triphenyl sulfoxonium cation, diisopropylphenyl sulfoxonium cation, diphenyl (4-phenylsulfanyl-phenyl)-sulfoxonium cation, 4,4'-bis(diphenyl sulfoxonium) diphenyl sulfide, 4,4'-bis[di[(4-(2-hydroxy-ethoxy)-phenyl)] sulfoxonium]diphenyl sulfide, 4,4'-bis(diphenylsulfoxonium) biphenylene, diphenyl(o-fluorophenyl) sulfoxonium cation, diphenyl(m-chlorophenyl) sulfoxonium cation, diphenyl(p-bromophenyl) sulfoxonium cation, diphenyl (p-cyanophenyl) sulfoxonium cation, diphenyl(m-nitrophenyl) sulfoxonium cation, diphenyl(2,4,6-tribromophenyl) sulfoxonium cation, diphenyl(pentafluorophenyl) sulfoxonium cation, diphenyl(p-(trifluoromethyl)phenyl) sulfoxonium cation, diphenyl(p-hydroxyphenyl) sulfoxonium cation, diphenyl(p-mercaptophenyl) sulfoxonium cation, diphenyl(p-methylsulfinylphenyl) sulfoxonium cation, diphenyl(p-methylsulfonylphenyl) sulfoxonium cation, diphenyl (o-acetylphenyl) sulfoxonium cation, diphenyl(o-benzoylphenyl) sulfoxonium cation, diphenyl (p-methylphenyl) sulfoxonium cation, diphenyl(p-isopropylphenyl) sulfoxonium cation, diphenyl(p-octadecylphenyl) sulfoxonium cation, diphenyl(p-cyclohexylphenyl) sulfoxonium cation, diphenyl(p-methoxyphenyl) sulfoxonium cation, diphenyl(o-methoxycarbonylphenyl) sulfoxonium cation, diphenyl (p-phenylsulfanylphenyl) sulfoxonium cation, (7-methoxy-2-oxo-2H-chromen-4-yl) diphenylsulfoxonium cation, (4-methoxynaphthalen-1-yl) diphenylsulfoxonium cation, diphenyl(p-isopropoxycarbonylphenyl) sulfoxonium cation, diphenyl (2-naphthyl) sulfoxonium cation, diphenyl(9-anthryl) sulfoxonium cation, ethyldiphenylsulfoxonium cation, methylethyl(o-tolyl) sulfoxonium cation, methyl di(p-tolyl) sulfoxonium cation, tri(p-tolyl) sulfoxonium cation, diisopropyl(4-phenylsulfanylphenyl) sulfoxonium cation, diphenyl(2-thienyl) sulfoxonium cation, diphenyl(2-furyl) sulfoxonium cation, diphenyl (9-ethyl-9Hcarbazol-3-yl) sulfoxonium cation, and the like, and are not limited thereto.

Examples of phosphonium cations may include, for example, trimethylphenylphosphonium cation, triethylphenylphosphonium cation, tetraphenylphosphonium cation, triphenyl(p-fluorophenyl) phosphonium cation, triphenyl(o-chlorophenyl) phosphonium cation, triphenyl(m-bromophenyl) phosphonium cation, triphenyl(p-cyanophenyl) phosphonium cation, triphenyl(m-nitrophenyl) phosphonium cation, triphenyl(p-phenylsulfanylphenyl) phosphonium cation, (7-methoxy-2-oxo-2H-chromen-4-yl) triphenylphosphonium cation, triphenyl(o-hydroxyphenyl) phosphonium cation, triphenyl(o-acetylphenyl) phosphonium cation, triphenyl(m-benzoylphenyl) phosphonium cation, triphenyl (p-methylphenyl) phosphonium cation, triphenyl(p-isopropoxyphenyl) phosphonium cation, triphenyl(o-methoxycarbonylphenyl) phosphonium cation, triphenyl(1-naphthyl) phosphonium cation, triphenyl(9-anthryl) phosphonium cation, triphenyl(2-thienyl) phosphonium cation, triphenyl(2-furyl) phosphonium cation, triphenyl(9-ethyl-9Hcarbazol-3-yl) phosphonium cation, and the like, and are not limited thereto.

The other cations may be used alone or in combination of two or more types.

The salt compound according to an embodiment may include a compound represented below.

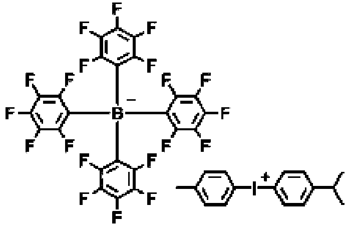

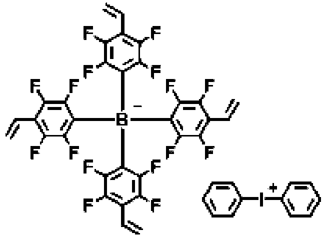

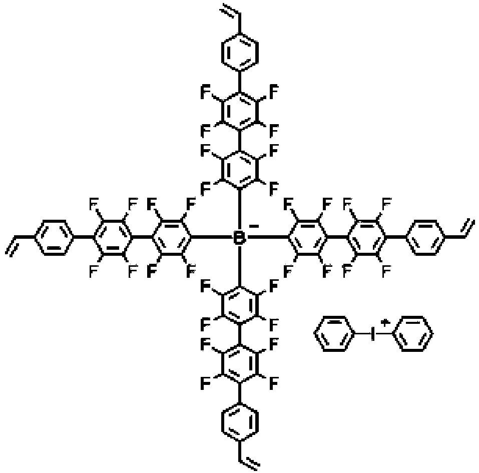

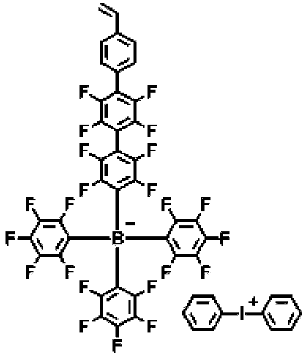

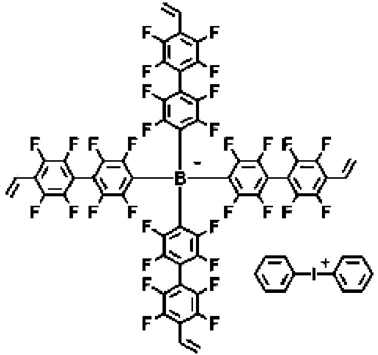

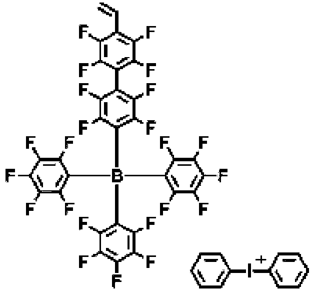

The salt compound may be used alone or in combination of two or more types. The salt compound may be a commercially available product or may be a synthetic product. Method of synthesizing the salt compound is not particularly limited, for example, the salt compound may be synthesized based on the knowledge of known synthesis methods. For instance, it can be synthesized using the method described in the embodiments or according to the method described in the examples. For example, in the method described in the examples, it can be synthesized by changing raw materials or reaction conditions, adding or omitting some steps, or appropriately combining known synthesis methods.

An amount of the low molecular weight compound in the composition according to one embodiment may be greater than or equal to about 30 wt% and less than or equal to about 99 wt%, for example, greater than or equal to about 50 wt% and less than or equal to about 95 wt%, or, for example, greater than or equal to about 70 wt% and less than or equal to about 90 wt%, based on a total weight of the low molecular weight compound and the salt compound according to one embodiment.

An amount of the salt compound in the composition according to an embodiment may be greater than or equal to about 1 wt% and less than or equal to about 70 wt%, for example, greater than or equal to about 5 wt% and less than or equal to about 50 wt%, or for example, greater than or equal to about 10 wt% and less than or equal to about 30 wt%, based on a total weight of the low molecular weight compound and the salt compound according to one embodiment,

The composition according to an embodiment may further include a solvent, in addition to the low molecular weight compound and the salt compound. The solvent may be, for example, in particular not limited to, the one that has a boiling point of greater than or equal to about 100°C and less than or equal to about 350°C at atmospheric pressure (101.3 kPa, 1 atm). The boiling point of the solvent at atmospheric pressure may be, for example, greater than or equal to about 150°C and less than or equal to about 320°C, or for example, greater than or equal to about 180°C and less than or equal to about 300°C. **If** the boiling point of the solvent at atmospheric pressure is within the above range, the film-forming properties and processability in wet film-forming methods, such as the inkjet method, may be improved. Solvents with a boiling point at atmospheric pressure of greater than or equal to about 100°C and less than or equal to about 350°C are not particularly limited, and commonly known solvents may be appropriately employed. Below, solvents with a boiling point at atmospheric pressure of greater than or equal to about 100°C and less than or equal to about 350°C are given as examples but are not limited thereto.

A hydrocarbon-based solvent may include octane, nonane, decane, undecane, dodecane, and the like, and is not limited thereto.

An aromatic hydrocarbon-based solvent may include toluene, xylene, ethylbenzene, n-propylbenzene, isopropylbenzene, mesitylene, n-butylbenzene, sec-butylbenzene, **1-**phenylpentane, 2-phenylpentane, 3-phenylpentane, phenylcyclopentane, phenylcyclohexane, 2-ethylbiphenyl, 3-ethylbiphenyl, etc., and is not limited thereto.

An ether-based solvent may include 1,4-dioxane, 1,2-diethoxyethane, diethyleneglycoldimethylether, diethyleneglycoldiethylether, anisole, ethoxybenzene, 3-methylanisole, m-dimethoxybenzene, etc., and is not limited thereto.

A ketone-based solvent may include 2-hexanone, 3-hexanone, cyclohexanone, 2-heptanone, 3-heptanone, 4-heptanone, cycloheptanone, etc., and is not limited thereto.

An ester-based solvent may include butyl acetate, butyl propionate, butyl butyrate, propylene carbonate, methyl benzoate, ethyl benzoate, propyl benzoate, butyl benzoate, etc., and is not limited thereto.

A nitrile-based solvent may include benzonitrile, 3-methylbenzonitrile, etc., and is not limited thereto.

An amide-based solvent may include N,N-dimethylformamide, N,N-dimethylacetamide, and N-methyl-2-pyrrolidone, etc., and is not limited thereto.

The solvent may be used alone, or in combination of two or more types.

An amount of the solvent in the composition according to an embodiment is not particularly limited. For example, solid concentration (total concentration of the low molecular weight compounds and salt compound) of the composition may be, from the viewpoint of easy application, for example, greater than or equal to about 0.1 % by weight (0.1 wt%) and less than or equal to about 10 wt%, or for example, greater than or equal to about 0.5 wt% and less than or equal to about 5 wt%.

The method of preparing the composition according to an embodiment is not particularly limited. For example, a method of sequentially adding and mixing a low molecular weight compound and a salt compound in a solvent; a method of adding and mixing a low molecular weight compound and a salt compound in a solvent at once; a method of separately preparing a solution of the low molecular weight compound and a solution of the salt compound and then mixing them, and the like. The temperature and mixing time during mixing are also not particularly limited.

### Electroluminescent device

Hereinafter, an electroluminescence device according to an embodiment will be described in detail with reference to the Figure. The figure is a schematic representation showing an electroluminescence device according to an embodiment. In the present specification, "electroluminescence device" may be abbreviated as "EL device."

As shown in the figure, the EL device 100 according to an embodiment comprises a substrate 110, a first electrode 120 on the substrate 110, a hole injection layer 130 on the first electrode 120, a hole transport layer 140 on the hole injection layer 130, an emitting layer 150 on the hole transport layer 140, an electron transport layer 160 on the emitting layer 150, an electron injection layer 170 on the electron transport layer 160, and a second electrode 180 on the electron injection layer 170.

The substrate 110 may be a substrate used as a general EL device. For example, the substrate 110 may be a semiconductor substrate such as, for example, a glass substrate, a silicon substrate, or a transparent plastic substrate.

The first electrode 120 is formed on the substrate 110. The first electrode 120 may, for example, provide holes and may be an anode, and may be formed by a metal, an alloy, a conductive compound, or the like having a large work function. For example, the first electrode 120 may be formed as a transparent electrode using indium tin oxide (In₂O₃-SnO₂: ITO), indium zinc oxide (In₂O₃-ZnO), tin oxide (SnO₂), or zinc oxide (ZnO), etc., which has excellent transparency and conductivity. Additionally, the first electrode 120 may be formed as a reflective electrode by laminating magnesium (Mg), aluminum (Al), etc. on the transparent conductive film. Additionally, after forming the first electrode 120 on the substrate 110, if necessary, cleaning and UV-ozone treatment may be performed.

The hole injection layer 130 may be formed on the first electrode 120. The hole injection layer 130 may be a layer that facilitates the injection of holes from the first electrode 120, and specifically, may be formed to have a thickness (dry film thickness; the same applies hereinafter) of, for example, about 10 nm to about 1000 nm, or for example, about 20 nm to about 50 nm.

According to an embodiment, the hole injection layer 130 may include a cured material formed by curing the composition according to an embodiment (a cured material obtained by curing the composition according to an embodiment, a cured material of the composition according to an embodiment). Hereinafter, the cured material formed by curing the composition according to an embodiment (a cured material obtained by curing the composition according to an embodiment, a cured material of the composition according to an embodiment) will be simply referred to as the "cured material according to an embodiment".

According to an embodiment, the hole injection layer 130 may include a cured material according to an embodiment and a carboxyl-containing compound used in the positive aging described below.

The pre-cured film containing a composition according to an embodiment may be formed by a coating method (solution coating method). For example, it can be formed using a solution coating method, such as, for example, spin coating, casting, micro gravure coating, gravure coating, bar coating, roll coating, wire bar coating, dip coating, spray coating, screen printing, flexographic printing, offset printing, inkjet printing, and the like.

After that, the film formed by the coating method is cured. The curing method is not particularly limited, and it may be thermal curing. Thermal curing can be carried out in an air atmosphere or in an inert gas atmosphere. The temperature for thermal curing is not particularly limited, but, for example, it may be greater than or equal to 80°C, greater than or equal to 100°C, greater than or equal to 130°C, greater than or equal to 150°C, or greater than or equal to 200°C. In addition, the temperature for thermal curing may, for example, be less than or equal to 250°C, less than or equal to 230°C, less than or equal to 200°C, less than or equal to 180°C, or less than or equal to 150°C. The heat-curing time is not particularly limited and may, for example, be 5 minutes or more, 10 minutes or more, 15 minutes or more, 20 minutes or more, or 30 minutes or more. Further, the heat-curing time may, for example, be 70 minutes or less, 60 minutes or less, 50 minutes or less, 30 minutes or less, or 20 minutes or less. In this way, a cured product according to an embodiment may be obtained. Also, in this way, a layer containing the cured product according to an embodiment may be obtained.

In addition to the cured product according to an embodiment (and, if necessary, a carboxyl-containing compound), or when any layer other than the hole injection layer 130 of the EL device 100 contains the cured product according to an embodiment (and, if necessary, a carboxyl-containing compound), the hole injection layer 130 may be formed to include a known hole injection material. The hole injection layer 130 may be formed using a known hole injection material. The known hole injection materials forming the hole injection layer 130 may include, for example, poly(ether ketone)-containing triphenylamine (TPAPEK), 4-isopropyl-4'-methyldiphenyliodonium tetrakis(pentafluorophenyl)borate (PPBI), N,N'-diphenyl-N,N'-bis-[4-(phenyl-m-tolyl-amino)-phenyl]-biphenyl-4,4'-diamine (DNTPD), copper phthalocyanine, 4,4',4"-tris(3-methylphenylphenylamino)triphenylamine (m-MTDATA), N,N'-di(1-naphthyl)-N,N'-diphenylbenzidine (NPB), 4,4',4"-tris(diphenylamino)triphenylamine (TDATA), 4,4',4"-tris(N,N-2-naphthylphenylamino)triphenylamine (2-TNATA), polyaniline/dodecylbenzenesulphonic acid, poly(3,4-ethylenedioxythiophene)/poly(4-styrenesulfonate): PEDOT/PSS, or polyaniline/10-camphorsulfonic acid, but are not limited thereto.

The hole transport layer 140 may be formed on the hole injection layer 130. The hole transport layer 140 may be a layer having a function of transporting holes, and may be formed with a thickness of, for example, about 10 nm to about 150 nm, for example, about 20 nm to about 50 nm. The hole transport layer 140 may be formed by a solution coating method using a composition according to an embodiment. According to this method, the durability (luminescence life-span) of the EL device 100 may be extended. Additionally, it is possible to improve the current efficiency of the EL device 100 and reduce the driving voltage. In addition, since the hole transport layer may be formed by a solution coating method, a large-area film may be efficiently formed.

In an embodiment, the hole transport layer 140 may include a cured material according to an embodiment. According to an embodiment, the hole transport layer 140 may include a cured material according to an embodiment and a carboxyl-containing compound used for positive aging as described below. Specific examples of the coating method and curing method are the same as those described for the hole injection layer 130. In addition to the cured product according to an embodiment (and, if necessary, a carboxyl group-containing compound), or when any layer other than the hole transport layer 140 of the EL device 100 includes the cured product according to an embodiment (and, if necessary, a carboxyl group-containing compound), the hole transport layer 140 may be formed to include a known hole transport material. The known hole transport materials may include, for example, a carbazole derivative such as 1,1-bis[(di-4-tolylamino)phenyl]cyclohexane (TAPC), N-phenylcarbazole and polyvinylcarbazole, N,N'-bis(3-methylphenyl)-N,N'-diphenyl-[1,1-biphenyl]-4,4'-diamine (TPD), 4,4',4"-tris(N-carbazolyl)triphenylamine (TCTA), or N,N'-di(1-naphthyl)-N,N'-diphenylbenzidine (NPB), etc., and are not limited thereto.

The light emitting layer 150 may be formed on the hole transport layer 140. The light emitting layer 150 may be a layer that emits light by fluorescence, phosphorescence, etc., and may be formed using a vacuum deposition method, spin coating method, inkjet printing method, etc. The light emitting layer 150 may be formed with a thickness of, for example, greater than or equal to about 10 nm and less than or equal to about 60 nm, or for example, greater than or equal to about 20 nm and less than or equal to about 50 nm. As the light emitting material of the light emitting layer 150, any known light emitting material may be used. However, the light emitting material included in the light emitting layer 150 may be a light emitting material capable of light emission from triplet excitons (i.e., phosphorescence). In this case, the driving life-span of the EL device 100 may be further improved.

The light emitting layer 150 is not particularly limited and may have a known configuration. For example, the light emitting layer includes at least one of semiconductor nanocrystal particles and/or an organometallic complex. That is, in an embodiment, the organic film may have a light emitting layer including at least one of semiconductor nanocrystal particles and an organometallic complex. In an embodiment, the organic film may include a light emitting layer that includes semiconductor nanocrystal particles. When the light emitting layer includes semiconductor nanocrystal particles, the EL device may be a quantum dot electroluminescence device (QLED), a quantum dot light emitting device, or a quantum dot light emitting diode. In addition, when the light emitting layer includes an organometallic complex, the EL device may be an organic light emitting device (OLED).

In the form of a light emitting layer including semiconductor nanocrystal particles (QLED), the light emitting layer is a single layer or multiple layers of a plurality of semiconductor nanocrystal particles (quantum dots). Here, semiconductor nanocrystal particles (quantum dots) are particles of a certain size that have a quantum confinement effect. The diameter of semiconductor nanocrystal particles (quantum dots) is not particularly limited but may be approximately about 1 nm to about 10 nm.

The semiconductor nanocrystal particles (quantum dots) arranged in the light emitting layer may be synthesized by a wet chemical process, an organometallic chemical vapor deposition process, a molecular beam epitaxy process, or other similar processes. Among these, the wet chemical process is a method of growing particles by adding precursor materials to an organic solvent.

In a wet chemical process, when a crystal grows, an organic solvent is naturally distributed to the surface of the quantum dot crystal and acts as a dispersant, thereby controlling growth of the crystal. Accordingly, in wet chemical processes, the growth of semiconductor nanocrystal particles may be controlled easily and at low cost compared to vapor deposition methods such as metal organic chemical vapor deposition (MOCVD) or molecular beam epitaxy (MBE).

Semiconductor nanocrystal particles (quantum dots) may control the energy band gap by controlling their size, whereby light of various wavelengths may be obtained from the light emitting layer (quantum dot light emitting layer). Therefore, by using multiple quantum dots of different sizes, it is possible to create a display that emits (or emits light) light of multiple wavelengths. The size of the quantum dots may be selected to emit red, green, or blue light, enabling color displays to be constructed. Additionally, the size of the quantum dots may be combined to emit white light with various colors.

As the semiconductor nanocrystal particles (quantum dots), a semiconductor material selected from a Group II-VI semiconductor compound; a Group Ill-V semiconductor compound; a Group IV-VI group semiconductor compound; a Group IV element or compound; or a combination thereof may be used.

The Group II-VI semiconductor compound is not particularly limited, and examples thereof may include a binary compound selected from CdSe, CdTe, ZnS, ZnSe, ZnTe, ZnO, HgS, HgSe, HgTe, and a mixture thereof; a ternary compound selected from CdSeS, CdSeTe, CdSTe, ZnSeS, ZnTeSe, ZnSTe, HgSeS, HgSeTe, HgSTe, CdZnS, CdZnSe, CdZnTe, CdHgS, CdHgSe, CdHgTe, HgZnS, HgZnSe, HgZnTe, and a mixture thereof; and a quaternary compound selected from CdZnSeS, CdZnSeTe, CdZnSTe, CdHgSeS, CdHgSeTe, CdHgSTe, HgZnSeS, HgZnSeTe, HgZnSTe, and a mixture thereof.

The Group III-V semiconductor compound is not particularly limited, and examples thereof may include a binary compound selected from GaN, GaP, GaAs, GaSb, AlN, AlP, AlAs, AlSb, InN, InP, InAs, InSb, and a mixture thereof; a ternary compound selected from GaNP, GaNAs, GaNSb, GaPAs, GaPSb, AlNP, AlNAs, AlNSb, AlPAs, AlPSb, InNP, InNAs, InNSb, InPAs, InPSb, GaAlNP, and a mixture thereof; and a quaternary compound selected from GaAlNAs, GaAlNSb, GaAlPAs, GaAlPSb, GaInNP, GalnNAs, GaInNSb, GalnPAs, GaInPSb, InAlNP, lnAlNAs, InAlNSb, InAlPAs, InAlPSb, and a mixture thereof.

The Group IV-VI semiconductor compound is not particularly limited, and examples thereof may include a binary compound selected from SnS, SnSe, SnTe, PbS, PbSe, PbTe, and a mixture thereof; a ternary compound selected from SnSeS, SnSeTe, SnSTe, PbSeS, PbSeTe, PbSTe, SnPbS, SnPbSe, SnPbTe, and a mixture thereof; and a quaternary compound selected from SnPbSSe, SnPbSeTe, SnPbSTe, and a mixture thereof.

The Group IV element or compound is not particularly limited, and examples thereof may include a single elemental selected from Si, Ge, and a mixture thereof; or a binary elemental compound selected from SiC, SiGe, or a mixture thereof.

The semiconductor nanocrystal particles (quantum dots) may have a homogeneous single structure or a core-shell dual structure. The core and shell may include different materials. The material constituting each core and shell may be composed of different semiconductor compounds. However, the energy bandgap of the shell material may be larger than the energy bandgap of the core material. For example, the structures may be ZnTeSe/ZnSe/ZnS, InP/ZnSe/ZnS, CdSe/ZnS, InP/ZnS, etc.

For example, a producing process of a quantum dot having a core (CdSe)/shell (ZnS) structure is described. First, a core (CdSe) precursor material such as (CH3)2Cd (dimethylcadmium) and TOPSe (trioctylphosphine selenide) is injected into an organic solvent using TOPO (trioctylphosphine oxide) as a surfactant to form a crystal. At this time, after maintaining the crystal at a high temperature for a certain period of time so that it grows to a certain size, a precursor material for the shell (ZnS) is injected to form a shell on the surface of the already formed core. This allows the production of TOPO-capped CdSe/ZnS quantum dots.

Further, in the form of a light emitting layer including organometallic complex (OLED), the light emitting layer 150 may include, as a host material, for example, 6,9-diphenyl-9'-(5'-phenyl-[1,1':3',1"-terphenyl]-3-yl)3,3'-bi[9H-carbazole], 3,9-diphenyl-5-(3-(4-phenyl-6-(5'-phenyl-[1,1":3',1"-terphenyl]-3-yl)-1,3,5-triazine-2-yl)phenyl)-9H-carbazole, 9,9'-diphenyl-3,3'-bi[9H-carbazole], tris(8-quinolinato)aluminium (Alq3), 4,4'-bis(carbazol-9-yl)biphenyl (CBP), poly(n-vinyl carbazole) (PVK), 9,10-di(naphthalene-2-yl)anthracene (ADN), 4,4',4"-tris(N-carbazolyl)triphenylamine (TCTA), 1,3,5-tris(N-phenyl-benzimidazol-2-yl)benzene (TPBI), 3-tert-butyl-9,10-di(naphth-2-yl)anthracene (TBADN), distyrylarylene (DSA), 4,4'-bis(9-carbazole)-2,2'-dimethyl-biphenyl (dmCBP), and the like.

In addition, the light-emitting layer 150 may include, as dopant materials, for example, perylene and its derivatives, rubrene and its derivatives, coumarin and its derivatives, 4-dicyanomethylene-2-(p-dimethylaminostyryl)-6-methyl-4H-pyran (DCM) and its derivatives, bis[2-(4,6-difluorophenyl)pyridinate]picolinate iridium(III) (Flrpic), bis(1-phenylisoquinoline)(acetylacetonate)iridium(III)(Ir(piq)₂(acac)), tris(2-phenylpyridine)iridium(III) (Ir(ppy)₃), iridium (Ir) complexes, such as, for example, tris(2-(3-p-xylol)phenyl)pyridine iridium(III) and other, osmium (Os) complexes, platinum complexes, and the like. Among these, for example, the light emitting material may be a light emitting organometallic complex compound.

The method for forming the light emitting layer is not particularly limited. It may be formed by coating a coating solution including at least one of semiconductor nanocrystal particles and an organometallic complex (solution coating method). At this time, as a solvent constituting the coating solution, a solvent that does not dissolve the material in the hole transport layer, such as, for example, a hole transport material, may be selected.

The electron transport layer 160 may be formed on the light emitting layer 150. The electron transport layer 160 may be a layer that has the function of transporting electrons and may be formed by using a vacuum deposition method, spin coating method, inkjet method, or the like. The electron transport layer 160 may be formed to have a thickness of, for example, greater than or equal to about 15 nm and less than or equal to about 50 nm.

The electron transport layer 160 may be formed of a known electron transport material. Examples of known electron transport materials may include (8-quinolinolato)lithium (lithium quinolate, Liq), tris(8-quinolinolato)aluminium (Alq₃), or a compound having a nitrogen-containing aromatic ring. Examples of the compound having a nitrogen-containing aromatic ring may include a compound including a pyridine ring, such as, for example, 1,3,5-tri[(3-pyridyl)-phen-3-yl]benzene, a compound including a triazine ring, such as, for example, 2,4,6-tris(3'-(pyridin-3-yl)biphenyl-3-yl)-1,3,5-triazine, a compound including an imidazole ring, such as, for example, 2-(4-(N-phenylbenzoimidazolyl-1-yl-phenyl)-9,10-dinaphthylanthracene or 1,3,5-tris(N-phenyl-benzimidazol-2-yl)benzene (TPBI). The electron transport material may be used alone or as a mixture of two or more types.

The electron injection layer 170 may be formed on the electron transport layer 160. The electron injection layer 170 may be a layer that has the function of facilitating the injection of electrons from the second electrode 180. The electron injection layer 170 may be formed by using a vacuum deposition method or the like. The electron injection layer 170 may be formed to have a thickness of, for example, greater than or equal to about 0.1 nm and less than or equal to about 5 nm, or for example, greater than or equal to about 0.3 nm and less than or equal to about 2 nm. Any known material may be used as a material for forming the electron injection layer 170. For example, the electron injection layer 170 may be formed by a lithium compound such as, for example, (8-quinolinato)lithium (lithium quinolate, Liq) and lithium fluoride (LiF), sodium chloride (NaCl), cesium fluoride (CsF), lithium oxide (Li₂O), or barium oxide (BaO).

The second electrode 180 may be formed on the electron injection layer 170. The second electrode 180 may be formed by using a vacuum deposition method or the like. The second electrode 180 may provide electrons, and, for example, be a cathode, and may be formed by a metal, alloy, or conductive compound having a small work function. For example, the second electrode 180 may be formed as a reflective electrode using a metal, such as, for example, lithium (Li), magnesium (Mg), aluminum (Al), calcium (Ca), or an alloy, such as, for example, aluminum-lithium (Al-Li), magnesium-indium (Mg-In), or magnesium-silver (Mg-Ag). The second electrode 180 may be formed with a thickness of, for example, from about 10 nm to about 200 nm, for example, from about 50 nm to about 150 nm. Alternatively, the second electrode 180 may be formed as a transparent electrode by a thin film of the metal material having a thickness of 20 nm or less, a transparent conductive film, such as, for example, indium tin oxide (In₂O₃-SnO₂) or indium zinc oxide (In₂O₃-ZnO).

Regarding the film-forming method of layers other than organic films containing a cured product (and a carboxyl group-containing compound if necessary) according to an embodiment, it is not particularly limited. The layers other than the organic film containing a cured product (and a carboxyl group-containing compound if necessary) according to an embodiment may, for example, be formed by vacuum deposition or by a solution coating method.

### Positive aging, and Carboxyl-containing compound

As stated, a unique technology of QD-LED is the positive aging caused by an acid compound, and by the positive aging, the luminous efficiency and device lifespan of QD-LEDs may be improved. An electroluminescent device (for example, a QD-LED) according to an embodiment may include an organic layer containing a cured material according to an embodiment. Thus, even when positive aging is performed using an acid compound, the reduction of driving voltage, and the improvement effects on durability (luminous lifetime) and luminous efficiency (current efficiency) of the device may be achieved. Therefore, the electroluminescent device according to an embodiment (for example, QD-LED) may have excellent performance.

Acidic compound used in the positive aging may include, for example, a carboxyl-containing compound, sulfinic acid-containing compound, sulfonic acid-containing compound, and the like. Among these, for instance, a carboxyl-containing compound may be used.

The carboxyl-containing compound may include at least one selected from, for example, acetic acid, propionic acid, butyric acid, valeric acid, caproic acid, malonic acid, succinic acid, glutaric acid, adipic acid, pimelic acid, (meth)acrylic acid, poly(meth)acrylic acid, benzoic acid, hexahydrobenzoic acid, terephthalic acid, 1,4-cyclohexanedicarboxylic acid, citric acid, malic acid, 3-isopropylmalic acid, itaconic acid, or citraconic acid, but are not limited thereto.

The method of positive aging is not particularly limited, for example, after fabricating a QD-LED, a resin containing an acid compound or a solution containing an acid compound (for example, a solution using alcohol as a solvent), is mounted or applied onto the first electrode or the second electrode of the QD-LED, and then irradiated with an active energy beam to form an acid compound layer. Thereafter, positive aging may be performed under a heat-treatment condition. At this time, the acid compound may be a compound containing a carboxyl group.

As a solvent used in a solution containing the above compound, in addition to the solvent described herein, an alcohol-based solvent, for example, ethanol, n-propanol, isopropanol, butanol, methyl cellosolve, ethylene glycol monobutyl ether, or propylene glycol monomethyl ether may also be used. The aforementioned solvent may be used alone, or in combination of two or more different solvents. The concentration of the solution containing the acid compound may not be particularly limited, for example, of about 0.5% by weight or more and about 30% by weight or less, for instance, about 1% by weight or more and about 20% by weight or less.

The active energy rays used may include ultraviolet rays, such as, for example, the g-line (wavelength 436 nm), h-line (wavelength 405 nm), i-line (wavelength 365 nm), and the like. Additionally, wavelengths of less than or equal to 300 nm, such as, for example, KrF rays (wavelength 248 nm), ArF rays (wavelength 193 nm), etc. may also be used. The cumulative luminous flux of the active energy rays may be, for example, greater than or equal to about 5 mJ/cm²and less than or equal to about 20 mJ/cm².

Thickness of the oxide compound layer is not particularly limited, for example, the thickness may be greater than or equal to about 10 nm, greater than or equal to about 30 nm, greater than or equal to about 50 nm, greater than or equal to about 70 nm, greater than or equal to about 90 nm, greater than or equal to about 100 nm, greater than or equal to about 120 nm, greater than or equal to about 140 nm, greater than or equal to about 160 nm, greater than or equal to about 180 nm, greater than or equal to about 200 nm, greater than or equal to about 250 nm, greater than or equal to about 300 nm, greater than or equal to about 350 nm, greater than or equal to about 400 nm, greater than or equal to about 450 nm, greater than or equal to about 500 nm, greater than or equal to about 550 nm, greater than or equal to about 600 nm, greater than or equal to about 650 nm, greater than or equal to about 700 nm, greater than or equal to about 750 nm, greater than or equal to about 800 nm, greater than or equal to about 850 nm, greater than or equal to about 900 nm, greater than or equal to about 950 nm, greater than or equal to about 1 µm, or greater than or equal to about 1.5 µm. In addition, the thickness of the oxide compound layer may be, for example, less than or equal to about 100 µm, less than or equal to about 90 µm, less than or equal to about 80 µm, less than or equal to about 70 µm, less than or equal to about 60 µm, less than or equal to about 50 µm, less than or equal to about 40 µm, less than or equal to about 30 µm, less than or equal to about 20 µm, less than or equal to about 10 µm, less than or equal to about 9 µm, less than or equal to about 8 µm, less than or equal to about 7 µm, less than or equal to about 6 µm, less than or equal to about 5 µm, less than or equal to about 4 µm, less than or equal to about 3 µm, less than or equal to about 2 µm, or less than or equal to about 1 µm.

The QD-LED device, in which this oxide compound layer has been formed, may be maintained at a predetermined temperature for a predetermined period to perform positive aging. The period of positive aging may, for example, be 12 hours or more, 20 hours or more, 24 hours or more, 2 days or more, or 3 days or more. In addition, the period of positive aging may, for example, be 30 days or less, 20 days or less, 10 days or less, or 7 days or less, and these examples are not limiting.

The temperature for positive aging may be, for example, greater than or equal to about 30°C, greater than or equal to about 35°C, greater than or equal to about 40°C, greater than or equal to about 50°C, greater than or equal to about 55°C, greater than or equal to about 60°C, or greater than or equal to about 65°C.

After performing positive aging, the cured material and carboxyl-containing compound according to an embodiment may be included in any one of the layers (organic layers) arranged between, for example, the first electrode 120 and the second electrode 180. That is, according to an embodiment, an organic layer (hereinafter also referred to as "the organic layer according to an embodiment") including a cured product and a carboxyl group-containing compound according to an embodiment may be provided. According to another embodiment, an electroluminescent device including is provided, the electroluminescent device includes a first electrode layer that supplies holes, a second electrode layer that supplies electrons, and at least one organic layer provided between the first electrode layer and the second electrode layer, wherein at least one layer of the at least one organic layer includes an organic film including a cured product and a carboxyl group-containing compound according to an embodiment.

According to an embodiment, the cured product and the carboxyl-containing compound may be included in hole injection layer 130 as a hole injection material, in hole transport layer 140 as a hole transport material, or in light-emitting layer 150 as a light-emitting material (host). For example, the cured product and the carboxyl-containing compound according to an embodiment may be included in hole injection layer 130 as a hole injection material, or in hole transport layer 140 as a hole transport material. For example, a cured material according to an embodiment and a carboxyl-containing compound may be included in hole injection layer 130 as a hole injection material. That is, in an embodiment, at least one layer selected from the hole transport layer, the hole injection layer, or the light emitting layer may include an organic film according to an embodiment. In an embodiment, at least one layer selected from the hole transport layer or the hole injection layer may include an organic film according to an embodiment. In an embodiment, the hole injection layer may include an organic film according to an embodiment.

In summary, as an example of an electroluminescent device according to an embodiment, EL device 100 according to an embodiment has been described. The EL device 100 according to the embodiment may include an organic layer (for example, a hole transport layer or a hole injection layer) that includes a cured material according to an embodiment and a carboxyl group-containing compound. As a result, the EL device may exhibit excellent positive aging resistance, improved durability (luminous lifespan), and luminous efficiency (current efficiency), leading to an EL device with reduced driving voltage.

The laminated structure of the EL device 100 according to an embodiment is not limited to the above example. The EL device 100 according to an embodiment may be formed with another known laminated structure. For example, in the EL device 100, one or more of the hole injection layer 130, hole transport layer 140, electron transport layer 160, and electron injection layer 170 may be omitted, and additionally, other layers may be provided. Furthermore, each layer of the EL device 100 may be formed as a single layer or as multiple layers.

For example, the EL device 100 may further include a hole blocking layer between the hole transport layer 140 and the light emitting layer 150 to prevent excitons or holes from diffusing into the electron transport layer 160. The hole blocking layer may be formed, for example, from oxadiazole derivatives, triazole derivatives, or phenanthroline derivatives, etc.

In addition, a composition according to an embodiment may be applied to electroluminescent devices other than the QD-LED or OLED. Other electroluminescent devices to which the composition according to an embodiment can be applied are not particularly limited, but for example, may include organic-inorganic perovskite light-emitting devices, and the like.

Although the examples have been described in detail according to embodiments, these are descriptive and exemplary, not limiting, and it is clear that the scope of the embodiment should be interpreted according to the attached claims.

The above examples include the following embodiments.
1. A low molecular weight compound represented by Chemical Formula (1) or Chemical Formula (2): 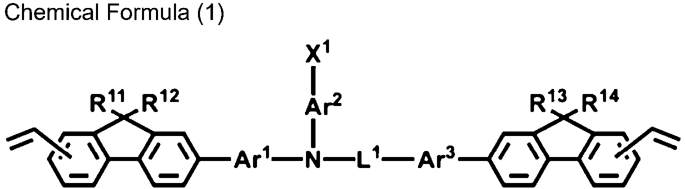 in Chemical Formula (1),
   R¹¹ to R¹⁴ are, each independently, a hydrogen atom, or a hydrocarbon group having 1 to 16 carbon atoms,
   Ar¹ is single bond, a substituted or unsubstituted aromatic hydrocarbon ring group, or a substituted or unsubstituted aromatic heterocyclic group,
   Ar² is a substituted or unsubstituted aromatic hydrocarbon ring group, or a substituted or unsubstituted aromatic heterocyclic group,
   Ar³ is a substituted or unsubstituted carbazole, a substituted or unsubstituted fluorene, a substituted or unsubstituted benzene, or a substituted or unsubstituted biphenylene group,
   L¹ is single bond, or a group represented by chemical formula (1-a), 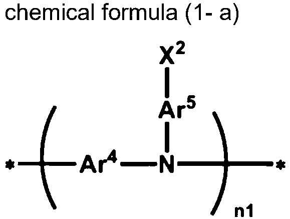
   in chemical formula (1-a),
   Ar⁴ and Ar⁵ are, each independently, a substituted or unsubstituted aromatic hydrocarbon ring group, or a substituted or unsubstituted aromatic heterocyclic group,
   X² is a hydrogen atom, or a group selected from Group 1,
   n1 is an integer of 1 or 2, provided that
   when n1 is an integer, 2, each Ar⁴ are the same or different,
   when n1 is an integer, 2, each Ar⁵ are the same or different,
   when n1 is an integer, 2, each X² are the same or different,
   * is a bonding position, and
   X¹ is a hydrogen atom, or a group selected from Group 1: 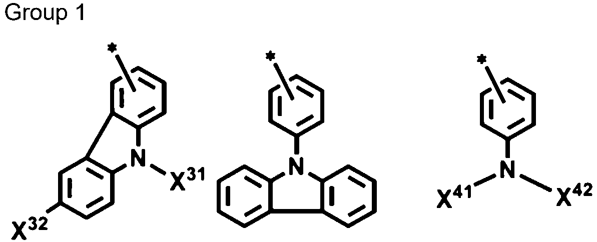
   in Group 1,
   X³¹ and X³² are, each independently, a hydrogen atom, a group selected from Group 2, or a combination thereof,
   X⁴¹ and X⁴² are, each independently, a hydrogen atom, a group selected from Group 2, or a combination thereof, and
   * is a bonding position: 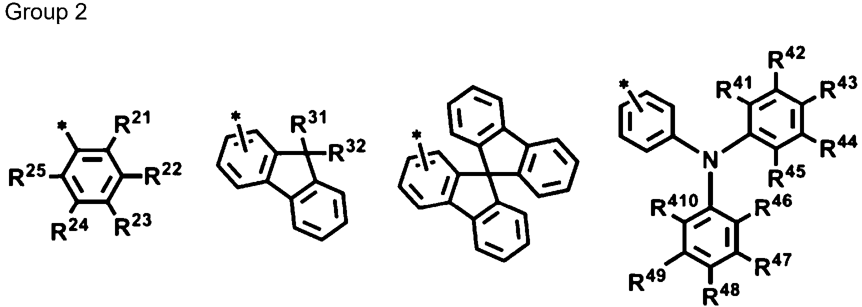
   in Group 2,
   R²¹ to R²⁵ are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
   R³¹ and R³² are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
   R⁴¹ to R⁴⁹ and R⁴¹⁰ are, each independently, a hydrogen atom, a fluorine atom, an unsubstituted linear alkyl group having 1 to 12 carbon atoms, an unsubstituted branched alkyl group having a carbon number of 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3, and * is a bonding position: 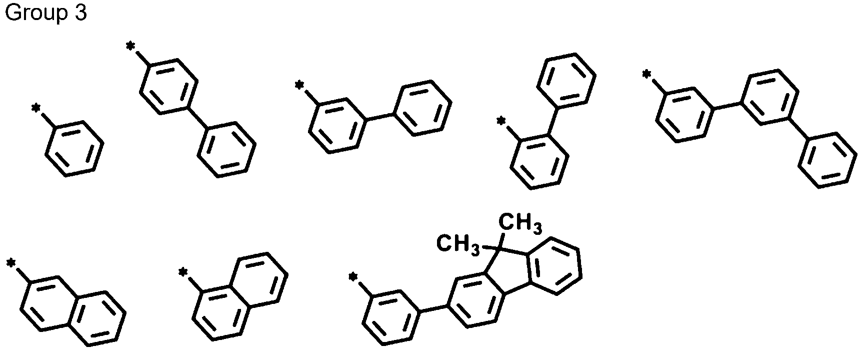
   in Group 3,
   * is a bonding position: 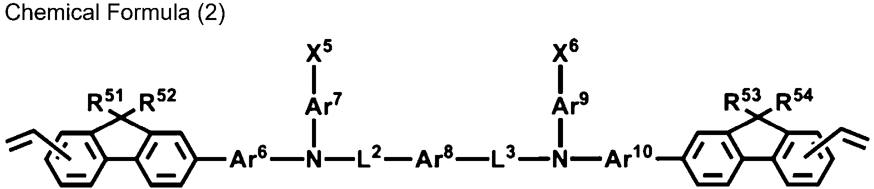
   in Chemical Formula (2),
   R⁵¹ to R⁵⁴ are, each independently, a hydrogen atom, or a hydrocarbon group having 1 to 16 carbon atoms,
   Ar⁶ and Ar¹⁰ are, each independently, single bond, a substituted or unsubstituted aromatic hydrocarbon ring group, or a substituted or unsubstituted aromatic heterocyclic group,
   Ar⁷ and Ar⁹ are, each independently, a substituted or unsubstituted aromatic hydrocarbon ring group, or a substituted or unsubstituted aromatic heterocyclic group,
   Ar⁸ is a substituted or unsubstituted carbazole, a substituted or unsubstituted fluorene, a substituted or unsubstituted benzene, or a substituted or unsubstituted biphenyl ring,
   L² is a single bond, or a group represented by chemical formula (2-a): 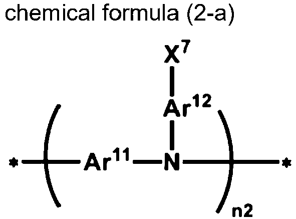
   in chemical formula (2-a),
   Ar¹¹ and Ar¹² are, each independently, a substituted or unsubstituted aromatic hydrocarbon ring group, or a substituted or unsubstituted aromatic heterocyclic group,
   X⁷ is a hydrogen atom, or a group selected from Group 1,
   n2 is an integer of 1 or 2, wherein
   when n2 is an integer, 2, each Ar¹¹ are the same or different,
   when n2 is an integer, 2, each Ar¹² are the same or different,
   when n2 is an integer, 2, each X⁷ are the same or different,
   * is a bonding position, and
   L³ is a single bond, or a group represented by chemical formula (2-b), 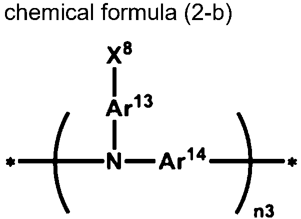
   in chemical formula (2-b),
   Ar¹³ and Ar¹⁴ are, each independently, a substituted or unsubstituted aromatic hydrocarbon ring group, or a substituted or unsubstituted aromatic heterocyclic group,
   X⁸ is a hydrogen atom, or a group selected from Group 1,
   n3 is an integer of 1 or 2, wherein
   when n3 is an integer of 2, each Ar¹³ are the same or different,
   when n3 is an integer of 2, each Ar¹⁴ are the same or different,
   when n3 is an integer of 2, each X⁸ are the same or different,
   * is a bonding position, and
   X⁵ and X⁶ are, each independently, a hydrogen atom, or a group selected from Group 1.
2. The low molecular weight compound according to item 1,
   wherein in Chemical Formula (1),
   R¹¹ to R¹⁴ are, each independently, a hydrogen atom, or a hydrocarbon group having 1 to 10 carbon atoms,
   Ar¹ and Ar² are, each independently, a substituted or unsubstituted aromatic hydrocarbon ring group,
   Ar³ is a substituted or unsubstituted fluorene ring group, a substituted or unsubstituted benzene ring group, or a substituted or unsubstituted biphenyl ring group,
   L¹ is single bond, or a group represented by chemical formula (1-a), wherein in chemical formula (1-a),
   Ar⁴ and Ar⁵ are, each independently, a substituted or unsubstituted aromatic hydrocarbon ring group,
   n1 is an integer of 1 or 2,
   X¹ of Chemical Formula (1) and X² of chemical formula (1-a) are, each independently, a hydrogen atom, or a group represented by the following chemical formula: 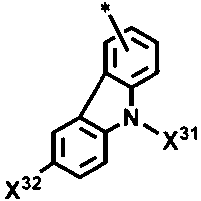
   wherein in the chemical formula, X³¹ and X³² are, each independently, a hydrogen atom, or a group represented in Group 2.
3. The low molecular weight compound according to item 1 or 2,
   wherein in Chemical Formula (1),
   R¹¹ to R¹⁴ are, each independently, a hydrocarbon group having 1 to 6 carbon atoms,
   Ar¹ to Ar³ are, each independently, a substituted or unsubstituted benzene ring group, a biphenyl ring group, or a fluorene ring group,
   L¹ is single bond, or a group represented by chemical formula (1-a), wherein in chemical formula (1-a),
   Ar⁴ and Ar⁵ are, each independently, a substituted or unsubstituted benzene ring group, a biphenyl ring group, or a fluorene ring group,
   n1 is an integer of 1 or 2,
   X¹ of Chemical Formula (1) and X² of chemical formula (1-a) are, each independently, a hydrogen atom, or a group represented by the following chemical formula: 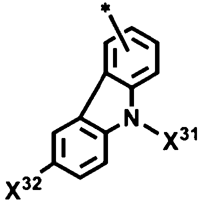
   wherein in the chemical formula, X³¹ and X³² are, each independently, a hydrogen atom, or a group represented by chemical formula (a): 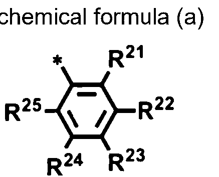
   wherein in chemical formula (a),
   R²¹ and R²⁵ are, each independently, a hydrogen atom, a fluorine atom, an unsubstituted linear alkyl group having 1 to 12 carbon atoms, an unsubstituted branched alkyl group having 1 to 12 carbon atoms, a linear alkyl group having 1 to 12 carbon atoms and substituted by a fluorine atom, a branched alkyl group having 1 to 12 carbon atoms and substituted by a fluorine atom, or a group listed in Group 3.
4. The low molecular weight compound according to any one of items 1 to 3,
   wherein
   X¹ of Chemical Formula (1) and X² of chemical formula (1-a) are, each independently, a hydrogen atom, or a group selected from Group (1-1): 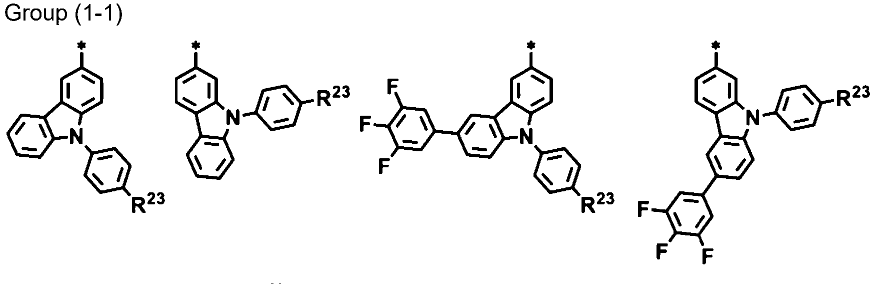
   wherein in Group (1-1), R²³ is a hydrogen atom, a fluorine atom, an unsubstituted linear alkyl group having 1 to 12 carbon atoms, an unsubstituted branched alkyl group having 1 to 12 carbon atoms, a linear alkyl group having 1 to 12 carbon atoms and substituted by a fluorine atom, a branched alkyl group having 1 to 12 carbon atoms and substituted by a fluorine atom, or a group selected from Group 3.
5. The low molecular weight compound according to any one of items 1 to 4, wherein
   in Chemical Formula (2),
   R⁵¹ to R⁵⁴ are, each independently, a hydrocarbon group having 1 to 10 carbon atoms,
   Ar⁶ and Ar¹⁰ are, each independently, a single bond, or a substituted or unsubstituted aromatic hydrocarbon ring group,
   Ar⁷ and Ar⁹ are, each independently, a substituted or unsubstituted aromatic hydrocarbon ring group,
   Ar⁸ is a substituted or unsubstituted fluorene ring, substituted or unsubstituted benzene ring, or substituted or unsubstituted biphenyl ring,
   L² is a single bond, or a group represented by chemical formula (2-a),
   wherein in chemical formula (2-a),
   Ar¹¹ and Ar¹² are, each independently, a substituted or unsubstituted aromatic hydrocarbon ring,
   X⁷ is a hydrogen atom, or a group selected from Group 1,
   L³ is single bond, or a group represented by chemical formula (2-b),
   wherein in chemical formula (2-b),
   Ar¹³ and Ar¹⁴ are, each independently, a substituted or unsubstituted aromatic hydrocarbon ring, and
   X⁸ is a hydrogen atom, or a group selected from Group 1, and
   wherein in chemical formula (2),
   X⁵ and X⁶ are, each independently, a hydrogen atom, or a group selected from Group 1.
6. The low molecular weight compound according to any one of items 1 to 5,
   wherein
   in Chemical Formula (2),
   R⁵¹ to R⁵⁴ are, each independently, a hydrocarbon group having 1 to 6 carbon atoms,
   Ar⁶ and Ar¹⁰ are both single bonds,
   Ar⁷ and Ar⁹ are, each independently, a substituted or unsubstituted aromatic hydrocarbon ring group,
   L² and L³ are both single bonds, and
   X⁵ and X⁶ are, each independently, a hydrogen atom, or a group selected from Group 1.
7. The low molecular weight compound according to any one of items 1 to 6, wherein the compound is selected from the following group: 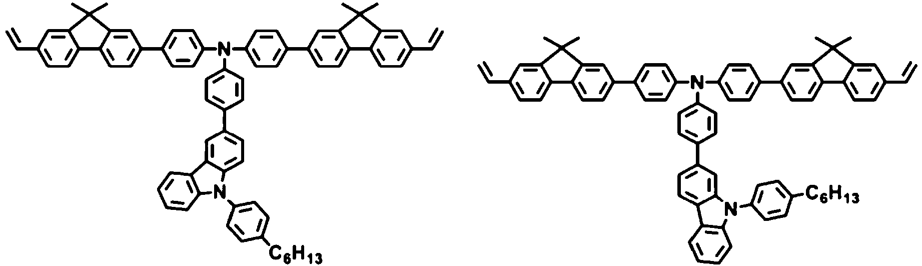 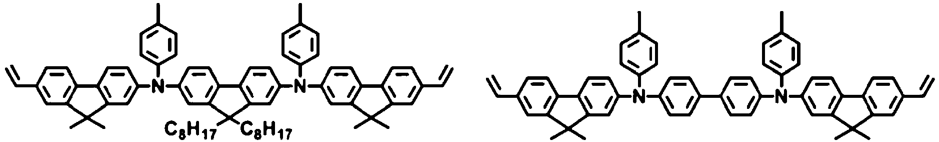 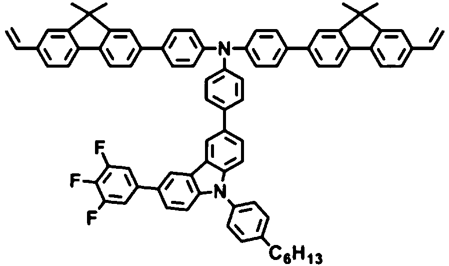
8. A composition including the low molecular weight compound according to any one of items 1 to 7, and a salt compound including a borate anion represented by Chemical Formula (3) and an iodonium cation: 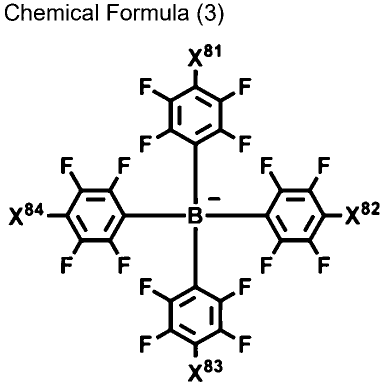 wherein in chemical formula (3),
   X⁸¹ to X⁸⁴ are, each independently, a hydrogen atom, a fluorine atom, or a group represented by chemical formula (3-a),

      chemical formula (3-a) *-L⁴-(Ar¹⁶)ₙ₆-(Z)ₙ₄
   wherein in chemical formula (3-a),
   L⁴ is independently a single bond or a group represented by chemical formula (3-b),
   Ar¹⁶ is a substituted or unsubstituted aromatic hydrocarbon ring group, or a substituted or unsubstituted aromatic heterocyclic group,
   Z is a fluorine atom, or a group selected from Group 4,
   n4 is an integer from 1 to 5,
   n6 is an integer of 0 or 1, wherein
   when there are two or more groups represented by Chemical formula (3-a), each L⁴ are the same or different,
   when there are two or more groups represented by Chemical formula (3-a), each Ar¹⁶ are the same or different,
   when there are two or more groups represented by Chemical formula (3-a), each Z are the same or different,
   when there are two or more groups represented by Chemical formula (3-a), each n4 are the same or different,
   when there are two or more groups represented by Chemical formula (3-a), each n6 are the same or different,
   when n4 is an integer of greater than or equal to 2, each Z are the same or different, and
   * is a bonding position, 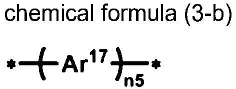
   wherein in chemical formula (3-b),
   Ar¹⁷ is a substituted or unsubstituted aromatic hydrocarbon ring group, or a substituted or unsubstituted aromatic heterocyclic group,
   n5 is an integer from 1 to 5, and when n5 is an integer of greater than or equal to 2, each Ar¹⁷ are the same or different, and
   * is a bonding position, and 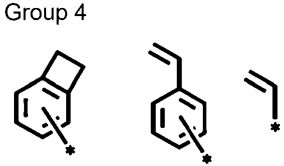
   wherein in Group 4,
   * is a bonding position.
9. The composition according to item 8, wherein the borate anion is represented by the following compounds: 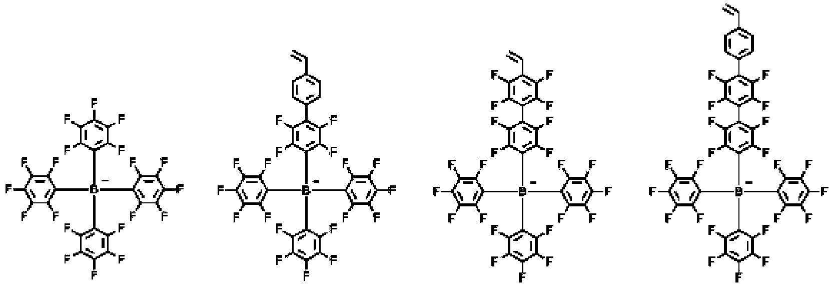 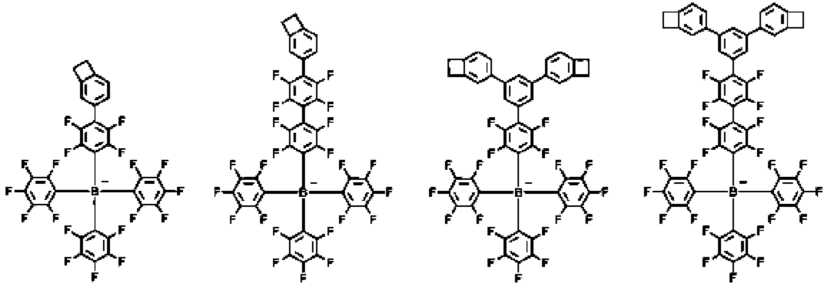 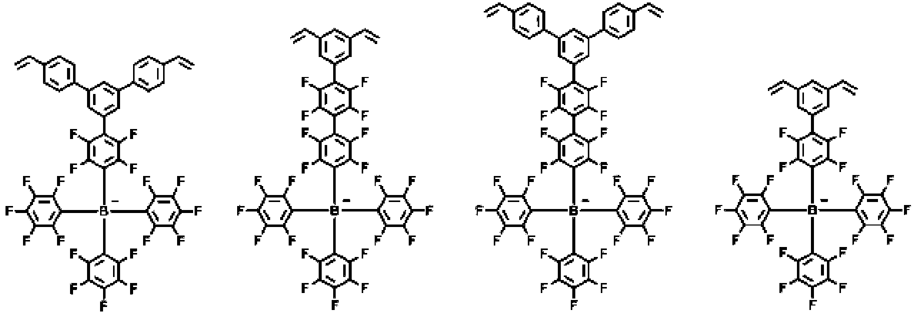 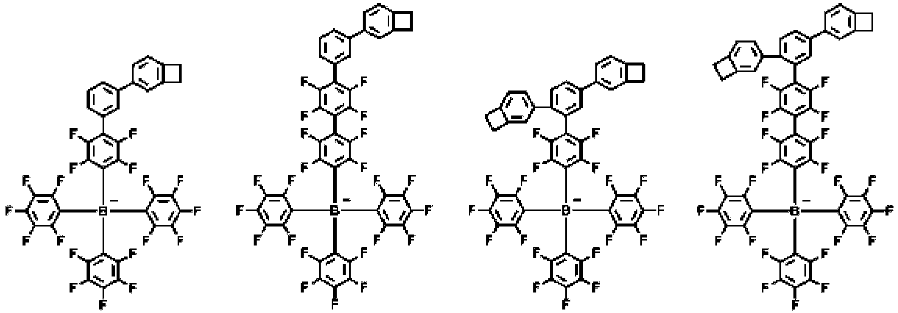 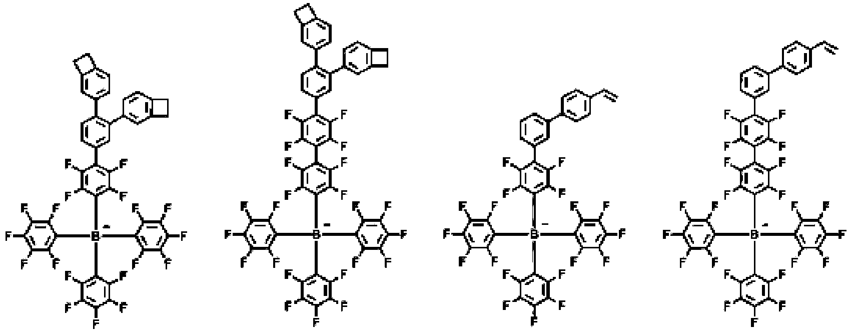 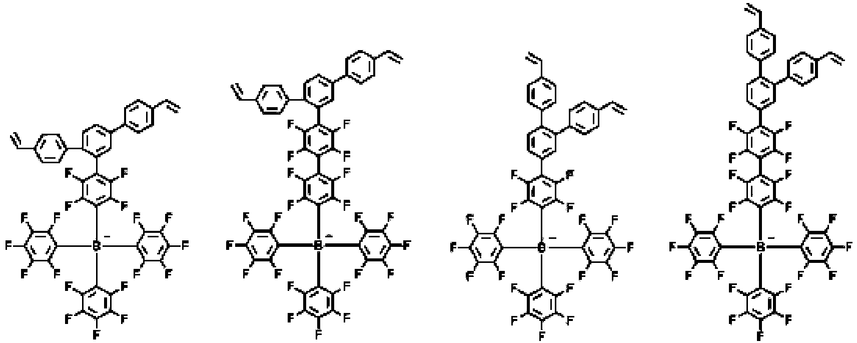 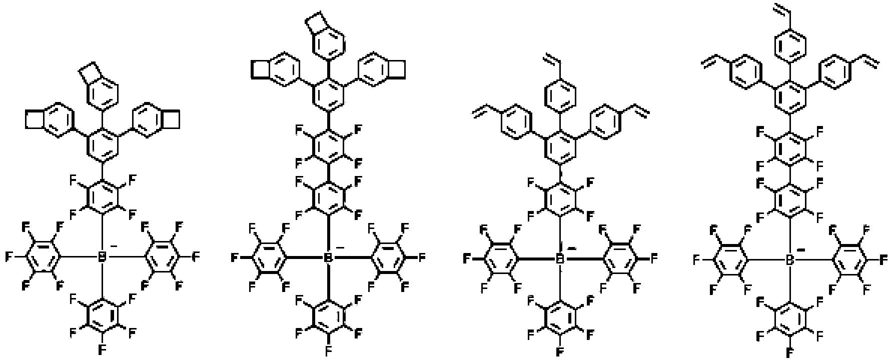 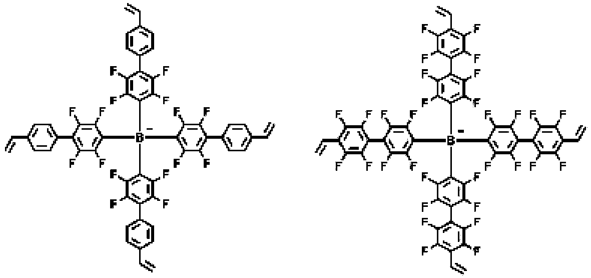 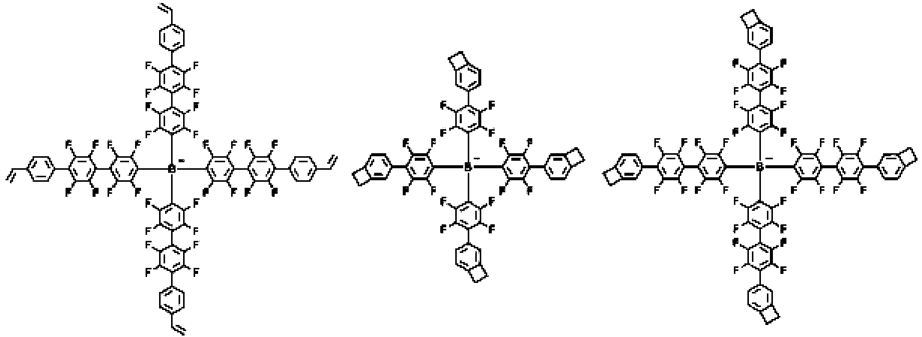 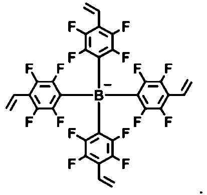
10. The composition according to item 8 or item 9, wherein an energy difference between the ionization potential of the low molecular weight compound and an electron affinity of the borate anion is greater than or equal to about 0.75 eV, and the ionization potential of the low molecular weight compound is deeper than ionization potential of the iodonium cation.
11. The composition according to any one of items 8 to 10, wherein the electron affinity of the borate anion is greater than or equal to about 5.50 eV and less than or equal to about 6.50 eV.
12. The composition according to any one of items 8 to 11, wherein the iodonium cation is any one of difuryliodonium cation, dithienyliodonium cation, bis(4,5-dimethyl-2-furyl) iodonium cation, bis(5-chloro-2-thienyl) iodonium cation, bis (5-cyano-2-furyl) iodonium cation, bis(5-nitro-2-thienyl) iodonium cation, bis(5-acetyl-2-furyl) iodonium cation, bis(5-carboxy-2-thienyl) iodonium cation, bis(5-methoxycarbonyl-2-furyl) iodonium cation, bis(5-phenyl-2-furyl) iodonium cation, bis(5-(p-methoxyphenyl)-2-thienyl) iodonium cation, bis(5-vinyl-2-furyl) iodonium cation, bis(5-ethynyl-2-thienyl) iodonium cation, bis(5-cyclohexyl-2-furyl) iodonium cation, bis (5-hydroxy-2-thienyl) iodonium cation, bis(5-phenoxy-2-furyl) iodonium cation, bis(5-mercapto-2-thienyl) iodonium cation, bis(5-butylthio-2-thienyl) iodonium cation, bis(5-phenylthio-2-thienyl) iodonium cation, diphenyliodonium cation, bis(p-tolyl)iodonium cation, bis(p-octylphenyl) iodonium cation, bis(p-octadecylphenyl) iodonium cation, bis(p-octyloxyphenyl) iodonium cation, bis(p-octadecyloxyphenyl) iodonium cation, phenyl(p-octadecyloxyphenyl) iodonium cation, 4-isopropyl-4'-methyldiphenyliodonium cation, (4-isobutylphenyl)-p-tolyliodonium cation, bis(1-naphthyl) iodonium cation, bis(4-phenylsulfanylphenyl) iodonium cation, phenyl(6-benzoyl- 9-ethyl-9H-carbazol-3-yl) iodonium cation, (7-methoxy-2-oxo-2H-chromen-3-yl)-4'-isopropylphenyliodonium cation, or a combination of any two or more iodonium cations listed above.
13. The composition according to any one of items 8 to 12, wherein the ionization potential of the iodonium cation is greater than or equal to about 4.00 eV and less than or equal to about 4.95 eV.
14. The composition according to any one of items 8 to 13, wherein an amount of the salt compound in the composition is greater than or equal to about 1 weight percent (wt%) and less than or equal to about 70 wt% based on a total weight of the low molecular weight compound and the salt compound.
15. An organic film including a cured material obtained by curing the composition according to any one of items 8 to 14, and a carboxyl-containing compound.
16. The organic film according to item 15, wherein the carboxyl-containing compound is acetic acid, propionic acid, butyric acid, valeric acid, caproic acid, malonic acid, succinic acid, glutaric acid, adipic acid, pimelic acid, (meth)acrylic acid, poly(meth)acrylic acid, benzoic acid, hexahydrobenzoic acid, terephthalic acid, 1,4-cyclohexanedicarboxylic acid, citric acid, malic acid, 3-isopropylmalic acid, itaconic acid, citraconic acid, or a combination thereof.
17. An electroluminescent device, comprising:
   a first electrode supplying holes,
   a second electrode supplying electrons, and
   an organic layer disposed between the first electrode and the second electrode,
   wherein at least one layer of the organic layer includes the organic film according to item 15 or item 16.
18. The electroluminescent device according to item 17, wherein the at least one layer of the organic layer including the organic film is a hole injection layer.
19. The electroluminescent device according to item 17 or item 18, wherein the at least one organic layer comprises a light emitting layer including semiconductor nanoparticles, an organometallic complex, or a combination thereof.
20. The electroluminescent device according to any one of items 17 to 19, wherein light emitting layer includes semiconductor nanoparticles.

### Examples

The present disclosure is described in more detail using the following examples and comparative examples. However, the technical range of the present disclosure is not limited to the following examples. In the following examples, unless specifically described, each operation was performed at room temperature (25 °C). In addition, unless specifically stated, "%" and "a part" mean "weight%" and "a part by weight", respectively.

The ionization potential of the low molecular weight compound and electron affinity of the salt compound are calculated as described below. Here, ΔE is produced by subtracting ionization potential of the low molecular weight compound from the calculated electron affinity of the salt compound.

### Calculation Method

The ionization potential of the low molecular weight compound, electron affinity of the salt compound, and ionization potential of the iodonium cation are determined using Gaussian 16 (Gaussian Inc.) as a calculation software and as the functional B3LYP, and the basis function 6-31G (d, p ) by Density Functional Theory (DFT).

The structural confirmation of the low molecular weight compound is determined by liquid chromatography mass spectrometry (LC/MS). For example, a sample compound was dissolved in tetrahydrofuran to a concentration of 0.1 wt%, and LC-MS measuring device 1260 Infinity-4 quadrupole 6100MS (Agilent Technologies Company Ltd.) was used to analyze the mass.

### Synthesis of Low molecular weight compound

### Synthesis Example 1: Synthesis of Low molecular weight compound 1

Low molecular weight compound 1 is synthesized according to Reaction Scheme 1.

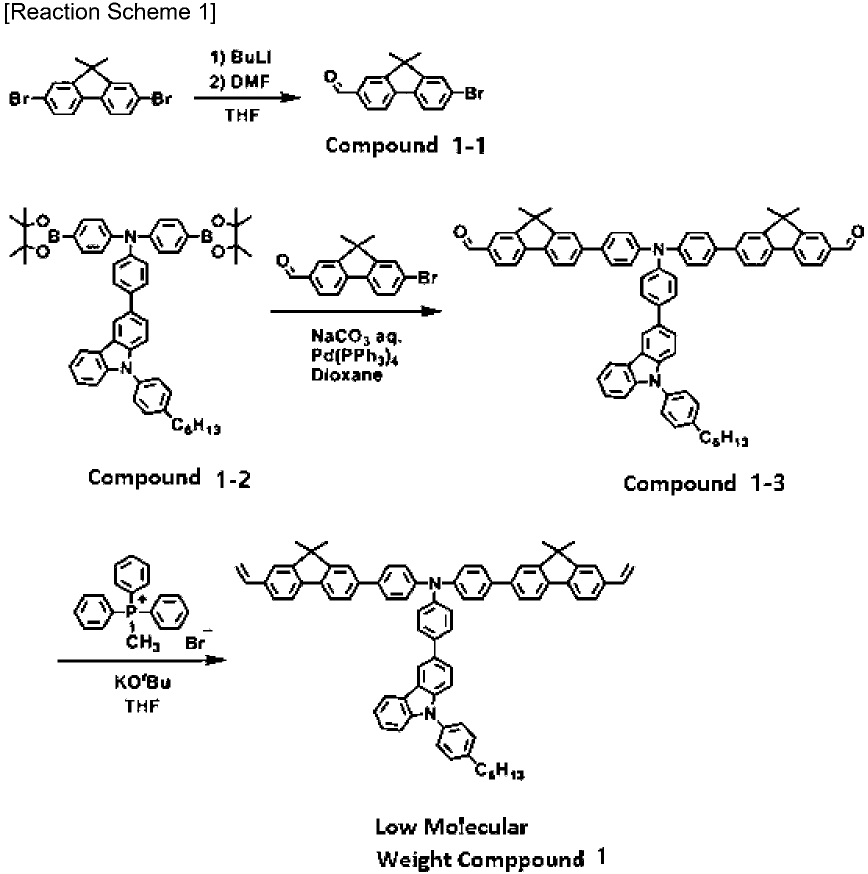

### (Synthesis of Compound 1-1)

Under an argon atmosphere, into a 1000 mL four-neck flask, 2,7-dibromo-9,9-dimethylfluorene (25.0 g) and tetrahydrofuran (THF, anhydrous, 400 mL) were added, and the mixture was stirred and cooled to -78°C. To the resulting solution, a hexane solution of n-butyllithium (1.6 mol/L, 44.4 mL) was added dropwise, and the mixture was stirred at -78°C for 30 minutes. Then, N,N-dimethylformamide (DMF, 16.0 g) was added at -78°C, and the mixture was stirred overnight. Water (200 mL) was then added to the reaction mixture, and liquid-liquid separation was performed. The obtained organic layer was concentrated, and the residue was purified by column chromatography (eluent: toluene/hexane) to afford Compound 1-1 (14.9 g).

### (Synthesis of Compound 1-3)

In a 500 mL four-neck flask replaced with argon, 15.0 g of Compound 1-2 synthesized with reference to Japanese Unexamined Patent Publication No. 2021-138915, 10.9 g of Compound 1-1 obtained above, 5.8 g of sodium carbonate, 0.5 g of tetrakis(triphenylphosphine)palladium(0) (Pd(PPh₃)₄), 200 mL of 1,4-dioxane, and 100 mL of water were added, and the mixture was stirred for 7 hours in a thermostatic bath set at 100°C. Then, the obtained reaction solution was extracted with toluene (100 mL × 2), the organic layer was washed with water (100 mL × 2), and subsequently dried over magnesium sulfate. The solvent was removed under reduced pressure, and the resulting residue was purified by column chromatography (eluent: toluene/hexane) to afford Compound 1-3 (11.2 g).

### (Synthesis of Low molecular weight compound 1)

Methyl triphenylphosphonium bromide (3.9 g), potassium tert-butoxide (1.4 g), and tetrahydrofuran (dehydrated, 100 mL) were added to a 300 mL-4-neck flask replaced with argon, and cooled to 0°C while stirring. In the resulting mixed solution, tetrahydrofuran solution (50 mL of dehydration) of Compound 1-3 (5.0 g) obtained from the above was dripped at 0 °C, and stirred for 3 hours (h) at room temperature after dripping. Water (200 mL) was then added to the obtained reaction solution, and liquid-liquid separation was performed. The resulting organic layer was concentrated and purified by column chromatography (eluent: toluene/hexane) and reprecipitation (solvent: THF/methanol) to obtain the low molecular weight compound 1 (3.2 g). LC-MS: 1007([M+H]⁺).

### Synthesis Example 2: Synthesis of Low molecular weight compound 2

Low molecular weight compound 2 was prepared according to Reaction Scheme 2.

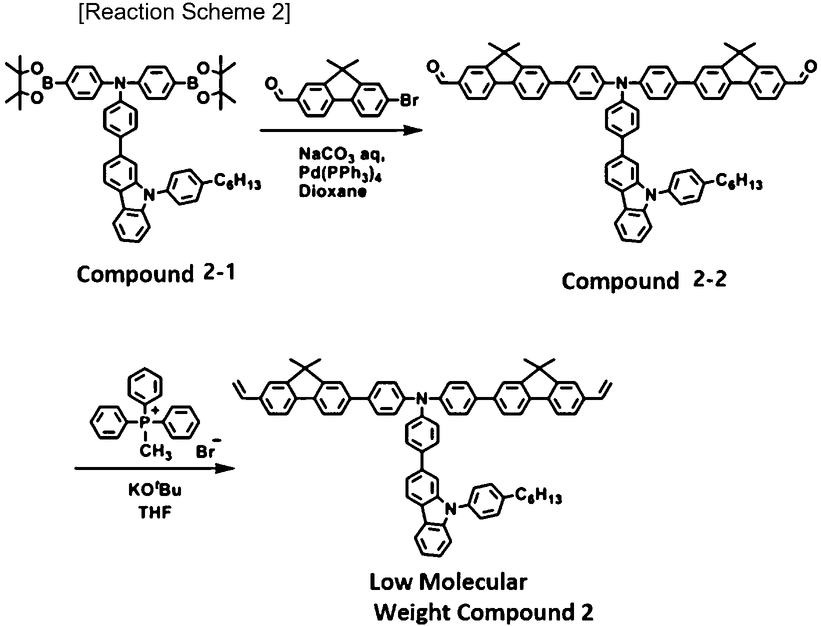

### (Synthesis of Compound 2-2)

To a 500 mL four-neck flask substituted with argon, 15.0 g of Compound 2-1 synthesized with reference to Japanese Patent Laid-open Publication No. 2021-138915, 10.9 g of Compound 1-1 obtained in Synthetic Example 1, 5.8 g of sodium carbonate, 0.5 g of tetrakis(triphenylphosphine)palladium(0) (Pd(PPh₃)₄), 200 mL of 1,4-dioxane, and 100 mL of water were added, and the mixture was stirred for 7 hours in a thermostatic bath set at 100°C. The resulting reaction solution was extracted with toluene (100 mL × 2), the organic layer was washed with water (100 mL × 2), then dried over magnesium sulfate, and the solvent was removed under reduced pressure. The residue obtained was purified by column chromatography (eluent: toluene/hexane) to obtain Compound 2-2 (9.8 g).

### (Synthesis of Low molecular weight compound 2)

Into a 300 mL 4-neck flask substituted with argon, methyltriphenylphosphonium bromide (3.9 g), potassium tert-butoxide (1.4 g), and tetrahydrofuran (anhydrous, 100 mL) were added, and the mixture was stirred while cooling to 0°C. To the resulting mixture, a tetrahydrofuran solution (anhydrous, 50 mL) of Compound 2-2 (5.0 g) obtained above was added dropwise, and after the addition, the mixture was stirred at room temperature for 3 hours. Water (200 mL) was then added to the resulting reaction solution, and liquid-liquid separation was performed. The obtained organic layer was concentrated, and the residue was purified by column chromatography (toluene/hexane) and reprecipitation (THF/methanol) to obtain the Low molecular weight compound 2 (3.6 g). LC-MS: 1007([M+H]⁺).

### Synthesis Example 3: Synthesis of Low molecular weight compound 3

Low molecular weight compound 3 was synthesized according to Reaction Scheme 3.

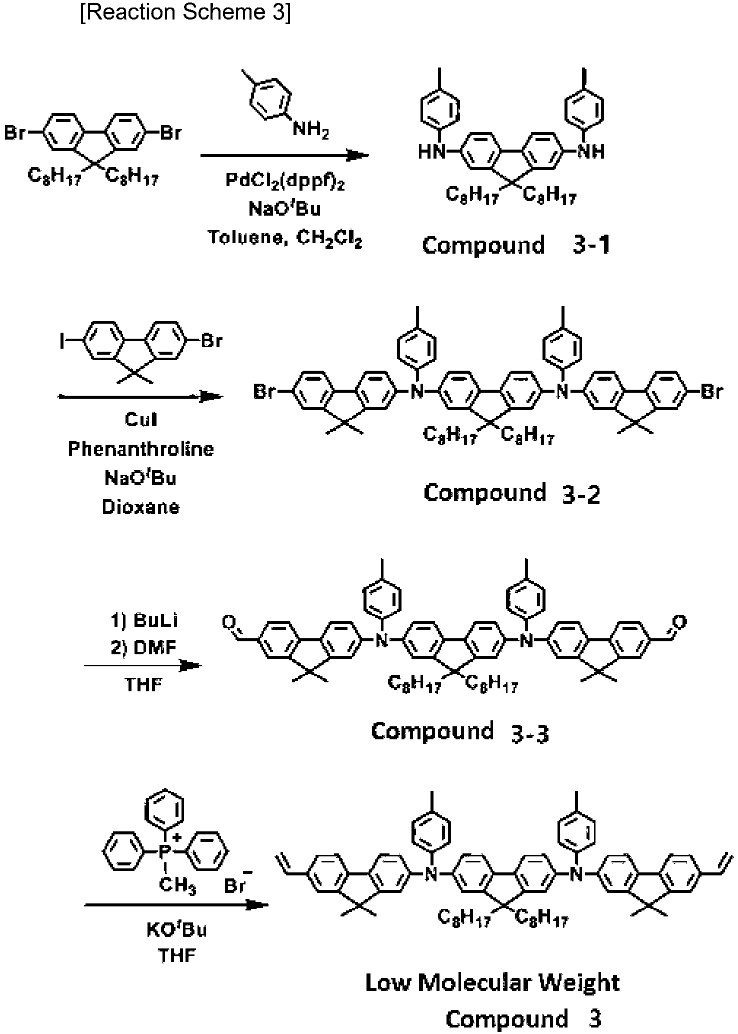

### (Synthesis of Compound 3-1)

Into a 500 mL three-neck flask, which had been substituted with argon, were added 9,9-Dibromo-2,7-dihexylfluorene (20.0 g), 4-methylaniline (9.1 g), [1,1'-bis(diphenylphosphino)ferrocene]dichloropalladium(lI) (PdCl₂(dppf)₂·CH₂Cl₂) (0.7 g), sodium tert-butoxide (t-BuONa, 11.7 g), and toluene (250 mL), and the mixture was stirred for 5 hours in a thermostatic bath set at 120°C. Afterward, the reaction solution was cooled to room temperature, and insoluble materials were removed by filtration using celite as a filtration aid. The resulting solution was concentrated under reduced pressure to remove the solvent, and the residue was purified by column chromatography (eluent: toluene/hexane) to obtain Compound 3-1 (15.1 g).

### (Synthesis of Compound 3-2)

In a 100 mL, 3-neck flask substituted with argon, Compound 3-1 (10.0 g) obtained above, 2-bromo-7-iodo-9,9-dimethyl-9H-fluorene (14.0 g), copper(I) iodide (Cul, 0.3 g), phenanthroline (0.6 g), sodium tert-butoxide (t-BuONa, 4.8 g), and 1,4-dioxane (20 mL) were added and stirred in a thermostatic bath set at 110°C for 18 hours. Then, the reaction solution was cooled to room temperature, and insoluble substances were removed using celite as a filtration agent. The obtained solution was subjected to reduced pressure distillation to remove the solvent, and the residue was dissolved in a mixed solvent of toluene (100 mL) and hexane (100 mL), followed by the addition of activated carbon (20.0 g) and refluxing for 30 minutes. Then, for the obtained mixture, insoluble materials were filtered using celite as a filtration agent, and the solvent was removed from the obtained solution by reduced pressure distillation. The residue was purified by column chromatography (eluent: toluene/hexane) to obtain Compound 3-2 (10.6 g).

### (Synthesis of Compound 3-3)

In a 200 mL-4-neck flask, which is substituted with argon, Compound 3-2 (5.0 g) obtained from the above and tetrahydrofuran (dehydrated, 50 mL) were added and cooled to -78 °C while stirring. A hexane solution (1.6 mol/L, 2.7 mL) of n-butyllithium was dripped and stirred for 30 min at -78 °C. In the resulting mixed solution, N,N-dimethyl formamide (1.0 g) was dripped at -78 °C and stirred overnight. After that, water (50 mL) was added to the obtained reaction solution, and liquid-liquid separation was performed. The resulting organic layer was concentrated and purified by column chromatography (eluent: toluene/hexane) to obtain Compounds 3-3 (3.2 g).

### (Synthesis of Low molecular weight compound 3)

In a 100 mL four-neck flask replaced with argon, methyltriphenylphosphonium bromide (2.6 g), potassium tert-butoxide (0.8 g), and tetrahydrofuran (dehydrated, 20 mL) were added and stirred while cooling to 0°C. To the resulting mixed solution, a tetrahydrofuran solution (dehydrated, 20 mL) of Compound 3-3 (3.0 g) obtained above was added dropwise, and after the addition, it was stirred at room temperature for 3 hours. Water (100 mL) was added to the resulting reaction solution, and liquid-liquid separation was performed. The obtained organic layer was concentrated, and the residue was purified by column chromatography (eluent: toluene/hexane) and recrystallization (THF/methanol) to obtain the Low molecular weight compound 3 (2.8 g). LC-MS: 1037 ([M+H]⁺).

### Synthesis Example 4: Synthesis of Low molecular weight compound 4

Low molecular weight compound 4 was prepared according to Reaction Scheme 4.

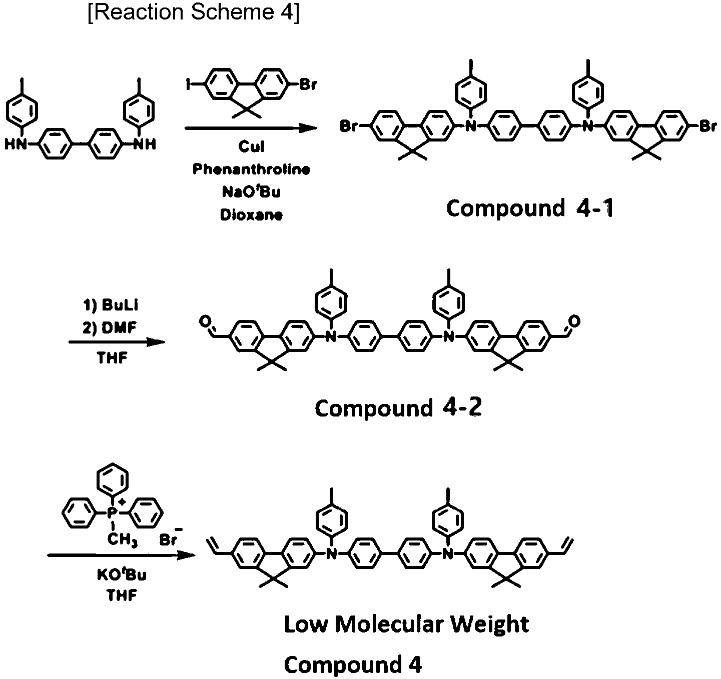

### (Synthesis of Compound 4-1)

In a 300 mL three-neck flask substituted with argon, N,N'-di-p-tolylbenzidine (20.0 g), 2-bromo-7-iodo-9,9-dimethyl-9H-fluorene (46.0 g), copper(I) iodide (Cul, 1.0 g), phenanthroline (2.9 g), sodium tert-butoxide (t-BuONa, 15.8 g), and 1,4-dioxane (60 mL) were added and stirred in a thermostatic bath set to 110°C for 20 hours. The resulting reaction solution was cooled to room temperature, and insoluble substances were removed by filtration through celite. The obtained solution was subjected to solvent removal by reduced-pressure distillation, and the residue was dissolved in a mixed solvent of toluene (300 mL) and hexane (100 mL), activated carbon (20.0 g) was added, and the mixture was refluxed for 30 minutes. Thereafter, the obtained mixture was filtered using celite as a filtration aid to remove insoluble materials, and the solvent was removed from the resulting solution by vacuum distillation. The residue was purified by column chromatography (eluent: toluene/hexane) to obtain Compound 4-1 (42.5 g).

### (Synthesis of Compound 4-2)

In a 500 mL, 4-neck flask substituted with argon, the obtained compound 4-1 (20.0 g) and tetrahydrofuran (anhydrous, 220 mL) were added and cooled to -78°C while stirring. To the resulting mixture, a hexane solution of n-butyllithium (1.6 mol/L, 13.6 mL) was added dropwise and stirred at -78°C for 30 minutes. Then, N,N-dimethylformamide (4.8 g) was added at -78°C and stirred overnight. Afterward, water (50 mL) was added to the obtained reaction solution, and the mixture was subjected to liquid-liquid separation. The resulting organic layer was concentrated, and the residue was purified by column chromatography (eluent: toluene/hexane) to obtain compound 4-2 (14.6 g).

### (Synthesis of Low molecular weight compound 4)

In a 300 mL, 4-neck flask substituted with argon, methyltriphenylphosphonium bromide (5.5 g), potassium tert-butoxide (1.7 g), and tetrahydrofuran (anhydrous, 31 mL) were added and the mixture was stirred while cooling to 0°C. To the resulting mixture, a tetrahydrofuran solution (anhydrous, 40 mL) of the previously obtained Compound 3-3 (5.0 g) was added dropwise, and after completion of the addition, the mixture was stirred at room temperature for 3 hours. Water (150 mL) was added to the resulting reaction mixture, and the solution was subjected to liquid-liquid separation. The obtained organic layer was concentrated, and the residue was purified by column chromatography (eluent: toluene/hexane) and reprecipitation (THF/methanol) to afford the low molecular weight compound 4 (4.2 g). LC-MS: 801 ([M+H]⁺).

### Synthesis Example 5: Synthesis of Low molecular weight compound 5

Low molecular weight compound 5 was prepared according to Reaction Scheme 5.

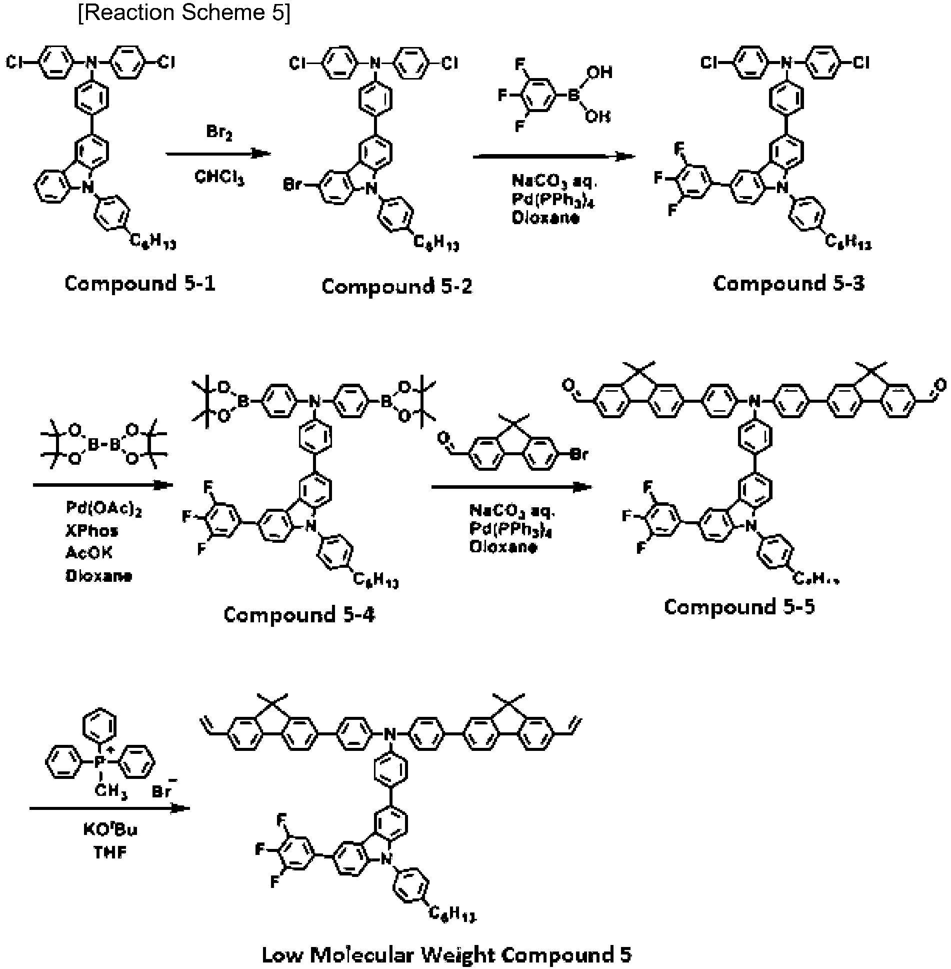

### (Synthesis of Compound 5-2)

Into a 500 mL four-neck flask substituted with argon, Compound 5-1 (20.0 g) synthesized with reference to Japanese Laid-open Publication No. 2021-138915 and chloroform (300 mL) were added and cooled to -10°C while stirring. Bromine (1.6 mL) was added dropwise to the resulting mixture, and the reaction was stirred at room temperature for 3 hours. To the resulting reaction solution, an aqueous solution of sodium thiosulfate was added, and liquid-liquid extraction was performed. The obtained organic layer was concentrated, and the residue was purified by column chromatography (eluent: toluene/hexane) to obtain Compound 5-2 (19.1 g).

### (Synthesis of Compound 5-3)

In a 500 mL four-neck flask substituted with argon, the Compound 5-1 (15.0 g) obtained above, 3,4,5-trifluorophenylboronic acid (4.4 g), sodium carbonate (4.3 g), tetrakis(triphenylphosphine)palladium(0) (Pd(PPh₃)₄, 0.6 g), 1,4-dioxane (200 mL), and water (100 mL) were added, and stirred for 8 hours in a thermostatic bath set at 100°C. After cooling the resulting reaction solution to room temperature, it was extracted with toluene (100 mL × 2), the organic layer was washed with water (100 mL × 2), dried over magnesium sulfate, and the solvent was removed under reduced pressure by distillation. The obtained residue was purified by column chromatography (eluent: toluene/hexane) to give Compound 5-3 (13.4 g).

### (Synthesis of Compound 5-4)

In a 200 mL three-neck flask substituted with argon, the Compound 5-3 obtained above (10.0 g), bispinacol diborate (10.1 g), potassium acetate (AcOK, 4.5 g), palladium acetate (0.2 g), 2-dicyclohexylphosphino-2',4',6'-triisopropylbiphenyl (XPhos, 0.6 g), and 1,4-dioxane (70 mL) were added and stirred for 3 hours in a thermostatic bath set to 110°C. Afterward, the resulting reaction solution was cooled to room temperature, and insolubles were removed by filtration using celite as the filter aid. The solvent was removed from the solution under reduced pressure by distillation, and the residue was dissolved in a mixed solvent of toluene (100 mL) and hexane (100 mL), activated carbon (20.0 g) was added, and the mixture was refluxed for 30 minutes. Then, the obtained mixture was filtered using celite as the filter aid to remove insolubles, and the solvent was removed under reduced pressure by distillation. The residue was purified by recrystallization (toluene/ethanol) to obtain Compound 5-4 (11.1 g).

### (Synthesis of Compound 5-5)

To a 300 mL four-neck flask substituted with argon, were added the Compound 5-4 obtained above (10.0 g), the Compound 1-1 obtained in Synthetic Example 1 (6.3 g), sodium carbonate (3.3 g), tetrakis(triphenylphosphine)palladium(0) (Pd(PPh₃)₄, 0.3 g), 1,4-dioxane (150 mL), and water (70 mL), and the mixture was stirred in a thermostatic bath set at 100°C for 7 hours. Thereafter, the reaction solution was extracted with toluene (100 mL × 2), the organic layer was washed with water (100 mL × 2), dried over magnesium sulfate, and the solvent was removed by distillation under reduced pressure. The resulting residue was purified by column chromatography (eluent: toluene/hexane) to obtain Compound 5-5 (8.4 g).

### (Synthesis of Low molecular weight compound 5)

In a 300 mL, 4-neck flask substituted with argon, methyltriphenylphosphonium bromide (5.5 g), potassium tert-butoxide (1.9 g), and tetrahydrofuran (anhydrous, 50 mL) were added, and the mixture was stirred while cooling to 0°C. A tetrahydrofuran solution (anhydrous, 20 mL) of Compound 5-5 (8.0 g) obtained above was added dropwise, and after the addition, the mixture was stirred at room temperature for 3 hours. Then, water (150 mL) was added to the resulting reaction solution, and a liquid-liquid extraction was performed. The obtained organic layer was concentrated, and the residue was purified by column chromatography (eluent: toluene/hexane) and reprecipitation (THF/methanol) to obtain the low molecular weight compound 5 (5.1 g). LC-MS: 1137 ([M+H]⁺).

### Synthesis of Dopant (salt compound)

### Synthesis of Dopant D-1

Dopant D-1 was prepared according to Reaction Scheme 6.

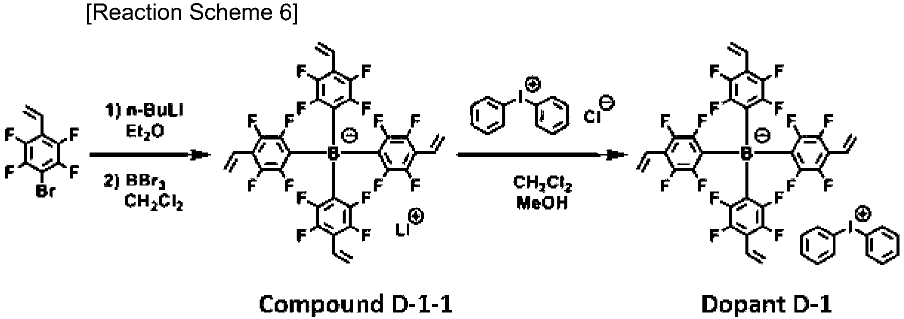

In a 100 mL four-neck flask replaced with argon, 1-bromo-2,3,5,6-tetrafluoro-4-vinylbenzene (5.00 g) and diethyl ether (60 mL) were added, and the mixture was stirred under an argon atmosphere and cooled to -78°C. A hexane solution of n-butyllithium (1.6 mol/L, 14.3 mL) was added dropwise, and the mixture was stirred at -78°C for 30 minutes. Next, a dichloromethane solution of 3-bromoborane (1 mol/L, 4.1 mL) was added dropwise to the reaction solution at -78°C, and the mixture was stirred overnight. Water (35 mL) was then added to the resulting reaction mixture, and a liquid-liquid extraction was performed. The obtained organic layer was concentrated to afford an off-white solid (3.78 g). The obtained solid was purified by column chromatography (dichloromethane/acetonitrile), yielding Compound D-1-1 as a white solid (2.09 g). The obtained Compound D-1-1 and diphenyliodonium chloride (0.92 g) were dissolved in a mixed solvent of dichloromethane/methanol (dichloromethane:methanol = 10:1 (v/v), 40 mL) and stirred for 1 hour. Water (35 mL) was added to the resulting mixed solution, and then the organic layer was washed. After removing the solvent by distillation, purification was performed by column chromatography (eluent: dichloromethane/acetonitrile), yielding Dopant D-1 (1.63 g).

### Synthesis of Dopant D-2

Dopant D-2 was prepared according to Reaction Scheme 7.

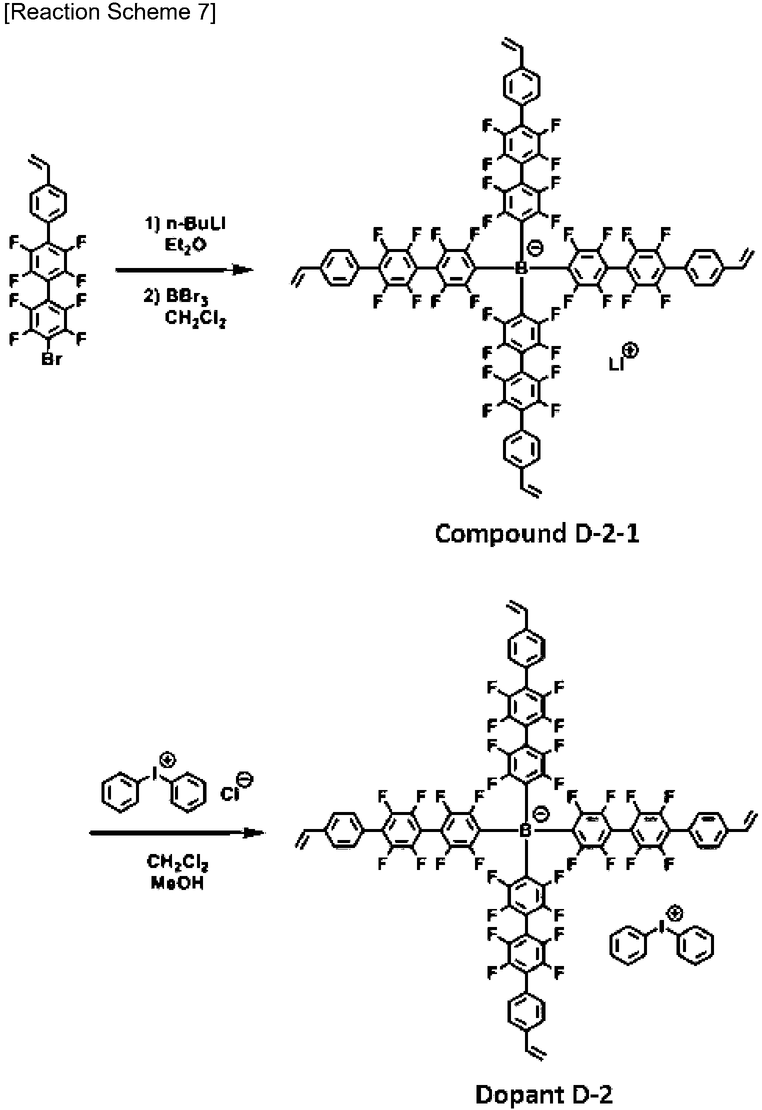

Into a 100 mL, 4-neck flask substituted with argon, 4-bromo-2,2',3,3',5,5',6,6'-octafluoro-4"-vinyl-1,1':4',1"-terphenyl (2.20 g) and diethyl ether (60 mL) were added, and the mixture was stirred under an argon atmosphere while cooling to -78°C. A hexane solution of n-butyllithium (1.6 mol/L, 3.20 mL) was added dropwise, and the mixture was stirred at -78°C for 30 minutes. Then, a dichloromethane solution of 3-bromoborane (1 mol/L, 0.99 mL) was added dropwise to the reaction solution at -78°C, and the mixture was stirred overnight. Afterwards, water (45 mL) was added to the resulting reaction solution, and a liquid-liquid extraction was performed. The obtained organic layer was concentrated to afford an off-white solid. The solid was purified by column chromatography (eluent: dichloromethane/acetonitrile) to obtain Compound D-2-1 as a white solid (0.93 g). The obtained Compound D-2-1 and diphenyliodonium chloride (0.18 g) were dissolved in a mixed solvent of dichloromethane/methanol (dichloromethane:methanol = 10:1 (v/v), 20 mL) and stirred for 1 hour. Water (20 mL) was then added to the resulting reaction solution, and the organic layer was washed. After removing the solvent by distillation, the mixture was purified by column chromatography (dichloromethane/acetonitrile) to obtain dopant D-2 (0.40 g).

### Synthesis of Dopant D-3

Dopant D-3 was prepared according to Reaction Scheme 8.

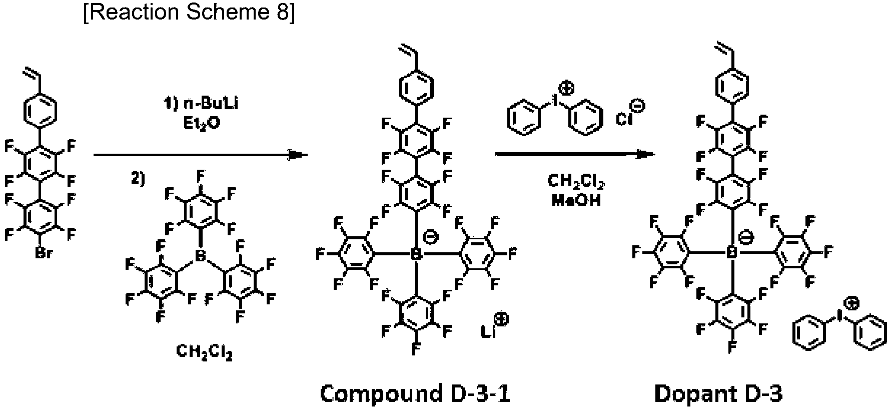

Into a 100 mL, 4-neck flask substituted with argon, 4-bromo-2,2',3,3',5,5',6,6'-octafluoro-4"-vinyl-1,1':4',1"-terphenyl (1.00 g) and diethyl ether (27 mL) were added, and the mixture was stirred under an argon atmosphere and cooled to -78°C. A solution of n-butyllithium in hexane (1.6 mol/L, 1.4 mL) was added dropwise, and the mixture was stirred at -78°C for 30 minutes. Next, tris(pentafluorophenyl)borane (0.942 g) was dissolved in dichloromethane (3.8 mL), added dropwise at -78°C, and the mixture was stirred overnight. Thereafter, water (20 mL) was added to the resulting reaction solution, and a liquid-liquid extraction was performed. The obtained organic layer was concentrated to yield an off-white solid. This solid was purified by column chromatography (eluent: dichloromethane/acetonitrile) to obtain Compound D-3-1 as a white solid (1.14 g). Next, the obtained Compound D-3-1 and diphenyliodonium chloride (0.58 g) were dissolved in a mixed solvent of dichloromethane/methanol (dichloromethane: methanol = 10:1 (v/v), 20 mL) and stirred for 1 hour. Water (10 mL) was added to the resulting reaction solution, and then the organic layer was washed. After removing the solvent by distillation, purification was carried out by column chromatography (eluent: dichloromethane/acetonitrile) to obtain Dopant D-3 (1.14 g).

### Synthesis of Dopant D-4

Dopant D-4 was prepared according to Reaction Scheme 9.

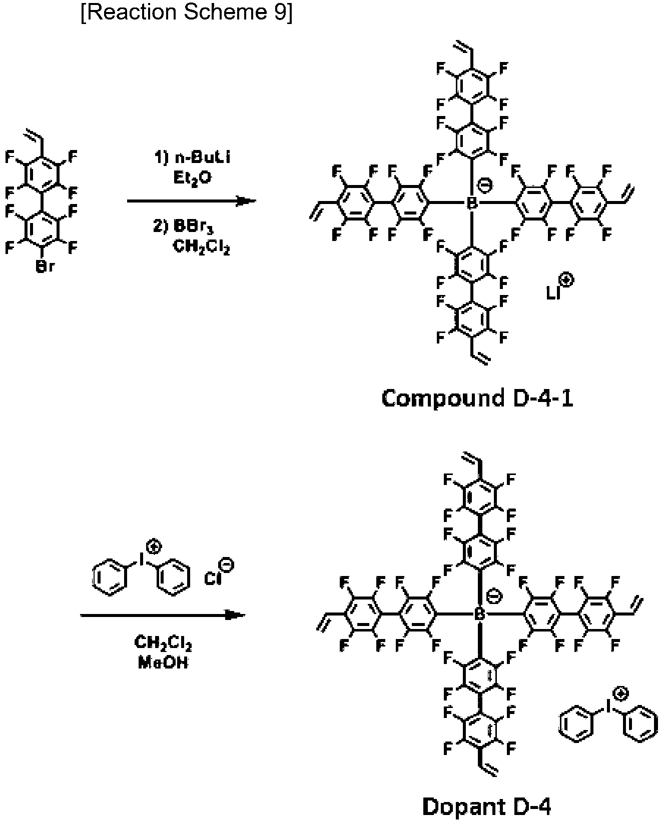

In a 100 mL four-neck flask replaced with argon, 4-bromo-2,2',3,3',5,5',6,6'-octafluoro-4"-vinyl-1,1'-biphenyl (5.00 g) and diethyl ether (45 mL) were added, and the mixture was stirred under an argon atmosphere while being cooled to -78°C. A hexane solution of n-butyllithium (1.6 mol/L, 9.0 mL) was added dropwise, and the mixture was stirred at -78°C for 30 minutes. Next, a dichloromethane solution of 3-bromoborane (1 mol/L, 2.57 mL) was added dropwise at -78°C and the mixture was stirred overnight. Afterwards, water (22 mL) was added to the obtained reaction solution, and the mixture was subjected to liquid-liquid extraction. The resulting organic layer was concentrated to obtain an off-white solid. The obtained solid was purified by column chromatography (eluent: dichloromethane/acetonitrile) to give Compound D-4-1 as a white solid (2.21 g). Next, Compound D-4-1 obtained and diphenyliodonium chloride (0.53 g) were dissolved in a mixed solvent of dichloromethane/methanol (dichloromethane: methanol = 10:1 (v/v), 20 mL) and stirred for 1 hour. Water (10 mL) was added to the resulting reaction solution, and the organic layer was washed. After removing the solvent by distillation, purification was carried out by column chromatography (eluent: dichloromethane/acetonitrile) to obtain Dopant D-4 (1.73 g).

### Synthesis of Dopant D-5

Dopant D-5 was prepared according to Reaction Scheme 10.

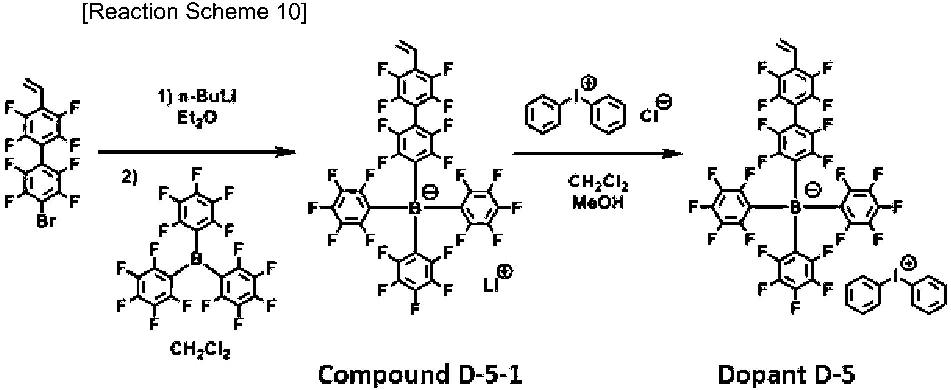

Into a 100 mL four-neck flask replaced with argon, 4-bromo-2,2',3,3',5,5',6,6'-octafluoro-4"-vinyl-1,1'-biphenyl (2.00 g) and diethyl ether (65 mL) were added, and the mixture was stirred under an argon atmosphere while cooling to -78°C. A solution of n-butyllithium in hexane (1.6 mol/L, 3.3 mL) was added dropwise and stirred for about 30 minutes while maintaining the internal temperature. Then, tris(pentafluorophenyl)borane (2.24 g) was dissolved in dichloromethane (mL) and added dropwise while maintaining the internal temperature, and the mixture was stirred overnight. Next, water (30 mL) was added to the reaction mixture, a liquid-liquid extraction was performed, and the organic layer was concentrated to obtain an off-white solid. The solid was subjected to column chromatography using dichloromethane/acetonitrile as the eluent to give a white solid D-5-1 (2.24 g). The white solid and diphenyliodonium chloride (0.83 g) were dissolved in a mixed solvent of dichloromethane/methanol (dichloromethane: methanol = 10/1 (v/v, 30 mL)) and stirred for 1 hour. Water (15 mL) was then added, and the organic layer was washed. After removing the solvent by distillation, column chromatography was performed using dichloromethane/acetonitrile as the eluent to obtain Dopant D-5 (1.68 g).

### Example 1

### Preparation of Composition 1, Fabrication of Electroluminescent Device 1, and Positive Aging

As a first electrode(anode), a glass substrate attached with a patterned indium tin oxide (ITO) film having a thickness of 150 nm was provided. The ITO attached glass substrate was washed with neutral detergent, deionized water, water, and isopropyl alcohol in order, and treated with UV-ozone processing.

A solution of low molecular weight compound 1 prepared in Synthesis Example 1 was prepared by dissolving it at a concentration of 3 wt% in a mixed solvent of xylene and butyl benzoate (xylene: butyl benzoate = 100:10 by volume). Separately, another solution was prepared by dissolving 4-isopropyl-4'-methyldiphenyliodonium tetrakis(pentafluorophenyl)borate (PIPB, manufactured by Tokyo Kasei Kogyo Kabushiki Kaisha), represented by the chemical formula below, at a concentration of 3 wt% in a mixed solvent of xylene and butyl benzoate (xylene: butyl benzoate = 100:10 by volume). The two solutions were mixed so that the weight ratio of low molecular weight compound 1 to PIPB was 90:10, thereby preparing Composition 1 (Coating Solution 1 for forming a hole injection layer). The electron affinity of the borate anion possessed by the above PIPB was 6.29 eV.

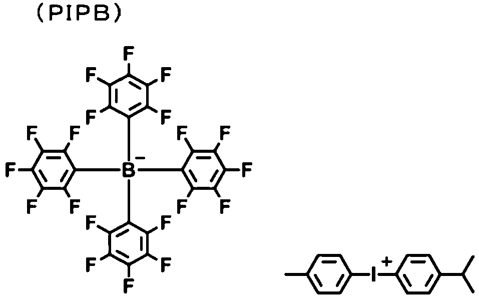

Next, on the above-mentioned ITO-attached glass substrate, Composition 1 (a coating solution for forming a hole injection layer) was applied by a spin-coating method to a thickness (dry film thickness) of 100 nm, then heated at 230°C for 30 minutes to form a hole injection layer with a thickness (dry film thickness) of 100 nm.

Subsequently, HTP-1 (the polymer compound P-3 described in Japanese Laid-open Publication No. 2021-138915), a hole transport material represented by the chemical formula below, was dissolved in xylene (solvent) at a concentration of 1 wt% to prepare a coating solution for forming a hole transport layer. On the hole injection layer formed above, the coating solution for forming the hole transport layer was applied by a spin-coating method to a thickness (dry film thickness) of 25 nm, then heated at 230°C for 30 minutes to form a hole transport layer with a thickness (dry film thickness) of 25 nm.

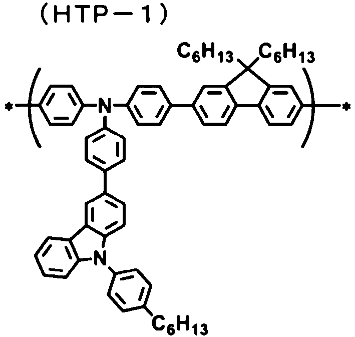

Next, blue quantum dots, ZnTeSe/ZnSe/ZnS (core/shell/shell; average diameter = 10 nm), were dispersed in octane at a concentration of 1.0 wt% to prepare a quantum dot dispersion. This quantum dot dispersion was applied onto the hole transport layer by spin coating so that the dry film thickness would be 25 nm, and then dried. As a result, a light emitting layer with a thickness (dry film thickness) of 25 nm was formed on the hole transport layer. When ultraviolet light was irradiated onto the quantum dot dispersion, the emitted light had a peak wavelength of 462 nm and a full width at half maximum (FWHM) of 30 nm.

The light emitting layer was fully dried.

Next, a dispersion of ZnMgO (solvent: ethanol, concentration: 10 wt%) was spin-coated onto the thermally treated quantum dot emitting layer, and heat-treated at 80°C to form an electron transport layer with a thickness of 20 nm. Aluminum (Al) was vacuum-deposited on the fabricated electron transport layer to form a second electrode with a thickness of 100 nm, creating a laminated structure and producing quantum dot electroluminescent device 1.

On the second electrode of the laminated structure, LOCTITE^{®} AA366 (manufactured by Henkel, containing methacrylate ester, acrylic acid, methacrylic acid, etc.) was applied, and ultraviolet light (wavelength: 365 nm, cumulative dose: 10 mJ/cm²) was irradiated to form an acid compound layer (thickness: 1.5 µm), resulting in quantum dot electroluminescent device 1 before treatment (before positive aging treatment). This was placed in a constant temperature oven at 70°C for 5 days to obtain quantum dot electroluminescent device 1 after treatment (after positive aging treatment).

### Fabricating MSM (metal-semiconductor-metal) device 1, and Positive Aging

As a first electrode(anode), a glass substrate attached with a patterned indium tin oxide (ITO) film having a thickness of 150 nm was provided. The ITO attached glass substrate was washed with neutral detergent, deionized water, water, and isopropyl alcohol in order, and treated with UV-ozone processing.

A solution of low molecular weight compound 1 synthesized in Synthetic Example 1 was dissolved in a mixed solvent of xylene and butyl benzoate (xylene: butyl benzoate = 100:10 by volume) at a concentration of 3 wt%. Separately, another solution was prepared by dissolving 4-isopropyl-4'-methyl diphenyliodonium tetrakis(pentafluorophenyl)borate

(PIPB, manufactured by Tokyo Chemical Industry Co., Ltd., see the above chemical formula) in a mixed solvent of xylene and butyl benzoate (xylene: butyl benzoate = 100:10 by volume) at a concentration of 3 wt%. The two solutions were mixed so that the mass ratio of low molecular weight compound 1 to PIPB was 90:10, thereby preparing Composition 1 (coating solution 1 for a hole injection layer).

Next, Composition 1 (coating solution for forming a hole injection layer) was applied onto the above-mentioned ITO-attached glass substrate by spin coating to a thickness (dry film thickness) of 100 nm, and then heated at 230°C for 30 minutes, thereby forming a hole injection layer with a thickness (dry film thickness) of 100 nm.

Using a vacuum deposition device, a mixed film of silver and magnesium (hereinafter, also referred to as 'AgMg') was deposited by the vacuum deposition device on the hole injection layer formed above, forming a second electrode (cathode) with a thickness of 20 nm, thereby producing MSM device 1.

Next, on the second electrode of this laminated structure, LOCTITE^{®} AA366 (manufactured by Henkel, containing methacrylic ester, acrylic acid, methacrylic acid, etc.) was applied, and ultraviolet light (wavelength: 365 nm, total irradiation: 10 mJ/cm²) was irradiated to form an acid compound layer (thickness: 1.5 µm), obtaining MSM device 1 before treatment (before positive aging treatment). This was then placed in a constant temperature oven at 70°C for 5 days to obtain MSM device 1 after treatment (after positive aging treatment).

### Examples 2 to 11

Except for changing the types of low molecular compounds and salt compounds as listed in Table 1 below, the same procedure as in Example 1 was followed to fabricate Example compositions 2 to 11, quantum dot electroluminescent devices 2 to 11, quantum dot electroluminescent devices 2 to 11 before treatment, quantum dot electroluminescent devices 2 to 11 after treatment, MSM devices 2 to 11, MSM devices 2 to 11 before treatment, and MSM devices 2 to 11 after treatment, respectively.

### Comparative Example 1

Except for using the compound HT-1 shown below instead of the low molecular weight compound 3, Comparative Composition 1, Comparative Electroluminescent Device 1, Comparative Pre-Treatment Electroluminescent Device 1, Comparative Post-Treatment Electroluminescent Device 1, Comparative MSM Device 1, Comparative Pre-Treatment MSM Device 1, and Comparative Post-Treatment MSM Device 1 were prepared in the same manner as in Example 5. The compound HT-1 was synthesized with reference to the description in ACS Materials Lett. 2023, 5, pp. 3184-3191.

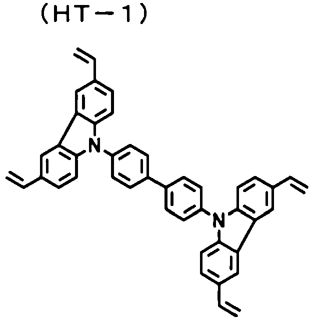

### Comparative Example 2

Except that the low molecular weight compound HT-2 shown below was used instead of the low molecular weight compound 1, the same procedure as in Example 1 was followed to produce Comparative Composition 2, Comparative Electroluminescent Device 2, Comparative Pre-Treatment Electroluminescent Device 2, Comparative Post-Treatment Electroluminescent Device 2, Comparative MSM Device 2, Comparative Pre-Treatment MSM Device 2, and Comparative Post-Treatment MSM Device 2.

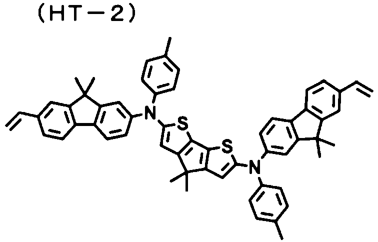

The above low molecular weight compound HT -2 was represented by the following Reaction Scheme 11.

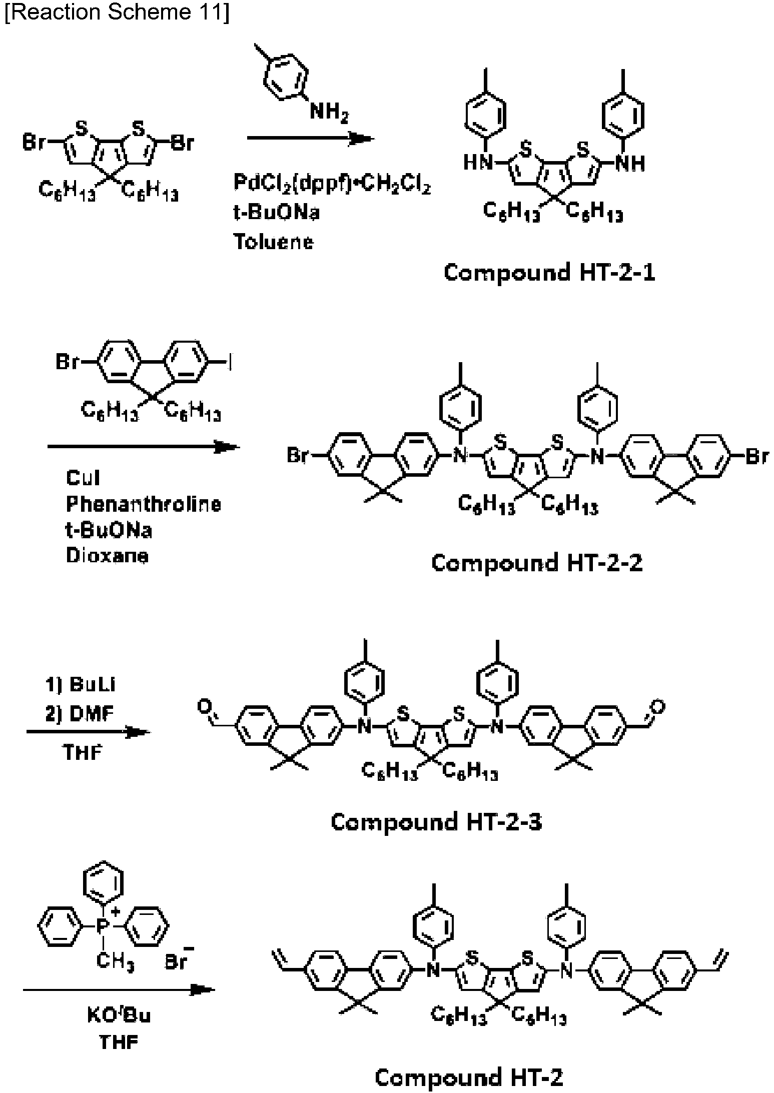

### (Synthesis of Compound HT-2-1)

In a 300 mL, 3-neck flask substituted with argon, 2,6-dibromo-4,4-dihexyl-4H-cyclopenta[2,1-b:3,4-b']dithiophene (10.0 g), p-toluidine (4.5 g), [1,1'-bis(diphenylphosphino)ferrocene]dichloropalladium(II) dichloromethane adduct (0.3 g), sodium tert-butoxide (5.7 g), and toluene (200 mL) were added and stirred at 120°C (bath temperature) for 12 hours. After cooling to room temperature, the precipitate was removed by filtration using celite as a filter aid. The solvent was removed under reduced pressure, and the residue was purified by silica gel column chromatography (eluent: toluene/hexane) to obtain Compound HT-2-1 (7.5 g).

### (Synthesis of Compound HT-2-2)

In a 300 mL three-neck flask substituted with argon, the Compound HT-2-1 (5.0 g) obtained above, 2-bromo-9,9-dimethyl-7-iodo-9H-fluorene (7.6 g), cuprous iodide (0.2 g), phenanthroline (0.3 g), sodium tert-butoxide (2.6 g), and 1,4-dioxane (10 mL) were added and stirred at 110°C (bath temperature) for 18 hours. After cooling to room temperature, insoluble substances were removed using celite as a filtration aid. The solvent was removed under reduced pressure by distillation, and the residue was dissolved in a mixed solvent of toluene (100 mL) and hexane (100 mL), activated charcoal (5.0 g) was added, and the mixture was refluxed for 30 minutes. The insoluble substances were filtered using celite as a filtration aid. The solvent was removed under reduced pressure by distillation, and the residue was purified by silica gel column chromatography (eluent: toluene/hexane) to obtain compound HT-2-2 (8.3 g).

### (Synthesis of Compound HT-2-3)

Into a 200 mL four-neck flask substituted with argon, were added the Compound HT-2-2 (5.0 g) obtained above and tetrahydrofuran (dehydrated, 50 mL), and the mixture was stirred while cooling to -78°C. A hexane solution of n-butyllithium (1.6 mol/L, 2.8 mL) was added dropwise, and the mixture was stirred at -78°C for 30 minutes. To the resulting mixture, N,N-dimethylformamide (1.0 g) was added at -78°C, and the mixture was stirred overnight. Thereafter, water (50 mL) was added to the resulting reaction solution, and a liquid-liquid extraction was performed. The obtained organic layer was concentrated, and the residue was purified by column chromatography (eluent: toluene/hexane) to give the Compound HT-2-3 (4.3 g).

### (Synthesis of Compound HT-2)

In a 100 mL, 4-neck flask substituted with argon, methyltriphenylphosphonium bromide (3.1 g), potassium t-butoxide (1.1 g), and tetrahydrofuran (anhydrous, 25 mL) were added and cooled to 0°C while stirring. To the resulting mixture, a tetrahydrofuran solution (anhydrous, 10 mL) of the Compound HT-2-3 (4.0 g) obtained above was added dropwise, and after addition, the mixture was stirred at room temperature for 3 hours. Water (100 mL) was added to the resulting reaction solution, and a liquid-liquid extraction was performed. The obtained organic layer was concentrated, and the residue was purified by column chromatography (eluent: toluene/hexane) and reprecipitation (THF/methanol) to afford the Compound HT-2 (3.6 g). LC-MS: 993 ([M+H]⁺).

### Evaluation of Conductivity Retention Rate

For each MSM device fabricated as described above, the conductivity retention rate was evaluated by the following method.

When a voltage is applied to the MSM device, current begins to flow at a certain voltage. Using a DC constant voltage power supply (manufactured by Kabushiki Kaisha Kikusui, source meter), a voltage was applied to the MSM device in increments of 0.2V from 0V to 10V, and the corresponding current values were measured. The current per unit area (current density) was calculated from the area of the MSM device, and the conductivity retention rate was calculated by dividing the current density of the processed MSM device by the current density of the unprocessed MSM device, and evaluated based on the following criteria:
⊚: greater than or equal to 80%
∘: greater than or equal to 60% less than 80%
×: Less than 60%

The conductivity retention rate for Comparative Example 2 was not evaluated as the charge transfer complex was not formed.

**Table 1**

| | HT Material | | Salt Compound | | | ΔE (eV) | Conductivity Retention Rate |
|---|---|---|---|---|---|---|---|
| | Type of low molecular weight compound | IP of low molecular weight compound | Type | EA of Borate anion (eV) | IP of lodonium cation (eV) | | |
| Ex. 1 | FV-1 | 5.27 | PIPB | 6.29 | 4.71 | 1.02 | ⊚ |
| Ex. 2 | FV-1 | 5.27 | D-2 | 6.08 | 4.84 | 0.81 | ○ |
| Ex. 3 | FV-2 | 5.33 | PIPB | 6.29 | 4.71 | 0.96 | ○ |
| Ex. 4 | FV-3 | 5.01 | PIPB | 6.29 | 4.71 | 1.28 | ⊚ |
| Ex. 5 | FV-3 | 5.01 | D-1 | 5.75 | 4.84 | 0.74 | ○ |
| Ex. 6 | FV-3 | 5.01 | D-2 | 6.08 | 4.84 | 1.07 | ⊚ |
| Ex. 7 | FV-3 | 5.01 | D-3 | 6.22 | 4.84 | 1.21 | ⊚ |
| Ex. 8 | FV-3 | 5.01 | D-4 | 6.17 | 4.84 | 1.16 | ⊚ |
| Ex. 9 | FV-3 | 5.01 | D-5 | 6.25 | 4.84 | 1.24 | ⊚ |
| Ex.10 | FV-4 | 5.08 | PIPB | 6.29 | 4.71 | 1.21 | ⊚ |
| Ex.11 | FV-5 | 5.27 | PIPB | 6.29 | 4.71 | 1.02 | ⊚ |
| Comp. Ex. 1 | HT-1 | 5.28 | D-1 | 5.75 | 4.84 | 0.47 | X |
| Comp. Ex. 2 | HT-2 | 4.71 | PIPB | 6.29 | 4.71 | 1.58 | Doping was not possible |

| | | | | | | | |
|---|---|---|---|---|---|---|---|
| HT Material: a material used as a low molecular weight compound IP: Ionic Potential EA: Electron Affinity FV-1 to FV-5: the low molecular weight compound 1 to the low molecular weight compound 5 | | | | | | | |

### [Measurement of Curing Temperature]

A differential scanning calorimeter (DSC) (manufactured by TA Instruments, product name: DSC-25) was used to heat a sample up to 320°C at a heating rate of 10°C/min to measure the heat balance. The onset temperature of the exothermic peak observed during the heating of the sample was defined as the curing temperature of the compound. The onset temperature is the intersection of the baseline before the start of the exotherm and the tangent drawn at the point where the rate of change of the exothermic amount first decreases after the start of the exotherm. Using the above method, the curing temperatures of the low molecular weight compound 3 and comparative compound HT-1 were measured. The results are listed in Table 2.

In addition, the current densities of quantum dot electroluminescent device 5 fabricated using low molecular weight compound 3 and of comparative quantum dot electroluminescent device 1 fabricated using comparative compound HT-1 were measured before positive aging. The current value per unit area (current density) was calculated from the area of the MSM device, and the conductivity retention rate was calculated by dividing the current density of the processed MSM device by the current density of the unprocessed MSM device.

The current density was expressed as a relative value with the value of the comparative quantum dot electroluminescent device 1 set to 1.0.

**Table 2**

| HT Material | curing temperature (°C) | Device No. | film-forming temperature (°C) | relative value of current density before Post Aging |
|---|---|---|---|---|
| compound 3 | 211 | 5 | 230 | 2.1 |
| HT-1 | 263 | Comparative 1 | 230 | 1.0 |

It was found that the low molecular weight compound 3 according to an embodiment has a curing temperature lower than the film-forming temperature. On the other hand, the comparative compound HT-1 had a curing temperature higher than the film-forming temperature. In addition, the quantum dot electroluminescent device 5 manufactured using the low molecular weight compound 3 was found to have a higher current density before positive aging compared to the comparative quantum dot electroluminescent device 1 manufactured using the comparative compound HT-1.

### Evaluation of Quantum dots Electroluminescent Devices

With regard to the quantum dot electroluminescent device 5 manufactured in Example 5 and the comparative quantum dot electroluminescent device 1 manufactured in Comparative Example 1, the driving voltage (Op.V.: Operation Voltage), luminous efficiency (EQE(%)), and luminous lifespan (LT50(hours)) were evaluated by the following method. The results are shown in Table 3 below.

### (Operation Voltage (Op.V.))

When a voltage is applied to each quantum dot electroluminescent device using a DC constant voltage power supply (manufactured by Kabushikigaisha Kiensu, source meter), current begins to flow at a constant voltage, and the quantum dot electroluminescent device emits light. The voltage at a current density of 5 mA/cm² was used as the operating voltage "Op.V.".

### (Luminous efficiency (EQE))

For each quantum dot electroluminescent device, when a voltage is applied, current begins to flow at a certain voltage, and the quantum dot electroluminescent device emits light. Using a DC constant voltage power supply (manufactured by Kabushiki Kaisha KENX, source meter), the voltage was gradually increased for each device, and the current value at that time was measured, and the luminance at the time of emission was measured using a luminance measurement device (manufactured by Kabushiki Kaisha Topcon Techno House, SR-3). Here, the measurement was stopped at the point when the luminance started to decay.

From the spectral radiance spectrum measured with the luminance measurement device, assuming Lambertian emission, the external quantum efficiency (EQE) at Op.V. was calculated.

### (Luminous lifespan (LT50))

Using a DC constant voltage power supply (manufactured by Kabushikigaisha Kenus, source meter) and applying a specified voltage to each quantum dot electroluminescent device, each quantum dot electroluminescent device was made to emit light. While measuring the emission of the quantum dot electroluminescent devices with a luminance measurement device (manufactured by Kabushikigaisha Topcon Technohouse, SR-3), the current was gradually increased, and when the luminance reached 650 nit (cd/m²), the current was kept constant and the devices were left to operate. As the luminance measured by the luminance measurement device gradually decreased, the time until it reached 50% of the initial luminance was defined as "LT50."

EQE and LT50 are expressed as relative values with the value of comparative quantum dot electroluminescent device 1 set to 1.0.

**Table 3**

| | Device No. | HT Material | Salt Compound | Op.V. (V) | Relative EQE | Relative LT50 |
|---|---|---|---|---|---|---|
| Ex. 5 | 5 | Low molecular weight compound 3 | PIPB | 3 | 1.2 | 1.6 |
| Comp. Ex. 1 | Comp. 1 | HT-1 | D-1 | 7.5 | 1 | 1 |

As shown from Table 3, the operation voltage (Op.V.) of the quantum dot electroluminescent device 5 is lower than that of comparative quantum dot electroluminescent device 1, and the EQE and LT50 of the quantum dot electroluminescent device 5 are higher than those of comparative quantum dot electroluminescent device 1.

It should be understood that embodiments described herein should be considered in a descriptive sense only and not for purposes of limitation. Descriptions of features or aspects within each embodiment should be considered as available for other similar features or aspects in other embodiments.

While one or more embodiments have been described with reference to the figures, it will be understood by those of ordinary skill in the art that various changes in form and details may be made therein without departing from the scope as defined by the following claims.

## Claims

1. A low molecular weight compound represented by Chemical Formula (1) or Chemical Formula (2): 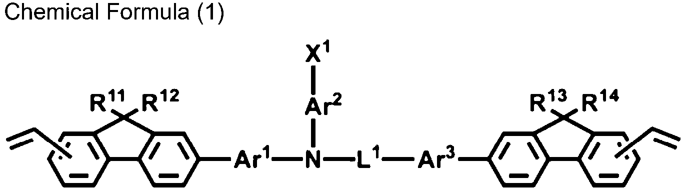 in Chemical Formula (1),
R¹¹ to R¹⁴ are, each independently, a hydrogen atom, or a hydrocarbon group having 1 to 16 carbon atoms,
Ar¹ is single bond, a substituted or unsubstituted aromatic hydrocarbon ring group, or a substituted or unsubstituted aromatic heterocyclic group,
Ar² is a substituted or unsubstituted aromatic hydrocarbon ring group, or a substituted or unsubstituted aromatic heterocyclic group,
Ar³ is a substituted or unsubstituted carbazole, a substituted or unsubstituted fluorene, a substituted or unsubstituted benzene, or a substituted or unsubstituted biphenylene group,
L¹ is single bond, or a group represented by chemical formula (1-a): 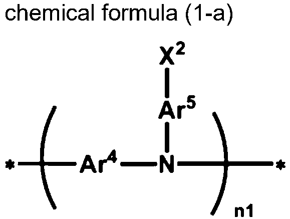
wherein in chemical formula (1-a),
Ar⁴ and Ar⁵ are, each independently, a substituted or unsubstituted aromatic hydrocarbon ring group, or a substituted or unsubstituted aromatic heterocyclic group,
X² is a hydrogen atom, or a group selected from Group 1,
n1 is an integer of 1 or 2,
wherein when n1 is 2, each Ar⁴ are the same or different,
when n1 is 2, each Ar⁵ are the same or different,
when n1 is 2, each X² are the same or different,
* is a bonding position, and
X¹ is a hydrogen atom, or a group selected from Group 1:
Group 1 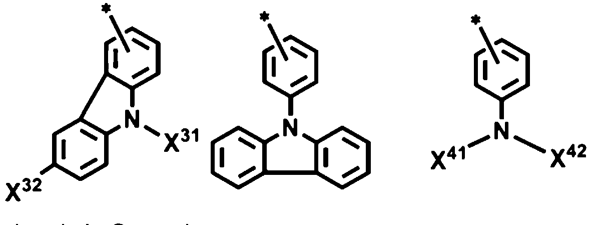
wherein in Group 1,
X³¹ and X³² are, each independently, a hydrogen atom, a group selected from Group 2, or a combination thereof,
X⁴¹ and X⁴² are, each independently, a hydrogen atom, a group selected from Group 2, or a combination thereof, and
* is a bonding position, 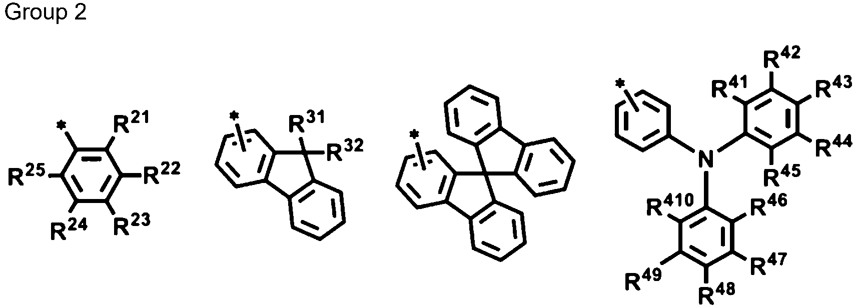
wherein in Group 2,
R²¹ to R²⁵ are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
R³¹ and R³² are, each independently, a hydrogen atom, a fluorine atom, an unsubstituted linear alkyl group having 1 to 12 carbon atoms, an unsubstituted branched alkyl group having 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3,
R⁴¹ to R⁴⁹ and R⁴¹⁰ are, each independently, a hydrogen atom, a fluorine atom, an unsubstituted linear alkyl group having 1 to 12 carbon atoms, an unsubstituted branched alkyl group having a carbon number of 3 to 12 carbon atoms, a linear alkyl group having 1 to 12 carbon atoms and substituted by a fluorine atom, a branched alkyl group having 3 to 12 carbon atoms and substituted by a fluorine atom, or a group selected from Group 3, and
* is a bonding position, 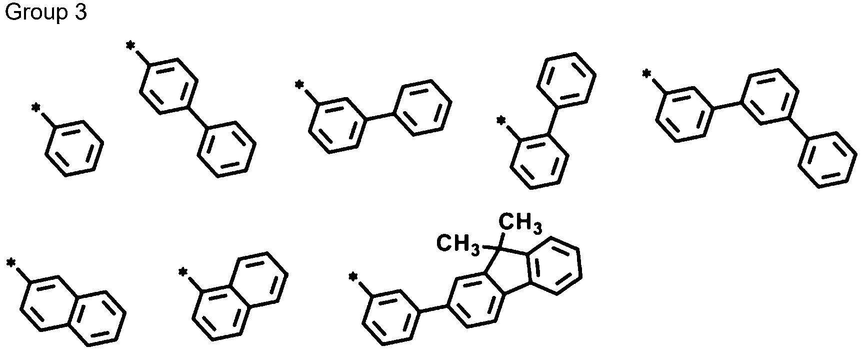
wherein in Group 3,
* is a bonding position; 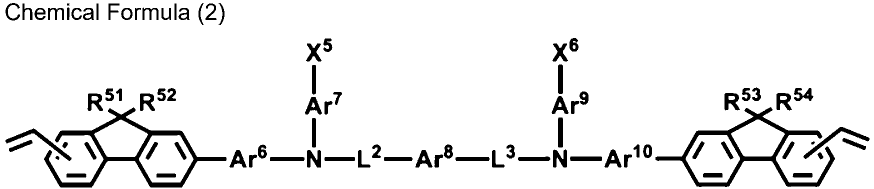
wherein in Chemical Formula (2),
R⁵¹ to R⁵⁴ are, each independently, a hydrogen atom, or a hydrocarbon group having 1 to 16 carbon atoms,
Ar⁶ and Ar¹⁰ are, each independently, single bond, a substituted or unsubstituted aromatic hydrocarbon ring group, or a substituted or unsubstituted aromatic heterocyclic group,
Ar⁷ and Ar⁹ are, each independently, a substituted or unsubstituted aromatic hydrocarbon ring group, or a substituted or unsubstituted aromatic heterocyclic group,
Ar⁸ is a substituted or unsubstituted carbazole, a substituted or unsubstituted fluorene, a substituted or unsubstituted benzene, or a substituted or unsubstituted biphenyl ring, and
L² is a single bond, or a group represented by chemical formula (2-a): 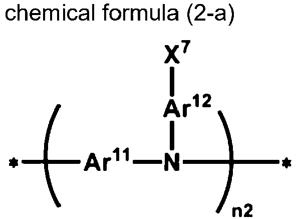
wherein in chemical formula (2-a),
Ar¹¹ and Ar¹² are, each independently, a substituted or unsubstituted aromatic hydrocarbon ring group, or a substituted or unsubstituted aromatic heterocyclic group,
X⁷ is a hydrogen atom or a group selected from Group 1,
n2 is an integer of 1 or 2,
wherein when n2 is 2, each Ar¹¹ are the same or different,
when n2 is 2, each Ar¹² are the same or different,
when n2 is 2, each X⁷ are the same or different,
* is a bonding position, and
L³ is a single bond, or a group represented by chemical formula (2-b): 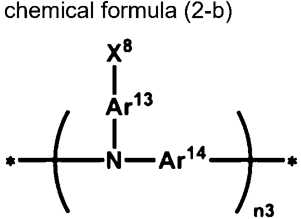
wherein in chemical formula (2-b),
Ar¹³ and Ar¹⁴ are, each independently, a substituted or unsubstituted aromatic hydrocarbon ring group, or a substituted or unsubstituted aromatic heterocyclic group,
X⁸ is a hydrogen atom or a group selected from Group 1,
n3 is an integer of 1 or 2, wherein
wherein when n3 is 2, each Ar¹³ are the same or different,
when n3 is 2, each Ar¹⁴ are the same or different,
when n3 is 2, each X⁸ are the same or different,
* is a bonding position, and
X⁵ and X⁶ are, each independently, a hydrogen atom or a group selected from Group 1.

2. The low molecular weight compound according to claim 1,
wherein in Chemical Formula (1),
R¹¹ to R¹⁴ are, each independently, a hydrogen atom, or a hydrocarbon group having 1 to 10 carbon atoms,
Ar¹ and Ar² are, each independently, a substituted or unsubstituted aromatic hydrocarbon ring group,
Ar³ is a substituted or unsubstituted fluorene ring group, a substituted or unsubstituted benzene ring group, or a substituted or unsubstituted biphenyl ring group,
L¹ is single bond, or a group represented by chemical formula (1-a),
wherein in chemical formula (1-a),
Ar⁴ and Ar⁵ are, each independently, a substituted or unsubstituted aromatic hydrocarbon ring group,
X¹ of Chemical Formula (1) and X² of chemical formula (1-a) are, each independently, a hydrogen atom, or a group represented by the following chemical formula: 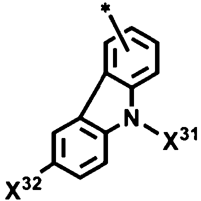
wherein in the chemical formula, X³¹ and X³² are, each independently, a hydrogen atom, or a group represented in Group 2.

3. The low molecular weight compound according to claims 1 or 2,
wherein in Chemical Formula (1),
R¹¹ to R¹⁴ are, each independently, a hydrocarbon group having 1 to 6 carbon atoms,
Ar¹ to Ar³ are, each independently, a substituted or unsubstituted benzene ring group, a biphenyl ring group, or a fluorene ring group,
L¹ is single bond, or a group represented by chemical formula (1-a), wherein in chemical formula (1-a),
Ar⁴ and Ar⁵ are, each independently, a substituted or unsubstituted benzene ring group, a biphenyl ring group, or a fluorene ring group,
X¹ of chemical formula (1) and X² of chemical formula (1-a) are, each independently, a hydrogen atom, or a group represented by the following chemical formula: 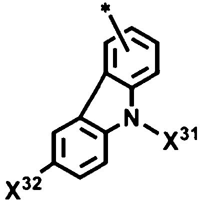
wherein in the chemical formula,
X³¹ and X³² are, each independently, a hydrogen atom, or a group represented by chemical formula (a): 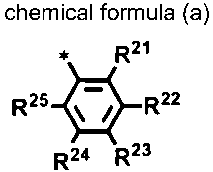
wherein in chemical formula (a),
R²¹ and R²⁵ are, each independently, a hydrogen atom, a fluorine atom, an unsubstituted linear alkyl group having 1 to 12 carbon atoms, an unsubstituted branched alkyl group having 1 to 12 carbon atoms, a linear alkyl group having 1 to 12 carbon atoms and substituted by a fluorine atom, a branched alkyl group having 1 to 12 carbon atoms and substituted by a fluorine atom, or a group listed in Group 3.

4. The low molecular weight compound according to any of claims 1-3, wherein X¹ of Chemical Formula (1) and X² of chemical formula (1-a) are, each independently, a hydrogen atom, or a group selected from Group (1-1): 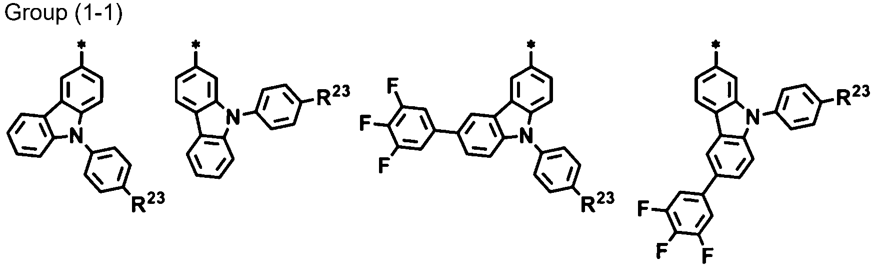 wherein in Group (1-1),
R²³ is a hydrogen atom, a fluorine atom, an unsubstituted linear alkyl group having 1 to 12 carbon atoms, an unsubstituted branched alkyl group having 1 to 12 carbon atoms, a linear alkyl group having 1 to 12 carbon atoms and substituted by a fluorine atom, a branched alkyl group having 1 to 12 carbon atoms and substituted by a fluorine atom, or a group selected from Group 3.

5. The low molecular weight compound according to any of claims 1-4,
wherein in Chemical Formula (2),
R⁵¹ to R⁵⁴ are, each independently, a hydrocarbon group having 1 to 10 carbon atoms,
Ar⁶ and Ar¹⁰ are, each independently, a single bond, or a substituted or unsubstituted aromatic hydrocarbon ring group,
Ar⁷ and Ar⁹ are, each independently, a substituted or unsubstituted aromatic hydrocarbon ring group,
Ar⁸ is a substituted or unsubstituted fluorene ring, a substituted or unsubstituted benzene ring, or a substituted or unsubstituted biphenyl ring, and
Ar¹¹ and Ar¹² are, each independently, a substituted or unsubstituted aromatic hydrocarbon ring, and
Ar¹³ and Ar¹⁴ are, each independently, a substituted or unsubstituted aromatic hydrocarbon ring.

6. The low molecular weight compound according to any of claims 1-5,
wherein in Chemical Formula (2),
R⁵¹ to R⁵⁴ are, each independently, a hydrocarbon group having 1 to 6 carbon atoms,
Ar⁶ and Ar¹⁰ are both single bonds,
Ar⁷ and Ar⁹ are, each independently, a substituted or unsubstituted aromatic hydrocarbon ring group, and
L² and L³ are both single bonds.

7. The low molecular weight compound according to any of claims 1-6, represented by the following compounds:

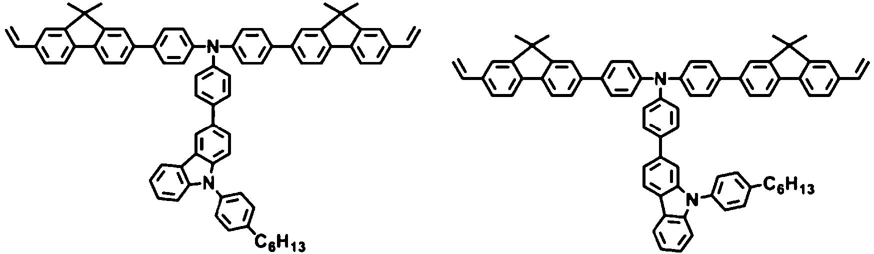

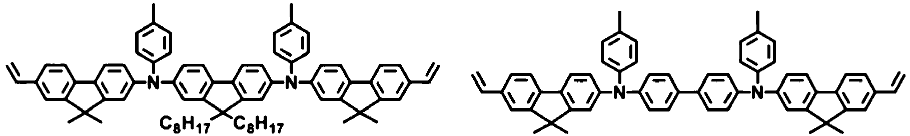

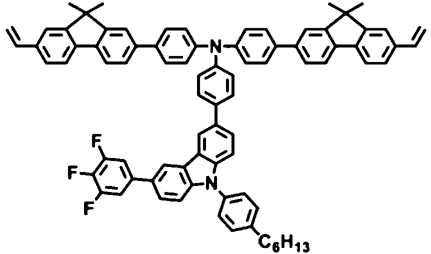

8. A composition comprising the low molecular weight compound according to any of claims 1-7, and a salt compound comprising a borate anion represented by Chemical Formula (3) and an iodonium cation: 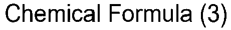 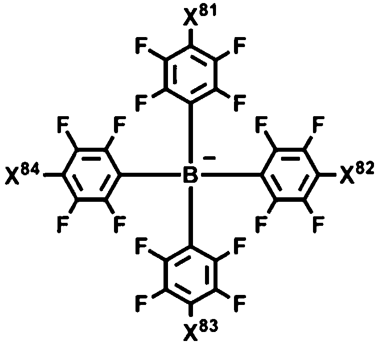 wherein in chemical formula (3),
X⁸¹ to X⁸⁴ are, each independently, a hydrogen atom, a fluorine atom, or a group represented by chemical formula (3-a):
chemical formula (3-a) *-L⁴-(Ar¹⁶)ₙ₆-(Z)ₙ₄
wherein in chemical formula (3-a),
L⁴ is independently a single bond or a group represented by chemical formula (3-b),
Ar¹⁶ is a substituted or unsubstituted aromatic hydrocarbon ring group or a substituted or unsubstituted aromatic heterocyclic group,
Z is a fluorine atom or a group selected from Group 4,
n4 is an integer from 1 to 5,
n6 is an integer of 0 or 1,
when there are two or more groups represented by chemical formula (3-a), each L⁴ are the same or different,
when there are two or more groups represented by chemical formula (3-a), each Ar¹⁶ are the same or different,
when there are two or more groups represented by chemical formula (3-a), each Z are the same or different,
when there are two or more groups represented by chemical formula (3-a), each n4 are the same or different,
when there are two or more groups represented by chemical formula (3-a), each n6 are the same or different,
when n4 is an integer of greater than or equal to 2, each Z are the same or different, and
* is a bonding position, 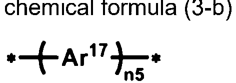
wherein in chemical formula (3-b),
Ar¹⁷ is a substituted or unsubstituted aromatic hydrocarbon ring group or a substituted or unsubstituted aromatic heterocyclic group,
n5 is an integer from 1 to 5, and when n5 is an integer of greater than or equal to 2, each Ar¹⁷ are the same or different, and
* is a bonding position, and 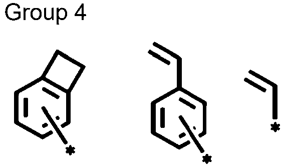
wherein in Group 4,
* is a bonding position.

9. The composition according to claim 8, wherein the borate anion is represented by the following compounds: 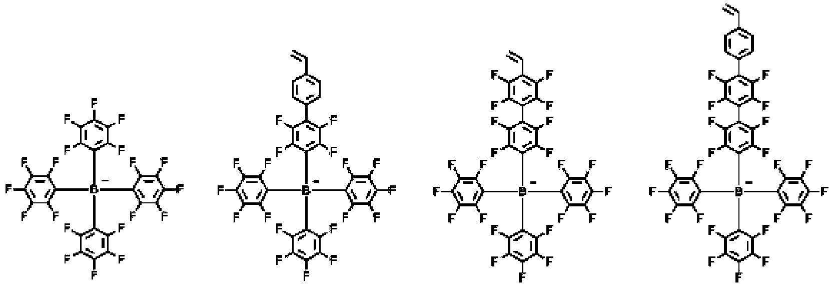 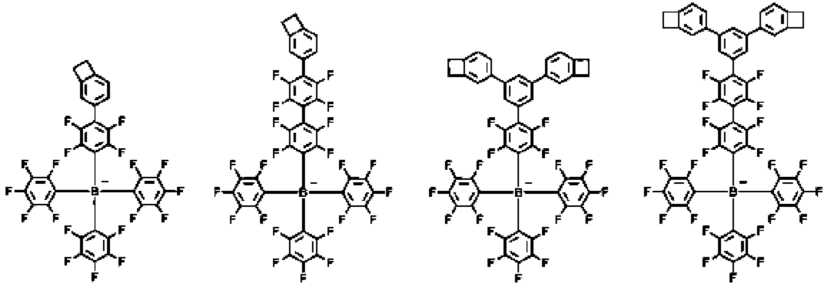 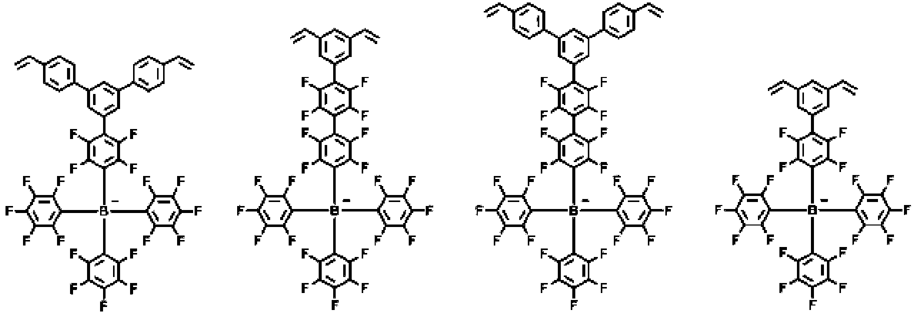 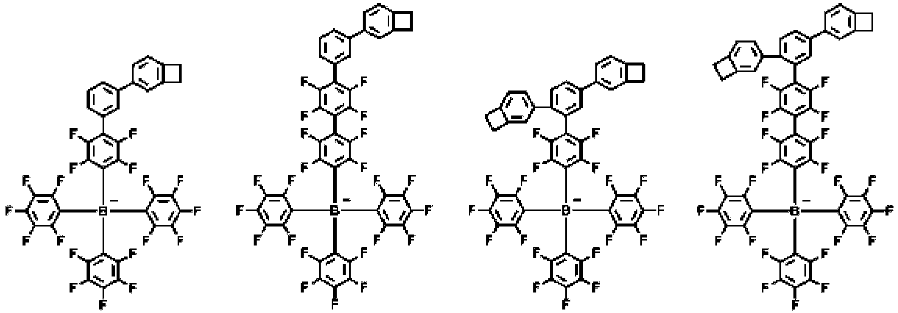 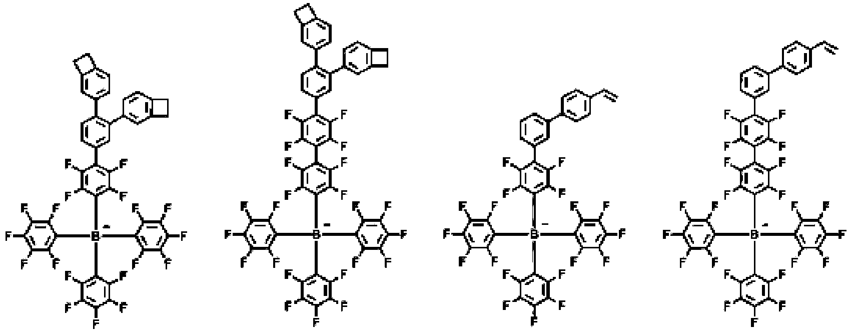 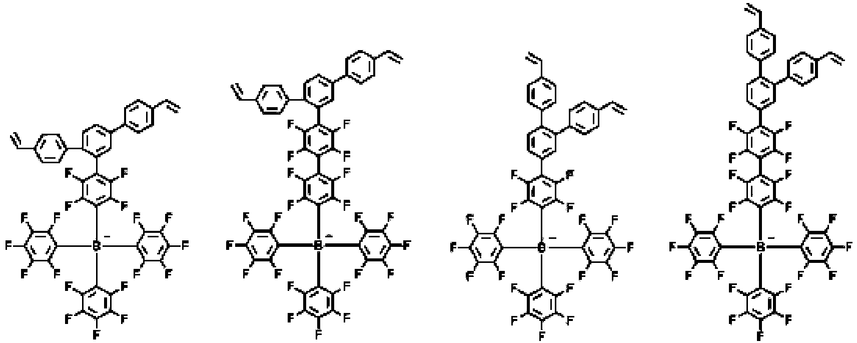 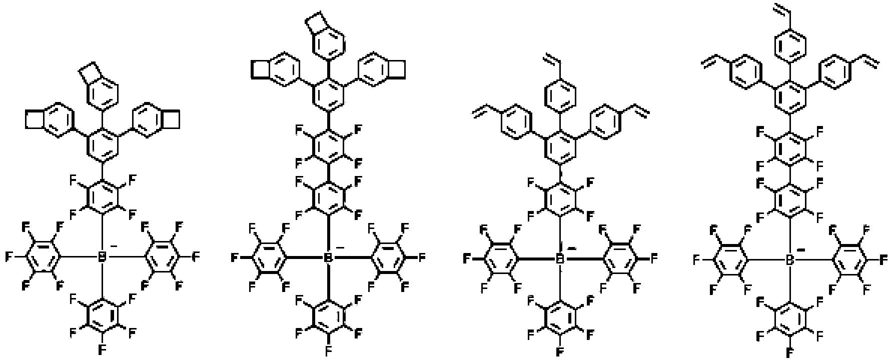 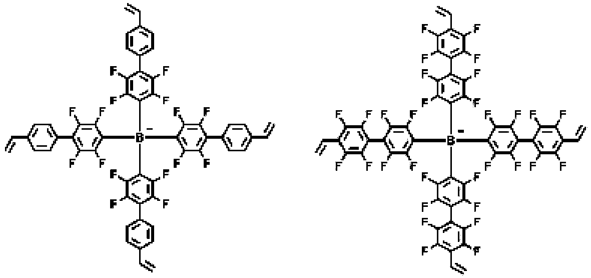 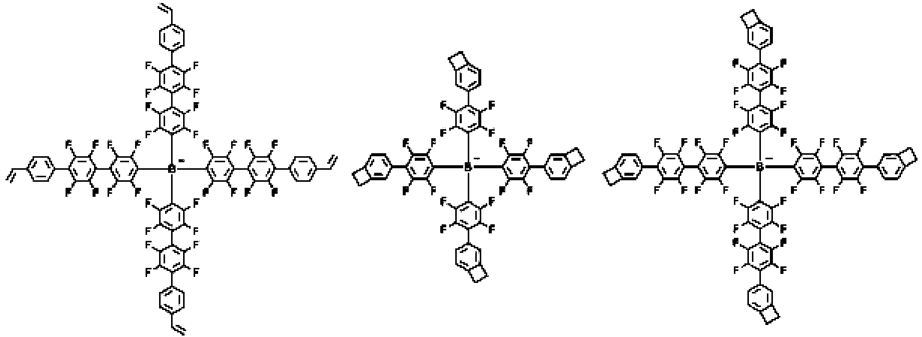 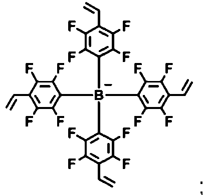 and/or
wherein an energy difference between an ionization potential of the low molecular weight compound and an electron affinity of the borate anion is greater than or equal to 0.75 eV, and the ionization potential of the low molecular weight compound is deeper than an ionization potential of the iodonium cation.

10. The composition according to claims 8 or 9, wherein the electron affinity of the borate anion is greater than or equal to 5.50 eV and less than or equal to 6.50 eV; and/or
wherein the iodonium cation is difuryliodonium cation, dithienyliodonium cation, bis(4,5-dimethyl-2-furyl) iodonium cation, bis(5-chloro-2-thienyl) iodonium cation, bis (5-cyano-2-furyl) iodonium cation, bis(5-nitro-2-thienyl) iodonium cation, bis(5-acetyl-2-furyl) iodonium cation, bis(5-carboxy-2-thienyl) iodonium cation, bis(5-methoxycarbonyl-2-furyl) iodonium cation, bis(5-phenyl-2-furyl) iodonium cation, bis(5-(p-methoxyphenyl)-2-thienyl) iodonium cation, bis(5-vinyl-2-furyl) iodonium cation, bis(5-ethynyl-2-thienyl) iodonium cation, bis(5-cyclohexyl-2-furyl) iodonium cation, bis (5-hydroxy-2-thienyl) iodonium cation, bis(5-phenoxy-2-furyl) iodonium cation, bis(5-mercapto-2-thienyl) iodonium cation, bis(5-butylthio-2-thienyl) iodonium cation, bis(5-phenylthio-2-thienyl) iodonium cation, diphenyliodonium cation, bis(p-tolyl)iodonium cation, bis(p-octylphenyl) iodonium cation, bis(p-octadecylphenyl) iodonium cation, bis(p-octyloxyphenyl) iodonium cation, bis(p-octadecyloxyphenyl) iodonium cation, phenyl(p-octadecyloxyphenyl) iodonium cation, 4-isopropyl-4'-methyldiphenyliodonium cation, (4-isobutylphenyl)-p-tolyliodonium cation, bis(1-naphthyl) iodonium cation, bis(4-phenylsulfanylphenyl) iodonium cation, phenyl(6-benzoyl- 9-ethyl- 9H-carbazol-3-yl) iodonium cation, (7-methoxy-2-oxo-2H-chromen-3-yl)-4'-isopropylphenyliodonium cation, or a combination of any two or more iodonium cations listed above.

11. The composition according to any of claims 8-10, wherein the ionization potential of the iodonium cation is greater than or equal to 4.00 eV and less than or equal to 4.95 eV; and/or
wherein an amount of the salt compound in the composition is greater than or equal to 1 weight percent and less than or equal to 70 weight percent based on a total weight of the low molecular weight compound and the salt compound.

12. An organic film comprising a cured material obtainable by curing the composition according to any of claims 8-11, and a carboxyl-containing compound.

13. The organic film according to claim 12, wherein the carboxyl-containing compound is acetic acid, propionic acid, butyric acid, valeric acid, caproic acid, malonic acid, succinic acid, glutaric acid, adipic acid, pimelic acid, (meth)acrylic acid, poly(meth)acrylic acid, benzoic acid, hexahydrobenzoic acid, terephthalic acid, 1,4-cyclohexanedicarboxylic acid, citric acid, malic acid, 3-isopropylmalic acid, itaconic acid, citraconic acid, or a combination thereof.

14. An electroluminescent device, comprising:
a first electrode supplying holes,
a second electrode supplying electrons, and
an organic layer disposed between the first electrode and the second electrode,
wherein at least one layer of the organic layer includes the organic film according to claims 12 or 13;
preferably wherein the at least one layer of the organic layer including the organic film is a hole injection layer.

15. The electroluminescent device according to claim 14, wherein the organic layer comprises a light emitting layer including semiconductor nanoparticles, an organometallic complex, or a combination thereof;
preferably wherein the light emitting layer includes semiconductor nanoparticles.
